# EUROPEAN PATENT SPECIFICATION

(11) **EP 2 068 841 B1**
(45) Date of publication and mention of the grant of the patent: **26.09.2018**
(21) Application number: 07871127.2
(22) Date of filing: 05.10.2007
(51) Int. Cl.: A61K 9/48, A61K 9/16, A61K 9/24, A61K 31/44, A61K 31/4439, A61K 9/00, A61K 9/14, A61K 9/20, A61K 9/28, A61K 9/50

(54) **NOVEL FORMULATIONS OF PROTON PUMP INHIBITORS AND METHODS OF USING THESE FORMULATIONS**
NEUARTIGE FORMULIERUNGEN VON PROTONENPUMPENHEMMERN UND VERFAHREN ZU IHRER VERWENDUNG
NOUVELLES FORMULATIONS POUR UNE LIBÉRATION IMMÉDIATE D'INHIBITEURS DE POMPE À PROTONS ET PROCÉDÉS D'UTILISATION DE CES FORMULATIONS

(30) Priority: 05.10.2006 US 828374 P
(43) Date of publication of application: 17.06.2009
(73) Proprietor: Santarus, Inc., San Diego, CA 92130 (US)
(72) Inventor: HALL, Warren, Del Mar, CA 92014 (US); WESTON, Laura, Escondido, CA 92026 (US); OLMSTEAD, Kay, San Diego, CA 92127 (US); GALLO, Laura, Oceanside, CA 92057 (US); BOWE, Craig, Encinitas, CA 92024 (US)
(74) Representative: Avidity IP
(86) International application number: PCT/US2007/080641
(87) International publication number: WO 2008/067037

(56) References cited:
- WO-A1-01/51050
- WO-A1-03/009846
- WO-A1-2005/115474
- US-A1- 2005 037 070
- US-A1- 2005 037 070
- US-A1- 2005 112 193
- US-A1- 2005 249 806
- US-A1- 2005 266 071
- US-A1- 2005 266 071
- US-B1- 6 284 271
- LETERME P ET AL: "Influence of the morphogranulometry and hydrophobicity of talc on its antisticking power in the production of tablets", INTERNATIONAL JOURNAL OF PHARMACEUTICS, ELSEVIER BV, NL, vol. 289, no. 1-2, 31 January 2005 (2005-01-31), pages 109-115, XP027624308, ISSN: 0378-5173 [retrieved on 2005-01-31]
- Rowe ET AL: "Sodium Stearyl Fumarate" In: "Handbook of pharmaceutical excipients", 2 February 2003 (2003-02-02), PHARMACEUTICAL PRESS, GB, XP055253337, ISBN: 978-0-85369-472-4 pages 585-587,

## Description

### TECHNICAL FIELD

The present invention relates to combinations of a proton pump inhibiting agent and at least one buffering agent that have been found to possess improved bioavailability, chemical stability, dissolution profiles, disintegration times, as well as other improved pharmacokinetic, pharmacodynamic, chemical and/or physical properties. The present invention is directed to methods, kits, combinations, and compositions for treating, preventing or reducing the risk of developing a gastrointestinal disorder or disease including nocturnal acid breakthrough, or the symptoms associated therewith.

The scope of the invention is defined by the claims. Any references in the description to methods of treatment refer to the compounds, pharmaceutical compositions and medicaments of the present invention for use in a method for treatment of the human (or animal) body by therapy (or for diagnosis).

### BACKGROUND OF THE INVENTION

Upon ingestion, most acid-labile pharmaceutical compounds must be protected from contact with acidic stomach secretions to maintain their pharmaceutical activity. To accomplish this, compositions with enteric- coatings have been designed to dissolve at a pH to ensure that the drug is released in the proximal region of the small intestine (duodenum), rather than the acidic environment of the stomach.

A class of acid-labile pharmaceutical compounds that are administered as enteric-coated dosage forms are proton pump inhibiting agents. Exemplary proton pump inhibitors include, omeprazole (Prilosec®), lansoprazole (Prevacid®), esomeprazole (Nexium®), rabeprazole (Aciphex®), pantoprazole (Protonix®), pariprazole, tenatoprazole, and leminoprazole. The drugs of this class suppress gastrointestinal acid secretion by the specific inhibition of the H⁺/K⁺-ATPase enzyme system (proton pump) at the secretory surface of the gastrointestinal parietal cell. See, e.g., Fellenius et al., Substituted Benzimidazoles Inhibit Gastrointestinal Acid Secretion by Blocking H+/K+-ATPase, Nature, 290: 159-161 (1981); Wallmark et al., The Relationship Between Gastrointestinal Acid Secretion and Gastrointestinal H+/K+-ATPase Activity, J. Biol. Chem., 260: 13681-13684 (1985); and Fryklund et al, Function and Structure of Parietal Cells After H+/K+-ATPase Blockade, Am. J. Physiol., 254 (1988). Most proton pump inhibitors are susceptible to acid degradation and, as such, are rapidly destroyed in a low pH environment. Therefore, if the enteric-coating of these formulated products is disrupted (e.g., trituration to compound a liquid, or chewing the capsule or tablet) or the buffering agent fails to sufficiently neutralize the gastrointestinal pH, the drug will be exposed to degradation by the gastrointestinal acid in the stomach. Omeprazole is one example of a proton pump inhibitor which is a substituted bicyclic aryl- imidazole that inhibits gastrointestinal acid secretion.

Non-enteric coated pharmaceutical compositions containing buffers have been described in, *e.g.* U.S. Patent Nos. 5,840,737; 6,489,346; 6,645,988; and 6,699,885; and U.S. Patent Application Nos. 10/898,135, 10/783,871; 10/938,766; 111138,763; 11/287,888; 10/893,203; 11/338,608; and 10/893,092. These compositions and methods involve the administration of one or more buffering agents with an acid labile pharmaceutical agent, such as a proton pump inhibitor. The buffering agent is thought to prevent substantial degradation of at least some the acid labile pharmaceutical agent in the acidic environment of the stomach by raising the pH.

There remains a need for novel pharmaceutical formulations that rapidly, efficiently and effectively release proton pump inhibitors into the gastrointestinal tract for absorption of an intact, non-acid degraded or non-acid reacted form of a proton pump inhibitor into the bloodstream. There also remains a need for new methods for treating and or preventing gastrointestinal disorders such as nocturnal acid breakthrough and nighttime gastric acidity. US 2005/0249806 and WO 2003/009846 disclose compositions of proton pump inhibitors.

### Summary

The present invention provides a pharmaceutical composition in the form of a capsule which achieves an *in vitro* initial rise in pH within about 4 minutes comprising:
(a) 10 mg to 100 mg of at least one acid labile bicyclic-aryl-imidazole proton pump inhibiting agent;
(b) at least one anatacid in an amount sufficient to increase gastric fluid pH to a pH that prevents acid degradation of at least some of the proton pump inhibitor in the gastric fluid; wherein the antacid comprises at least 400 mg of NaHCO₃; and '
(c) 0.5 wt-% to 3 wt-% of the hydrophilic lubricant sodium stearyl fumarate.

Described herein is a pharmaceutical composition comprising a proton pump inhibiting agent and a buffering agent for oral administration and ingestion by a subject. In one case, upon administration to a subject, the composition contacts the gastric fluid of the stomach and increases the gastric pH of the stomach to a pH that prevents or inhibits acid degradation of the proton pump inhibiting agent in the gastric fluid of the stomach and allows a measurable serum concentration of the proton pump inhibiting agent to be absorbed into the blood serum of the subject, such that pharmacokinetic and pharmacodynamic parameters can be obtained using testing procedures known to those skilled in the

Described herein are pharmaceutical compositions in solid dosage form comprising (a) a therapeutically effective amount of at least one acid labile proton pump inhibiting agent; (b) at least one antacid; and may or may not include (c) a hydrophilic lubricant. Further, this general case includes methods of treating or preventing nocturnal GERD symptoms in a patient in need by administering these compositions, methods of treating or preventing nocturnal acid breakthrough in a patient in need by administering these compositions, and methods for reducing nighttime gastric acidity in a patient in need by administering these compositions.

Described herein are pharmaceutical compositions in solid dosage form comprising (a) a therapeutically effective amount of at least one acid labile proton pump inhibiting agent; and (b) between about 20 mEq to about 40 mEq of antacid, as well as methods of treating or preventing nocturnal GERD symptoms in a patient in need by administering these compositions, methods of treating or preventing nocturnal acid breakthrough in a patient in need by administering these compositions, and methods for reducing nighttime gastric acidity in a patient in need by administering these compositions.

Described herein are pharmaceutical compositions in solid dosage form comprising: (a) a therapeutically effective amount of at least one acid labile bicyclic-aryl-imidazole proton pump inhibiting agent; (b) at least one antacid; and (c) a hydrophilic lubricant.

Described herein are methods of treating or preventing nocturnal acid breakthrough in a patient by administering a pharmaceutical composition in solid dosage form at bedtime, wherein the pharmaceutical composition comprises: (a) a therapeutically effective amount of at least one acid labile bicyclic-aryl-imidazole proton pump inhibiting agent; (b) at least one antacid; and (c) a hydrophilic lubricant.

Described herein are methods of treating or preventing nocturnal acid breakthrough in a patient by administering a pharmaceutical composition in solid dosage form at bedtime, wherein the pharmaceutical composition comprises: (a) a therapeutically effective amount of at least one acid labile bicyclic-aryl-imidazole proton pump inhibiting agent; and (b) between about 20 mEq to about 40 mEq of antacid; wherein the composition is at least about 20% better at preventing nocturnal acid breakthrough than an enteric coated formulation of the proton pump inhibiting agent.

In some cases, the present invention relates to methods of reducing nighttime gastric acidity in a patient by administering a pharmaceutical composition in solid dosage form at bedtime, wherein the pharmaceutical composition comprises: (a) a therapeutically effective amount of at least one acid labile proton pump inhibiting agent; (b) at least one antacid; and (c) a hydrophilic lubricant.

Described herein methods of reducing nighttime gastric acidity in a patient by administering a pharmaceutical composition in solid dosage form at bedtime, wherein the pharmaceutical composition comprises: (a) a therapeutically effective amount of at least one acid labile proton pump inhibiting agent; and (b) between about 20 mEq to about 40 mEq of antacid; wherein after administration of the composition for 7 days, the composition is at least about 20% better at maintaining the pH of the patients stomach above 4 during the first 4 hours after administration.

Described herein are methods of reducing nighttime gastric acidity in a patient by administering a pharmaceutical composition in a caplet dosage form at bedtime, wherein the pharmaceutical composition comprises: (a) a therapeutically effective amount of at least one acid labile proton pump inhibiting agent; and (b) between about 15 mEq to about 40 mEq of antacid; wherein after administration of the composition for 7 days, the composition is at least about 20% better at maintaining the pH of the patients stomach above 4 during the first 4 hours after administration.

Described herein are methods of treating or preventing nocturnal acid breakthrough in a patient by administering a pharmaceutical composition in a caplet dosage form at bedtime, wherein the pharmaceutical composition comprises: (a) a therapeutically effective amount of at least one acid labile bicyclic-aryl-imidazole proton pump inhibiting agent; and (b) between about 15 mEq to about 40 mEq of antacid; wherein the composition is at least about 20% better at preventing nocturnal acid breakthrough than an enteric coated formulation of the proton pump inhibiting agent.

In some cases, the amount of proton pump inhibiting agent present in the pharmaceutical composition is about 20 mg. In some cases, the amount of proton pump inhibiting agent present in the pharmaceutical composition is about 40 mg.

In some cases, the proton pump inhibiting agent is omeprazole, lansoprazole, esomeprazole, rabeprazole, pantoprazole, pariprazole, tenatoprazole, or leminoprazole, or a free base, free acid, salt, hydrate, polymorph, enantiomer, isomer, tautomer, or prodrug thereof. In some cases, the proton pump inhibiting agent is omeprazole, or a free base, free acid, salt, hydrate, polymorph, enantiomer, isomer, tautomer, or prodrug thereof. In some cases, the proton pump inhibiting agent comprises lansoprazole, or a free base, free acid, salt, hydrate, polymorph, enantiomer, isomer, tautomer, or prodrug thereof. In some cases, the proton pump inhibiting agent comprises esomeprazole, or a free base, free acid, salt, hydrate, polymorph, enantiomer, isomer, tautomer, or prodrug thereof.

In some cases, the antacid is present in an amount of about 10 mEq to about 50 mEq. In some cases, the antacid is present in an amount of about 10 mEq to about 30 mEq. In some cases, the antacid is present in an amount of about 13 mEq. In some cases, the antacid is present in an amount of about 20 mEq. In some cases, the antacid is present in an amount of about 25 mEq.

In other cases, the antacid comprises at least about 400 mgs of sodium bicarbonate. In some cases, the antacid comprises a high efficiency antacid and a soluble antacid. In some cases the antacid comprises sodium bicarbonate and magnesium hydroxide. In other cases, the antacid is sodium bicarbonate.

In some cases the solid dosage form is a tablet, a chewable tablet, a caplet, or a capsule.

The hydrophilic lubricant is sodium stearyl fumarate.

In some cases the composition is at least about 20% better at preventing nocturnal acid breakthrough than an enteric coated formulation of the proton pump inhibiting agent. In some cases, the pharmaceutical composition is about 30% better at preventing nocturnal acid breakthrough than an enteric coated formulation of the proton pump inhibiting agent. In yet other cases, the pharmaceutical composition is about 40% better at preventing nocturnal acid breakthrough than an enteric coated formulation of the proton pump inhibiting agent.

In some cases, the pharmaceutical composition is administered once a day. In other cases, the pharmaceutical composition is administered twice a day. In yet other cases, the pharmaceutical composition is administered for two or more consecutive days. In some cases, the pharmaceutical composition is administered less than 1 hour before retiring to bed.

In some cases, the composition is at least about 20% better at maintaining the pH of the patient's stomach above 4 during the first 4 hours after administration. In some cases, following administration of the pharmaceutical composition the patient's average gastric pH for an 8-hour nighttime period is greater than about 4. In other cases, during an 8-hour nighttime period after administration of the pharmaceutical composition the patient's gastric pH is greater than about 4 at least about 50% of the time.

In some cases, the method treats or prevents heartburn.

In some cases, the pharmaceutical composition is in a solid dosage form comprising (a) about 10 mgs to about 100 mgs of at least one acid labile bicyclic-aryl-imidazole proton pump inhibiting agent; (b) at least one antacid in an amount sufficient to increase gastric fluid pH to a pH that prevents acid degradation of at least some of the proton pump inhibitor in the gastric fluid; wherein the antacid comprises at least about 400 mgs of NaHCO₃; and (c) about 0.5 wt-% to about 3 wt-% of a hydrophilic lubricant; wherein the composition achieves an in vitro initial rise in pH within about 4 minutes. In some cases, the composition achieves an in vitro initial pH of at least about 4 within about 2 minutes. In some cases, the hydrophilic lubricant is sodium stearyl fumarate. In some cases, the proton pump inhibitor is omeprazole, esomeprazole or lansoprazole, or a salt thereof. In some cases, the composition further comprises an antacid selected from potassium bicarbonate, sodium carbonate, calcium carbonate, magnesium oxide, magnesium hydroxide, magnesium carbonate, aluminum hydroxide, and mixtures thereof; and the total amount of antacid present in the capsule is about 10 mEq to about 30 mEq. In some cases, the sodium bicarbonate is present in an amount of at least about 800 mgs. In some cases, the composition further comprises between about 2 wt-% to about 6 wt-% croscarmellose sodium.

Described herein are methods for treating or preventing a gastrointestinal disorder in a patient comprising the step of administering a composition in a solid dosage form comprising: (a) about 10 mgs to about 100 mgs of at least one acid labile bicyclic-aryl-imidazole proton pump inhibiting agent; (b) at least one antacid in an amount sufficient to increase gastric fluid pH to a pH that prevents acid degradation of at least some of the proton pump inhibitor in the gastric fluid; wherein the antacid comprises at least about 400 mgs of NaHCO₃; and (c) about 0.5 wt-% to about 3 wt-% of sodium stearyl fumarate; wherein the composition is administered to a fasted subject daily and the Tmax of the proton pump inhibitor is less than about 45 minutes on Day **1** and Day 7 of administration of the composition. In some cases, the initial serum concentration of the proton pump inhibitor is greater than about 0.3 µ/ml within about 45 minutes after oral administration of the tablet to the subject. In some cases, the average Cₘₐₓ of the proton pump inhibiting agent is less than about 1250 ng/ml after oral administration of the tablet to the subject. In some cases, the solid dosage form is a tablet, a chewable tablet, a caplet, or a capsule.

Described herein are methods for treating or preventing nocturnal acid breakthrough or reducing nighttime gastric acidity in a patient by administering a pharmaceutical composition in solid dosage form at bedtime, wherein the pharmaceutical composition comprises: (a) about 10 mgs to about 100 mgs of at least one acid labile bicyclic-aryl-imidazole proton pump inhibiting agent; (b) at least one antacid in an amount sufficient to increase gastric fluid pH to a pH that prevents acid degradation of at least some of the proton pump inhibitor in the gastric fluid; wherein the antacid comprises at least about 400 mgs of NaHC03; and (c) about 0.5 wt-% to about 3 wt-% of sodium stearyl fumarate; wherein the composition is administered to a fasted subject daily and the Tmax of the proton pump inhibitor is less than about 45 minutes on Day 1 and Day 7 of administration. In some cases, the composition is at least about 30% better at preventing nocturnal acid breakthrough than an enteric coated formulation of the proton pump inhibiting agent. In some cases, the pharmaceutical composition is administered less than 1 hour before retiring to bed. In some cases, during an 8-hour nighttime period after administration of the pharmaceutical composition the patient's gastric pH is greater than about 4 at least about 50% of the time.

Described herein are methods for treating or preventing nocturnal acid breakthrough or reducing nighttime gastric acidity in a patient by administering a pharmaceutical composition in solid dosage form at bedtime, wherein the pharmaceutical composition comprises: (a) about 10 to about 100 mgs of at least one acid labile bicyclic-aryl-imidazole proton pump inhibiting agent; and (b) between about 20 mEq to about 40 mEq of antacid, wherein the antacid comprises at least about 400 mgs of NaHC03; and wherein after administration of the composition for 7 days, the composition is at least about 20% better at maintaining the pH of the patients stomach above 4 during the first 4 hours after administration. In some cases, the composition is at least about 30% better at maintaining the pH of the patient's stomach above 4 during the first 4 hours after administration. In some cases, following administration of the pharmaceutical composition the patient's average gastric pH for an 8-hour nighttime period is greater than about 4. In some cases, the pharmaceutical composition is administered once a day for two or more consecutive days. In some cases, the pharmaceutical composition is administered twice a day for two or more consecutive days. In some cases, the pharmaceutical composition is administered less than 1 hour before retiring to bed. In some cases, the amount of proton pump inhibiting agent is omeprazole or esomeprazole or a salt thereof and is present in the pharmaceutical composition is about 20 mg or about 40 mgs. In some cases, the antacid further comprises a high efficiency antacid. In some cases, the high efficiency antacid is magnesium hydroxide. In some cases, the solid dosage form is a caplet and the composition further comprises about 5 wt-% to about 10 wt% of a binder. In some cases, solid dosage form is a capsule and the composition further comprises less than about 3 wt-% of a binder.

Described herein are pharmaceutical compositions in a tablet dosage form comprising: (a) about 20 to about 100 mg of a proton pump inhibitor; and (b) at least about 400 mgs of directly compressible sodium bicarbonate; wherein the hardness of the tablet is between 10-20 kP. In some cases, the tablet achieves a hardness of 10-20 kP with less than 10,000 lbs of force. In some cases, the tablet achieves an in vitro initial rise in pH within about 4 minutes. In some cases, upon administration to a fasted subject, the tablet provides a Tmax between about 30 minutes and about 45 minutes on Day 1. In some cases, upon administration to a fasted subject, the tablet provides a Tₘₐₓ of less than about 45 minutes on Day 7. In some cases, the tablet comprises 750 mgs of the compressible sodium bicarbonate. In some cases, the directly compressible sodium bicarbonate comprises between about 90-98 wt-% sodium bicarbonate and about 2-10 wt-% hydroxypropyl cellulose. In some cases, the directly compressible sodium bicarbonate comprises about 2 wt-% to about 10 wt-% hydroxypropyl cellulose. In some cases, the directly compressible sodium bicarbonate is about 97 wt-% sodium bicarbonate and about 3 wt-% hydroxypropyl cellulose. In some cases, the directly compressible sodium bicarbonate is about 95 wt-% sodium bicarbonate and about 5 wt-% hydroxypropyl cellulose. In some cases, the directly compressible sodium bicarbonate comprises about 5 wt-% to about 10 wt-% pregelatinized starch. In some cases, the binder is hydroxypropyl cellulose and is present in an amount of about 3 wt-%. In some cases, disintegrant is croscarmellose sodium and is present in an amount of about 3 wt-%. In some cases, the lubricant is sodium stearyl fumarate and is present in an amount of about 0.5 wt-% to about 5 wt-%. In some cases, the directly compressible sodium bicarbonate is a combination of sodium bicarbonate and hydroxypropyl cellulose.

Described herein are pharmaceutical compositions in a tablet dosage form comprising: (a) about 20 mg to about 80 mg of a proton pump inhibitor selected from omeprazole and esomeprazole or a pharmaceutically acceptable salt, solvate or polymorph thereof; (b) about 400 mgs to about 1,400 mgs of directly compressible sodium bicarbonate; (c) about 2 wt-% to about 8 wt-% of a disintegrant; (d) about 3 wt-% to about 10 wt-% of a binder; and (e) about 0.5 wt-% and about 3 wt-% of a lubricant. In some cases, the tablet achieves an in vitro initial rise in pH within about 4 minutes. In some cases, the tablet achieves an in vitro initial rise in pH to at least about 4 within about 4 minutes. In some cases, upon administration to a fasted subject, the tablet provides a Tmax between about 30 minutes and about 45 minutes on Day 1. In some cases, upon administration to a fasted subject, the tablet provides a Tmax of about 45 minutes on Day 7. In some cases, the binder is hydroxypropyl cellulose and is present in an amount of about 3 wt-%. In some cases, the disintegrant is croscarmellose sodium and is present in an amount of about 3 wt-%. In some cases, the lubricant is sodium stearyl fumarate and is present in an amount of about 0.5 wt-% to about 5 wt-%. In some cases, the directly compressible sodium bicarbonate is a combination of sodium bicarbonate and hydroxypropyl cellulose. In some cases, the directly compressible sodium bicarbonate comprises between about 90-98 wt-% sodium bicarbonate and about 2-10 wt-% hydroxypropyl cellulose. In some cases, the directly compressible sodium bicarbonate is about 97 wt-% sodium bicarbonate and about 3 wt-% hydroxypropyl cellulose. In some cases, the directly compressible sodium bicarbonate is about 95 wt-% sodium bicarbonate and about 5 wt-% hydroxypropyl cellulose.

Described herein are pharmaceutical compositions comprising: (1) an immediate release portion of the composition comprising: (a) about 20 mgs to about 100 mgs of at least one acid labile bicyclic-aryl-imidazole proton pump inhibiting agent; and (b) at least one antacid in an amount sufficient to increase gastric fluid pH to a pH that prevents acid degradation of at least some of the proton pump inhibitor in the gastric fluid; wherein the antacid comprises at least about 400 mgs of directly compressible NaHC03; and (2) a sustained release portion of the composition comprising: (a) about 20 mgs to about 100 mgs of at least one acid labile bicyclic-aryl- imidazole proton pump inhibiting agent; and (b) about 10-80 wt-% of at least one slowly soluble polymer or a combination of slowly soluble polymers; wherein upon administration to a subject, a measurable serum level of the PPI is achieved for more than about 4 hours. In some cases, the composition is a tablet and the tablet achieves a hardness of 10-20 kP with less than 10,000 lbs of force. In some cases, the dosage form is a tablet. In some cases, the dosage form is a multilayer tablet. In some cases, the dosage form is a caspsule containing mini-tablets. In some cases, the dosage form is a capsule containing mini-tablets and powder. In various cases, wherein upon administration to a subject, the measurable serum level of the PPI is achieved for more than about 6 hours. In some cases, upon administration to a subject, the measurable serum level of the PPI is achieved for more than about 8 hours. In some cases, upon administration to a subject, the measurable serum level of the PPI is achieved for more than about 10 hours. In some cases, upon administration of the composition the Tmax of the composition is within about 60 minutes. In some cases, the polymer is selected from a cellulose ether or polyethylene oxide. In some cases, polymer is hydroxypropyl cellulose, hydroxypropyl methyl cellulose, or hydroxyethyl cellulose. In some cases, at least about 70% of the proton pump inhibitor in the immediate release portion of the composition is released within about 1 hour and less than about 80% of the proton pump inhibitor in the sustained release portion of the composition is released within 2 hours *in vitro.* In some cases, less than about 75% of the proton pump inhibitor in the sustained release portion of the composition is released within 4 hours *in vitro.* In some cases, less than about 75% of the proton pump inhibitor in the sustained release portion of the composition is released within 8 hours *in vitro.* In some cases, at least about 70% of the proton pump inhibitor in the immediate release portion of the composition is released within about 30 minutes *in vitro.*

### BRIEF DESCRIPTION OF THE DRAWINGS

T he invention is set forth in the appended claims. A better understanding of the features and advantages of the present invention will be obtained by reference to the following detailed description, and accompanying drawings and figures, which set forth illustrative cases described herein.

### FIGURES

In order that the invention may be more fully understood, it will now be described, by way of example, with reference to the accompanying drawing in which:
Fig. 1A is a graph demonstrating the delay in realization of maximum pH in capsules containing magnesium stearate lubricant vs. no lubricant. The 40 mg and 20 mg formulations for the omeprazole capsules containing magnesium stearate lubricant are listed in Table 1A1 and Table 1A2.
Fig. 1B demonstrates the Kinetic Stomach Model used to measure the impact on the in vitro pH of the pharmaceutical formulations. The procedure used to measure antacid performance in this model is described in Example 1.
Fig. 2 is a graphical representation comparing the pH profiles of omeprazole capsules containing magnesium stearate, sodium stearyl fumarate, and no lubricant. The 40 mg and 20 mg formulations for the omeprazole capsules containing magnesium stearate lubricant are listed in Table 1A1 and Table 1A2, and those formulations for sodium stearyl fumarate are listed in Tables 1A3 and 1A4.
Fig. 3 is a graph illustrating the pH profiles for two different types of lubricants at pH 1.4 and 4.2: magnesium stearate and sodium stearyl fumarate.
Fig. 4 is an in vivo comparison of the PK, pH, and aspirate profiles of omeprazole capsules containing magnesium stearate (i.e. the currently marketed Zegerid® capsules). The capsule formulation is listed in Table 1A1).
Fig. 5 is an in vivo comparison of the PK, pH, and aspirate profiles of omeprazole capsules containing sodium stearyl fumarate (i.e. reformulated Zegerid® capsules). The capsule formulation is listed in Table 1A3.
Fig. 6 is a pH profile of omeprazole capsules containing sodium stearyl fumarate (i.e. reformulated Zegerid® capsules) and those containing magnesium stearate lubricant (i.e. currently marketed Zegerid® capsule) once encapsulated on a high speed automatic encapsulator.
Fig. 7 is a graphic illustrating the comparative particle size distribution of sodium bicarbonate solutions with 5% HPC and 3% HPC from a 10% wt-% HPC solution of the formulations listed in Table 6A and Table 6B.
Fig. 8 is a graph that demonstrates the comparative sodium bicarbonate particle size distribution for five fluid bed trials coated with 3% HPC, prepared from a 7.5 w/w% solution.
Fig. 9 graphically illustrates the dissolution profiles of the immediate release/sustained release formulations described in Tables 9A1, 9A2, 9A4, 9A5, 9A8, and 9A9 and capsule formulation listed in Table 1A3.
Fig. 10 illustrates the pH profiles for 3% HPC compressible sodium bicarbonate in the following antacid strengths: 13 mEq, 15 mEq, and 17 mEq as listed in Tables 2A16, 2A17, and 2A18.
Fig. 11 illustrates a comparison in the pH profiles between capsules with magnesium stearate and capsules with sodium stearyl fumarate formulated pursuant to Table 2A4.
Fig. 12 illustrates a comparison in the pH profiles of coated and uncoated sodium bicarbonate pursuant to the formulation #3 listed in Table 2A10.
Fig. 13 illustrates a comparison in the pH profiles of coated and uncoated sodium bicarbonate pursuant to the formulation #4 listed in Table 2A10.
Fig. 14 illustrates a comparison in the pH profiles of omeprazole caplets with a magnesium stearate lubricant formulated pursuant to the formulation listed in Table 2A28 and Table 2A29.
Fig. 15 illustrates the pH profile of omeprazole caplets formulated pursuant to the formulation listed in Table 2A32.
Fig. 16 illustrates the pH profile of omeprazole caplets formulated pursuant to the formulation listed in Table 2A33.

### DETAILED DESCRIPTION

The present disclosure is illustrated herein with particular reference to omeprazole, hydroxyomeprazole, esomeprazole, tenatoprazole, lansoprazole, pantoprazole, rabeprazole, dontoprazole, habeprazole, periprazole, ransoprazole, pariprazole, or leminoprazole.

### Certain Terminology

To more readily facilitate an understanding of the invention and its preferred cases, the meanings of terms used herein will become apparent from the context of this specification in view of common usage of various terms and the explicit definitions of other terms described in the glossary below or in the ensuing description.

As used herein, the terms "comprising," "including," and "such as" are used in their open, nonlimiting sense.

The term "about" is used synonymously with the term "approximately." As one of ordinary skill in the art would understand, the exact boundary of "about" will depend on the component of the composition. Illustratively, the use of the term "about" indicates that values slightly outside the cited values, i.e., plus or minus 0.1% to 10%, which are also effective and safe.

As used herein, the phrase "acid-labile pharmaceutical agent" refers to any pharmacologically active drug subject to acid catalyzed degradation.

"Anti-adherents," "glidants," or "anti-adhesion" agents prevent components of the formulation from aggregating or sticking and improve flow characteristics of a material. These compounds include, e.g., colloidal silicon dioxide such as Cab-o-sil®; tribasic calcium phosphate, talc, corn starch, DL-leucine, sodium lauryl sulfate, magnesium stearate, calcium stearate, sodium stearate, kaolin, and micronized amorphous silicon dioxide (Syloid®) and the like.

"Antioxidants" include, e.g., butylated hydroxytoluene (BHT), sodium ascorbate, and tocopherol.

"Binders" impart cohesive qualities and include, *e.g.,* alginic acid and salts thereof; cellulose derivatives such as carboxymethylcellulose, methylcellulose (e.g., Methocel®), hydroxypropylmethylcellulose, hydroxyethylcellulose, hydroxypropylcellulose (e.g., Klucel®), ethylcellulose (e.g., Ethocel®), and microcrystalline cellulose (e.g., Avicel®); microcrystalline dextrose; amylose; magnesium aluminum silicate; polysaccharide acids; bentonites; gelatin; polyvinylpyrrolidone/vinyl acetate copolymer; crospovidone; povidone; starch; pregelatinized starch; tragacanth, dextrin, a sugar, such as sucrose *(e.g.,* Dipac®), glucose, dextrose, molasses, mannitol, sorbitol, xylitol *(e.g.,* Xylitab®), and lactose; a natural or synthetic gum such as acacia, tragacanth, ghatti gum, mucilage ofisapol husks, polyvinylpyrrolidone *(e.g.,* Polyvidone® CL, Kollidon® CL, Polyplasdone® XL-10), larch arabogalactan, Veegum®, polyethylene glycol, waxes, sodium alginate, and the like.

"Bioavailability" refers to the extent to which an active moiety, e.g., drug, prodrug, or metabolite, is absorbed into the general circulation and becomes available at the site of drug action in the body. Thus, a proton pump inhibitor administered through IV is 100% bioavailable. "Oral bioavailability" refers to the extent to which the proton pump inhibitor (or other active moiety) is absorbed into the general circulation and becomes available at the site of drug action in the body when the pharmaceutical composition is taken orally.

The term "bioequivalence" or "bioequivalent" means that two drug products do not differ significantly when the two products are administered at the same dose under similar conditions. A product can be considered bioequivalent to a second product if there is no significant difference in the rate and extent to which the active ingredient or active moiety becomes available at the site of drug action when the product is administered at the same molar dose as the second product under similar conditions in an appropriately designed study. Two products with different rates of absorption can be considered equivalent if the difference in the rate at which the active ingredient or moiety becomes available at the site of drug action is intentional and is reflected in the proposed labeling, is not essential to the attainment of effective body drug concentrations on chronic use, and is considered medically insignificant for the drug. Bioequivalence can be assumed when, for example, the 90% confidence interval ranges between 80% and 125% for the target parameters *(e.g.,* Cₘₐₓ and AUC).

"Carrier materials" include any commonly used excipients in pharmaceutics and should be selected on the basis of compatibility with the proton pump inhibitor and the release profile properties of the desired dosage form. Exemplary carrier materials include, e.g., binders, suspending agents, disintegration agents, filling agents, surfactants, solubilizers, stabilizers, lubricants, wetting agents, diluents, and the like. "Pharmaceutically compatible carrier materials" may comprise, e.g., acacia, gelatin, colloidal silicon dioxide, calcium glycerophosphate, calcium lactate, maltodextrin, glycerine, magnesium silicate, sodium caseinate, soy lecithin, sodium chloride, tricalcium phosphate, dipotassium phosphate, sodium stearoyl lactylate, carrageenan, monoglyceride, diglyceride, pregelatinized starch, and the like. See, e.g., Remington: The Science and Practice of Pharmacy, Twentieth Ed (Easton, Pa.: Mack Publishing Company, 2000); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins 1999).

The term "controlled release" includes any non-immediate release formulation, including but not limited to enteric-coated formulations and sustained release, delayed-release and pulsatile release formulations.

The term "delayed-release" includes any non-immediate release formulation, including but not limited to, film-coated formulations, enteric-coated formulations, encapsulated formulations, sustained release formulations and pulsatile release formulations. *See* Remington: The Science and Practice of Pharmacy, (20th Ed. 2000). As discussed herein, immediate and non-immediate release (or controlled release) can be defined kinetically by reference to the following equation:

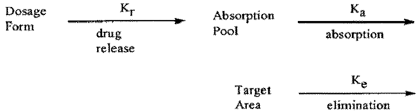

The absorption pool represents a solution of the drug administered at a particular absorption site, and Kᵣ. Kₐ, and Kₑ are first-order rate constants for: (1) release of the drug from the formulation; (2) absorption; and (3) elimination, respectively. For immediate release dosage forms, the rate constant for drug release Kᵣ is generally equal to or greater than the absorption rate constant Kₐ. For controlled release formulations, the opposite is generally true, that is, Kᵣ << Kₐ, such that the rate of release of drug from the dosage form is the rate- limiting step in the delivery of the drug to the target area.

A "derivative" is a compound that is produced from another compound of similar structure by the replacement of substitution of an atom, molecule or group by another suitable atom, molecule or group. For example, one or more hydrogen atom of a compound may be substituted by one or more alkyl, acyl, amino, hydroxyl, halo, haloalkyl, aryl, heteroaryl, cycloaolkyl, heterocycloalkyl, or heteroalkyl group to produce a derivative of that compound

"Diffusion facilitators" and "dispersing agents" include materials that control the diffusion of an aqueous fluid through a coating. Exemplary diffusion facilitators/dispersing agents include, e.g., hydrophilic polymers, electrolytes, Tween® 60 or 80, PEG and the like. Combinations of one or more erosion facilitator with one or more diffusion facilitator can also be used in the present invention.

The term "disintegrate" includes both the dissolution and dispersion of the dosage form when contacted with gastric fluid. "Disintegration agents" facilitate the breakup or disintegration of a substance. Examples of disintegration agents include a starch, e.g., a natural starch such as com starch or potato starch, a pregelatinized starch such as National 1551 or Amijel®, or sodium starch glycolate such as Promogel® or Explotab®; a cellulose such as a wood product, methylcrystalline cellulose, *e.g.,* Avicel®, Avicel® PH101, Avicel® PH102, Avicel® PH105, Elcema® PIOO, Emcocel®, Vivacel®, Ming Tia®, and Solka-Floc®, methylcellulose, croscarmellose, or a cross-linked cellulose, such as cross-linked sodium carboxymethylcellulose (Ac-Di-Sol®), cross-linked carboxymethylcellulose, or cross-linked croscarmellose; a cross-linked starch such as sodium starch glycolate; a cross-linked polymer such as crospovidone; a cross-linked polyvinylpyrrolidone; alginate such as alginic acid or a salt of alginic acid such as sodium alginate; a clay such as Veegum® HV (magnesium aluminum silicate); a gum such as agar, guar, locust bean, Karaya, pectin, or tragacanth; sodium starch glycolate; bentonite; a natural sponge; a surfactant; a resin such as a cation-exchange resin; citrus pulp; sodium lauryl sulfate; sodium lauryl sulfate in combination starch; and the like.

"Drug absorption" or "absorption" refers to the process of movement from the site of administration of a drug toward the systemic circulation, e.g., into the bloodstream of a subject.

An "enteric coating" is a substance that remains substantially intact in the stomach but dissolves and releases the drug once the small intestine is reached. Generally, the enteric coating comprises a polymeric material that prevents release in the low pH environment of the stomach but that ionizes at a slightly higher pH, typically a pH of 4 or 5, and thus dissolves sufficiently in the small intestines to gradually release the active agent therein.

The "enteric form of the proton pump inhibitor" is intended to mean that some or most of the proton pump inhibitor has been enterically coated to ensure that at least some of the drug is released in the proximal region of the small intestine (duodenum), rather than the acidic environment of the stomach.

"Fasting adult human subject" or "fasting subject" refers to, for example, any patient who has abstained from food for a period of time, *e.g.,* a patient who has not ingested a meal overnight *(e.g.,* 8 hours), a patient who has not ingested a meal in at least two hours, a patient with an empty stomach who is not suffering any meal- related symptoms that can be treated with a proton pump inhibitor, or any patient who has not ingested a meal such that the most recently ingested meal is digested and the patient is not suffering from any meal-related symptoms.

"Fed adult human subject" or "fed subject" refers to, for example, a patient who is initiating ingestion of a meal, a patient who has initiated ingestion of a meal a short time before administration (e.g., at about 10 minutes before, at about 30 minutes before, at about 45 minutes before, at about 60 minutes before, or at about 90 minutes before).

"Flavoring agents" or "sweeteners" useful in the pharmaceutical compositions of the present invention include, *e.g.,* acacia syrup, acesulfame K, alitame, anise, apple, aspartame, banana, Bavarian cream, berry, black currant, butterscotch, calcium citrate, camphor, caramel, cherry, cherry cream, chocolate, cinnamon, bubble gum, citrus, citrus punch, citrus cream, cotton candy, cocoa, cola, cool cherry, cool citrus, cyclamate, cylamate, dextrose, eucalyptus, eugenol, fructose, fruit punch, ginger, glycyrrhetinate, glycyrrhiza (licorice) syrup, grape, grapefruit, honey, isomalt, lemon, lime, lemon cream, monoammonium glyrrhizinate (MagnaSweet®), maltol, mannitol, maple, marshmallow, menthol, mint cream, mixed berry, neohesperidine DC, neotame, orange, pear, peach, peppermint, peppermint cream, Prosweet® Powder, raspberry, root beer, rum, saccharin, safrole, sorbitol, spearmint, spearmint cream, strawberry, strawberry cream, stevia, sucralose, sucrose, sodium saccharin, saccharin, aspartame, acesulfame potassium, mannitol, talin, sylitol, sucralose, sorbitol, Swiss cream, tagatose, tangerine, thaumatin, tutti fruitti, vanilla, walnut, watermelon, wild cherry, wintergreen, xylitol, or any combination of these flavoring ingredients, *e.g.,* anise-menthol, cherry-anise, cinnamon-orange, cherry- cinnamon, chocolate-mint, honey-lemon, lemon-lime, lemon-mint, menthol-eucalyptus, orange-cream, vanilla- mint, and mixtures thereof.

The phrase "gastrointestinal disorder" or "gastrointestinal disease" refers generally to a disorder or disease that occurs in a mammal due to an imbalance between acid and pepsin production, called aggressive factors, and mucous, bicarbonate, and prostaglandin production, called defensive factors. In mammals, such disorders or diseases include, but are not limited to, duodenal ulcer, gastric ulcer, acid dyspepsia, gastroesophageal reflux disease (GERD), severe erosive esophagitis, poorly responsive symptomatic gastroesophageal reflux disease, heartburn, other esophageal disorders, irritable bowel syndrome, nocturnal acid breakthrough and a gastrointestinal pathological hypersecretory condition such as Zollinger Ellison Syndrome. Treatment of these conditions is accomplished by administering to a subject a therapeutically effective amount of a pharmaceutical composition according to the present invention.

The phrase "gastrointestinal fluid" or "gastric fluid" refers to the fluid of stomach secretions of a subject or the equivalent thereof. An equivalent of stomach secretion includes, for example, an *in vitro* fluid having a similar content and/or pH as the stomach secretions. The content and pH of a particular stomach secretion is generally subject specific, and depends upon, among other things, the weight, sex, age, diet, or health of a particular subject. These particular stomach secretions can, for example, be mimicked or replicated by those skilled in the art, for example, those found in *in vitro* models used to study the stomach. One such model is commonly known as the "Kinetic Acid Neutralization Model," and can be used to experimentally study or determine release kinetics (for example, immediate release versus control release) of a component of the compositions of the present invention under predetermined experimental conditions; or acid degradation of a pharmaceutical agent of the compositions herein described under predetermined experimental conditions.

"Half-life" refers to the time required for the plasma drug concentration or the amount in the body to decrease by 50% from its maximum concentration.

The term "immediate release" is intended to refer to any PPI formulation in which all or part of the PPI is in solution either before administration or immediately (i.e., within about 30 minutes) after administration. For example, with an "immediate release" formulation, oral administration results in immediate release of the agent from the composition into gastric fluid. For delayed-release formulations, the opposite is generally true. The rate of release of drug from the dosage form is the rate-limiting step in the delivery of the drug to the target area. In some cases, the delayed release formulation is an enteric coated formulation.

"Lubricants" are compounds which prevent, reduce or inhibit adhesion or friction of materials. Exemplary lubricants include, e.g., stearic acid; calcium hydroxide, talc; a hydrocarbon such as mineral oil, or hydrogenated vegetable oil such as hydrogenated soybean oil (Sterotex®), Lubritab®, Cutina®; higher fatty acids and their alkali-metal and alkaline earth metal salts, such as aluminum, calcium, magnesium, zinc, stearic acid, sodium stearates, glycerol, talc, waxes, Stearowet®, boric acid, sodium acetate, leucine, a polyethylene glycol or a methoxypolyethylene glycol such as Carbowax™, sodium oleate, glyceryl behenate (Compitrol888®), glyceryl palmitostearate (Precirol®), colloidal silica such as Syloid™, Carb-O-Sil®, a starch such as com starch, silicone oil, a surfactant, and the like. Hydrophilic lubricants include, e.g., sodium stearyl fumerate (currently marketed under the trade name PRUV®), polyethylene glycol (PEG), magnesium lauryl sulfate, sodium lauryl sulfate (SLS), sodium benzoate, sodium chloride, and the like.

The term "measurable serum concentration" means the serum concentration (typically measured in mg, µg, or ng of therapeutic agent per ml, dl, or 1 of blood serum) of a therapeutic agent absorbed into the bloodstream after administration. One of ordinary skill in the art would be able to measure the serum concentration or plasma concentration of a proton pump inhibitor or a prokinetic agent. See, e.g., Gonzalez H. et al., J. Chromatogr. B. Analyt. Technol. Biomed. Life Sci., vol. 780, pp 459-65, (Nov. 25, 2002).

The term "pharmaceutically acceptable" is used adjectivally herein to mean that the modified noun is appropriate for use in a pharmaceutical product.

"Pharmacodynamics" refers to the factors which determine the biologic response observed relative to the concentration of drug at a site of action.

"Pharmacokinetics" refers to the factors which determine the attainment and maintenance of the appropriate concentration of drug at a site of action.

"Plasma concentration" refers to the concentration of a substance in blood plasma or blood serum of a subject. It is understood that the plasma concentration of a therapeutic agent may vary many-fold between subjects, due to variability with respect to metabolism of therapeutic agents. In accordance with one aspect described herein, the plasma concentration of a proton pump inhibitors may vary from subject to subject. Likewise, values such as maximum plasma concentration (Cmax) or time to reach maximum serum concentration (Tmax), or area under the serum concentration time curve (AUC) may vary from subject to subject. Due to this variability, the amount necessary to constitute "a therapeutically effective amount" of proton pump inhibitor, nonsteroidal antiinflammatory drug, or other therapeutic agent, may vary from subject to subject. It is understood that when mean plasma concentrations are disclosed for a population of subjects, these mean values may include substantial variation.

"Plasticizers" are compounds used to soften the microencapsulation material or film coatings to make them less brittle. Suitable plasticizers include, e.g., polyethylene glycols such as PEG 300, PEG 400, PEG 600, PEG 1450, PEG 3350, and PEG 800, stearic acid, propylene glycol, oleic acid, and triacetin.

The term "prevent" or "prevention," in relation to a gastrointestinal disorder or disease, means no gastrointestinal disorder or disease development if none had occurred, or no further gastrointestinal disorder or disease development if there had already been development of the gastrointestinal disorder or disease. Also considered is the ability of one to prevent some or all of the symptoms associated with the gastrointestinal disorder or disease.

A "prodrug" refers to a drug or compound in which the pharmacological action results from conversion by metabolic processes within the body. Prodrugs are generally drug precursors that, following administration to a subject and subsequent absorption, are converted to an active, or a more active species via some process, such as conversion by a metabolic pathway. Some prodrugs have a chemical group present on the prodrug which renders it less active and/or confers solubility or some other property to the drug. Once the chemical group has been cleaved and/or modified from the prodrug the active drug is generated. Prodrugs may be designed as reversible drug derivatives, for use as modifiers to enhance drug transport to site-specific tissues. The design of prodrugs to date has been to increase the effective water solubility of the therapeutic compound for targeting to regions where water is the principal solvent. See, e.g., Fedorak et al., Am. J. Physiology, 269:G210-218 (1995); McLoed et al., Gastroenterol., 106:405-413 (1994); Hochhaus et al., Biomed. Chrom., 6:283-286 (1992); J. Larsen and H. Bundgaard, Int. J. Pharmaceutics, 37, 87 (1987); J. Larsen et al., Int. J. Pharmaceutics, 47, 103 (1988); Sinkula et al., J. Pharm. Sci., 64:181-210 (1975); T. Higuchi and V. Stella, Pro-drugs as Novel Delivery Systems, Vol. 14 of the A.C.S. Symposium Series; and Edward B. Roche, Bioreversible Carriers in Drug Design, American Pharmaceutical Association and Pergamon Press, 1987.

"Serum concentration" refers to the concentration of a substance such as a therapeutic agent, in blood plasma or blood serum of a subject. It is understood that the serum concentration of a therapeutic agent may vary many-fold between subjects, due to variability with respect to metabolism of therapeutic agents. In accordance with one aspect described herein, the serum concentration of a proton pump inhibitors and/or prokinetic agent may vary from subject to subject. Likewise, values such as maximum serum concentration (Cmax) or time to reach maximum serum concentration (Tmax), or total area under the serum concentration time curve (AUC) may vary from subject to subject. Due to this variability, the amount necessary to constitute "a therapeutically effective amount" of proton pump inhibitor, prokinetic agent, or other therapeutic agent, may vary from subject to subject. It is understood that when mean serum concentrations are disclosed for a population of subjects, these mean values may include substantial variation.

The term "sustained release" is used in its conventional sense to refer to a drug formulation that provides for gradual release of a drug over an extended period of time, and, may sometimes, although not necessarily, result in substantially constant blood levels of a drug over an extended time period.

The terms "therapeutically effective amount" and "effective amount" in relation to the amount of proton pump inhibiting agent mean, consistent with considerations known in the art, the amount of proton pump inhibiting agent effective to elicit a pharmacologic effect or therapeutic effect (including, but not limited to, raising of gastric pH, raising pH in esophagus, reducing gastrointestinal bleeding, reducing or preventing gastric ulcers, reducing or preventing erosion of the esophagus, reducing in the need for blood transfusion, improving survival rate, more rapid recovery, H⁺, K⁺-ATPase inhibition or improvement or elimination of symptoms, and other indicators as are selected as appropriate measures by those skilled in the art), without undue adverse side effects. "Effective amount" in the context of a buffering agent means an amount sufficient to prevent the acid degradation of the PPI, in whole or in part, either *in vivo* or *in vitro.*

The term "treat" or "treatment" as used herein refers to any treatment of a disorder or disease associated with gastrointestinal disorder, and includes, but is not limited to, preventing the disorder or disease from occurring in a mammal which may be predisposed to the disorder or disease, but has not yet been diagnosed as having the disorder or disease; inhibiting the disorder or disease, for example, arresting the development of the disorder or disease; relieving the disorder or disease, for example, causing regression of the disorder or disease; or relieving the condition caused by the disease or disorder, for example, stopping the symptoms of the disease or disorder.

### Proton Pump Inhibitors

The terms "proton pump inhibitor," "PPI," and "proton pump inhibiting agent" can be used interchangeably to describe any acid labile pharmaceutical agent possessing pharmacological activity as an inhibitor of H+/K+-ATPase. A proton pump inhibitor may, if desired, be in the form of free base, free acid, salt, ester, hydrate, anhydrate, amide, enantiomer, isomer, tautomer, prodrug, polymorph, derivative, or the like, described that the free base, salt, ester, hydrate, amide, enantiomer, isomer, tautomer, prodrug, or any other pharmacologically suitable derivative is therapeutically active.

In various cases, the proton pump inhibitor can be a substituted bicyclic aryl-imidazole, wherein the aryl group can be, e.g., a pyridine, a phenyl, or a pyrimidine group and is attached to the 4- and 5- positions of the imidazole ring. Proton pump inhibitors comprising a substituted bicyclic aryl-imidazoles include, but are not limited to, omeprazole, hydroxyomeprazole, esomeprazole, lansoprazole, pantoprazole, rabeprazole, dontoprazole, habeprazole, pariprazole, tenatoprazole, ransoprazole, pariprazole, leminoprazole, or a free base, free acid, salt, hydrate, ester, amide, enantiomer, isomer, tautomer, polymorph, prodrug, or derivative thereof. See, e.g., The Merck Index, Merck & Co. Rahway, N.J. (2001). Other substituted bicyclic aryl-imidazole compounds as well as their salts, hydrates, esters, amides, enantiomers, isomers, tautomers, polymorphs, prodrugs, and derivatives may be prepared using standard procedures known to those skilled in the art of synthetic organic chemistry. See, e.g., March, Advanced Organic Chemistry: Reactions, Mechanisms and Structure, 4th Ed. (New York: Wiley-Interscience, 1992); Leonard et al., Advanced Practical Organic Chemistry (1992); Howarth et al., Core Organic Chemistry (1998); and Weisermel et al., Industrial Organic Chemistry (2002).

### Further Forms of the Proton Pump Inhibitors

### Isomers

The proton pump inhibitors useful in the composition described herein may exist as geometric isomers. The proton pump inhibitors useful in the invention may possess one or more double bonds. The proton pump inhibitors useful in the invention include all cis, trans, syn, anti, entgegen (E), and zusammen (Z) isomers as well as the corresponding mixtures thereof. In some situations, the proton pump inhibitors useful in the invention may exist as tautomers. The proton pump inhibitors useful in the invention include all possible tautomers within the formulas described herein.

The proton pump inhibitors useful in the composition may possess one or more chiral centers and each center may exist in the R or S configuration. The proton pump inhibitors useful in the invention include all diastereomeric, enantiomeric, and epimeric forms as well as the corresponding mixtures thereof. In additional cases of the compounds and methods described herein, mixtures of enantiomers and/or diastereoisomers, resulting from a single preparative step, combination, or interconversion may also be useful for the applications described herein.

In some cases, the proton pump inhibitors described herein can be prepared as their individual stereoisomers by reacting a racemic mixture of the compound with an optically active resolving agent to form a pair of diastereoisomeric compounds or complexes, separating the diastereomers and recovering the optically pure enantiomers. While resolution of enantiomers can be carried out using covalent diastereomeric derivatives of the compounds described herein, dissociable complexes are preferred (e.g., crystalline diastereomeric salts). Diastereomers have distinct physical properties (e.g., melting points, boiling points, solubilities, reactivity, etc.) and can be readily separated by taking advantage of these dissimilarities. The diastereomers can be separated by chromatography, or preferably, by separation/resolution techniques based upon differences in solubility. The single enantiomer of high optical purity (ee>90%) is then recovered, along with the resolving agent, by any practical means that would not result in racemization. A more detailed description of the techniques applicable to the resolution of stereoisomers of compounds from their racemic mixture can be found in Jean Jacques, Andre Collet, Samuel H. Wilen, "Enantiomers, Racemates and Resolutions," John Wiley And Sons, Inc., 1981, herein incorporated by reference in its entirety.

"Tautomers" of substituted bicyclic aryl-imidazoles include, e.g., tautomers of omeprazole such as those described in U.S. Patent Nos.: 6,262,085; 6,262,086; 6,268,385; 6,312,723; 6,316,020; 6,326,384; 6,369,087; and 6,444,689; and U.S. Patent Publication No. 02/0156103.

An exemplary "isomer" of a substituted bicyclic aryl-imidazole is the isomer of omeprazole including but not limited to isomers described in: Oishi et al., Acta Cryst. (1989), C45, 1921-1923; U.S. Patent No. 6,150,380; U.S. Patent Publication No. 02/0156284; and PCT Publication No. WO 02/085889.

### Labeled compounds

It should be understood that the proton pump inhibitors described herein include their isotopically-labeled equivalents, including their use for treating disorders. For example, the invention provides for methods of treating diseases, by administering isotopically-labeled proton pump inhibitors. The isotopically- labeled proton pump inhibitors useful in the invention can be administered as pharmaceutical compositions. Thus, the proton pump inhibitors described herein also include their isotopically-labeled isomers, which are identical to those recited herein, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, sulfur, fluorine and chloride, such as 2H, 3H, 11C, 13C, 14C, 15N, 180, 170, 31P, 32P, 35S, 18F, and 36C1, respectively. The proton pump inhibitors described herein, pharmaceutically acceptable salts, esters, prodrugs, solvate, hydrates or derivatives thereof which contain the aforementioned isotopes and/or other isotopes of other atoms are within the scope of this invention. Certain isotopically-labeled compounds, for example those into which radioactive isotopes such as 3H and 14C are incorporated, are useful in drug and/or substrate tissue distribution assays. Tritiated, i.e., 3H and carbon-14, i.e., 14C, isotopes are particularly preferred for their ease of preparation and detectability. Further, substitution with heavier isotopes such as deuterium, i.e., 2H, can afford certain therapeutic advantages resulting from greater metabolic stability, for example increased in vivo half-life or reduced dosage requirements and, hence, may be preferred in some circumstances. Isotopically labeled compounds, pharmaceutically acceptable salts, esters, prodrugs, solvates, hydrates or derivatives thereof can generally be prepared by carrying out procedures used to make the proton pump inhibitor, by substituting a readily available

The proton pump inhibitors described herein may be labeled by other means, including, but not limited to, the use of chromophores or fluorescent moieties, bioluminescent labels, or chemiluminescent labels.

### Pharmaceutically acceptable salts

The proton pump inhibitors described herein may also exist as their pharmaceutically acceptable salts, which may also be useful for treating disorders. For example, described herein are methods of treating diseases, by administering pharmaceutically acceptable salts of the proton pump inhibitors described herein. The pharmaceutically acceptable salts can be administered as pharmaceutical compositions.

Thus, the proton pump inhibitors described herein can be prepared as pharmaceutically acceptable salts formed when an acidic proton present in the parent compound either is replaced by a metal ion, for example an alkali metal ion, an alkaline earth ion, or an aluminum ion; or coordinates with an organic base. Base addition salts can also be prepared by reacting the free acid form of the proton pump inhibitors with a pharmaceutically acceptable inorganic or organic base, including, but not limited to organic bases such as ethanolamine, diethanolamine, triethanolamine, tromethamine, N-methylglucamine, and the like and inorganic bases such as aluminum hydroxide, calcium hydroxide, potassium hydroxide, sodium carbonate, sodium hydroxide, and the like. In addition, the salt forms of the disclosed proton pump inhibitors can be prepared using salts of the starting materials or intermediates.

Further, the proton pump inhibitors described herein can be prepared as pharmaceutically acceptable salts formed by reacting the free base form of the compound with a pharmaceutically acceptable inorganic or organic acid, including, but not limited to, inorganic acids such as hydrochloric acid, hydrobromic acid, sulfuric acid, nitric acid, phosphoric acid metaphosphoric acid, and the like; and organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, Q-toluenesulfonic acid, tartaric acid, trifluoroacetic acid, citric acid, benzoic acid, 3-(4-hydroxybenzoyl)benzoic acid, cinnamic acid, mandelic acid, arylsulfonic acid, methanesulfonic acid, ethanesulfonic acid, 1,2-ethanedisulfonic acid, 2-hydroxyethanesulfonic acid, benzenesulfonic acid, 2-naphthalenesulfonic acid, 4-methylbicyclo-[2.2.2]oct-2-ene-1-carboxylic acid, glucoheptonic acid, 4,4 '-methylenebis-(3-hydroxy-2-ene-1-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, and muconic acid.

Salt forms of proton pump inhibiting agents include, but are not limited to: a sodium salt form such as esomeprazole sodium, omeprazole sodium, rabeprazole sodium, pantoprazole sodium; or a magnesium salt form such as esomeprazole magnesium or omeprazole magnesium, described in U.S. Patent No. 5,900,424; a calcium salt form; or a potassium salt form such as the potassium salt of esomeprazole, described in U.S. Patent Application No. 02/0198239 and U.S. Patent No. 6,511,996. Other salts of esomeprazole are described in U.S. 4,738,974 and U.S. 6,369,085. Salt forms of pantoprazole and lansoprazole are discussed in U.S. Pat. Nos. 4,758,579 and 4,628,098, respectively.

### Cocrystals and Solvates

The proton pump inhibitors described herein may also exist in various cocrystal forms, which may also be useful for treating disorders. For example, described herein are methods of treating diseases, by administering cocrystals of the proton pump inhibitors useful in the invention. The cocrystals can be administered as pharmaceutical compositions. Preferably the cocrystals are pharmaceutically acceptable cocrystals.

Cocrystals contain either stoichiometric or non-stoichiometric amounts of a material, and may be formed during the process of crystallization with pharmaceutically acceptable materials such as water, ethanol, and the like. Solvates are formed when the material is a solvent. Hydrates are formed when the solvent is water, or alcoholates are formed when the solvent is alcohol. Solvates of the proton pump inhibitors useful in the invention can be conveniently prepared or formed during the processes described herein. By way of example only, hydrates of the proton pump inhibitors useful in the invention can be conveniently prepared by recrystallization from an aqueous/organic solvent mixture, using organic solvents including, but not limited to, dioxane, tetrahydrofuran or methanol. In addition, the proton pump inhibitors described herein can exist in unsolvated as well as solvated forms. In general, the solvated forms are considered equivalent to the unsolvated forms of the proton pump inhibitors described herein.

### Polymorphs

The proton pump inhibitors described herein may also exist in various polymorphic states, all of which are herein contemplated, and which may also be useful for treating disorders. For example, described herein are methods of treating diseases, by administering polymorphs of the proton pump inhibitors useful in the invention. The various polymorphs can be administered as pharmaceutical compositions.

Thus, the proton pump inhibitors described herein include all their crystalline forms, known as polymorphs. Polymorphs include the different crystal packing arrangements of the same elemental composition of the compound. Polymorphs may have different X-ray diffraction patterns, infrared spectra, melting points, density, hardness, crystal shape, optical and electrical properties, stability, solvates and solubility. Various factors such as the recrystallization solvent, rate of crystallization, and storage temperature may cause a single crystal form to dominate.

Exemplary "polymorphs" include, but are not limited to, those described in PCT Publication No. WO 92/08716, and U.S. Patent Nos. 4,045,563; 4,182,766; 4,508,905; 4,628,098; 4,636,499; 4,689,333; 4,758,579; 4,783,974; 4,786,505; 4,808,596; 4,853,230; 5,026,560; 5,013,743; 5,035,899; 5,045,321; 5,045,552; 5,093,132; 5,093,342; 5,433,959; 5,464,632; 5,536,735; 5,576,025; 5,599,794; 5,629,305; 5,639,478; 5,690,960; 5,703,110; 5,705,517; 5,714,504; 5,731,006; 5,879,708; 5,900,424; 5,948,773; 5,997,903; 6,017,560; 6,123,962; 6,147,103; 6,150,380; 6,166,213; 6,191,148; 5,187,340; 6,268,385; 6,262,086; 6,262,085; 6,296,875; 6,316,020; 6,328,994; 6,326,384; 6,369,085; 6,369,087; 6,380,234; 6,428,810; 6,444,689; and 6,462,0577.

### Micronized Proton Pump Inhibitors

Particle size of the proton pump inhibitor can affect the solid dosage form in numerous ways. Since decreased particle size increases in surface area (S), the particle size reduction provides an increase in the rate of dissolution (dM/dt) as expressed in the Noyes-Whitney equation: dM/dt = dS / h(Cs-C); where M =mass of drug dissolved; t = time; D = diffusion coefficient of drug; S = effective surface area of drug particles; H= stationary layer thickness; Cs = concentration of solution at saturation; and C = concentration of solution at time t.

Because most proton pump inhibitors (such as omeprazole) have poor water solubility, to aid the rapid absorption of the drug product, various cases of the present invention use micronized proton pump inhibitors. In some cases, the average particle size of at least about 90% the micronized proton pump inhibitor is less than about 200 µm, 150 µm, 100 µm, 80 µm, 60 µm, 40 µm, or less than about 35 µm, or less than about 30 µm, or less than about 25 µm, or less than about 20 µm, or less than about 15 µm, or less than about 10 µm, or less than about 5 µm. In other cases, at least 80% of the micronized proton pump inhibitor has an average particle size of less than about 200 µm, 150 µm, 100 µm, 80 µm, 60 µm, 40 µm, or less than about 35 µm, or less than about 30 µm, or less than about 25 µm, or less than about 20 µm, or less than about 15 µm, or less than about 10 µm, or less than about 5 µm. In still other cases, at least 70% of the micronized proton pump inhibitor has an average particle size of less than about 200 µm, 150 µm, 100 µm, 80 µm, 60 µm, 40 µm, or less than about 35 µm, or less than about 30 µm, or less than about 25 µm, or less than about 20 µm, or less than about 15 µm, or less than about 10 µm, or less than about 5 µm.

In some cases, compositions are described wherein the micronized proton pump inhibitor is of a size which allows greater than 75% of the proton pump inhibitor to be released within about 90 minutes, or within about 75 minutes, or within about 60 minutes, or within about 45 minutes, within about 30 minutes, or within about 20 minutes, or within about 10 minutes, or within about 5 minutes of dissolution testing. In another case described herein, the micronized proton pump inhibitor is of a size which allows greater than 90% of the proton pump inhibitor to be released within about 90 minutes, or within about 75 minutes, or within about 60 minutes, or within about 45 minutes, within about 30 minutes, or within about 20 minutes, or within about 10 minutes, or within about 5 minutes of dissolution testing. *See* U.S. Application No. 10/893,092, filed July 16, 2004, which claims priority to U.S. Provisional Application No. 60/488,324 filed July 18, 2003.

### Antacids

The pharmaceutical composition described herein comprise one or more antacids. A class of antacids useful in the present invention include, *e.g.,* antacids possessing pharmacological activity as a weak base or a strong base. In one case, the antacid, when formulated or delivered with a proton pump inhibiting agent, functions to substantially prevent or inhibit the acid degradation of the proton pump inhibitor by gastrointestinal fluid for a period of time, e.g., for a period of time sufficient to preserve the bioavailability of the proton pump inhibitor administered.

Antacids suitable for the compositions herein includes one or more of, e.g., alkali metal (a Group IA metal including, but not limited to, lithium, sodium, potassium, rubidium, cesium, and francium) or alkaline earth metal (Group IIA metal including, but not limited to, beryllium, magnesium, calcium, strontium, barium, radium) carbonates, phosphates, bicarbonates, citrates, borates, acetates, phthalates, tartrate, succinates and the like, such as sodium or potassium phosphate, citrate, borate, acetate, bicarbonate and carbonate.

In various cases, an antacid includes, e.g., an amino acid, an alkali salt of an amino acid, aluminum hydroxide, aluminum hydroxide/magnesium carbonate/calcium carbonate co-precipitate, aluminum magnesium hydroxide, aluminum hydroxide/magnesium hydroxide co-precipitate, aluminum hydroxide/sodium bicarbonate co-precipitate, aluminum glycinate, calcium acetate, calcium bicarbonate, calcium borate, calcium carbonate, calcium citrate, calcium gluconate, calcium glycerophosphate, calcium hydroxide, calcium lactate, calcium phthalate, calcium phosphate, calcium succinate, calcium tartrate, dibasic sodium phosphate, dipotassium hydrogen phosphate, dipotassium phosphate, disodium hydrogen phosphate, disodium succinate, dry aluminum hydroxide gel, L-arginine, magnesium acetate, magnesium aluminate, magnesium borate, magnesium bicarbonate, magnesium carbonate, magnesium citrate, magnesium gluconate, magnesium hydroxide, magnesium lactate, magnesium metasilicate aluminate, magnesium oxide, magnesium phthalate, magnesium phosphate, magnesium silicate, magnesium succinate, magnesium tartrate, potassium acetate, potassium carbonate, potassium bicarbonate, potassium borate, potassium citrate, potassium metaphosphate, potassium phthalate, potassium phosphate, potassium polyphosphate, potassium pyrophosphate, potassium succinate, potassium tartrate, sodium acetate, sodium bicarbonate, sodium borate, sodium carbonate, sodium citrate, sodium gluconate, sodium hydrogen phosphate, sodium hydroxide, sodium lactate, sodium phthalate, sodium phosphate, sodium polyphosphate, sodium pyrophosphate, sodium sesquicarbonate, sodium succinate, sodium tartrate, sodium tripolyphosphate, synthetic hydrotalcite, tetrapotassium pyrophosphate, tetrasodium pyrophosphate, tripotassium phosphate, trisodium phosphate, and trometamol. (Based in part upon the list described in The Merck Index, Merck & Co. Rahway, N.J. (2001)). In addition, due to the ability of proteins or protein hydrolysates to react with stomach acids, they too can serve as antacids in the present invention. Furthermore, combinations of the above mentioned antacids can be used in the pharmaceutical formulations described herein.

The antacids useful in the compositions herein also include antacids or combinations of antacids that interact with HCl (or other acids in the environment of interest) faster than the proton pump inhibitor interacts with the same acids. When placed in a liquid phase, such as water, these antacids produce and maintain a pH greater than the pKa of the proton pump inhibitor.

In various cases, the antacid is selected from sodium bicarbonate, sodium carbonate, calcium carbonate, magnesium oxide, magnesium hydroxide, magnesium carbonate, aluminum hydroxide, and mixtures thereof.

In some cases, the antacid is present in the pharmaceutical formulations of the present invention in an amount greater than about 10 mEq of antacid. In other cases, the antacid is present in the pharmaceutical formulations described herein in an amount greater than about 15 mEq of antacid. In other cases, the antacid is present in the pharmaceutical formulations described herein in an amount greater than about 20 mEq of antacid.

In yet another case, the antacid is present in the pharmaceutical formulations described herein in an amount greater than about 25 mEq of antacid. In other cases, the antacid is present in the pharmaceutical formulations of the present invention in an amount greater than about 30 mEq. In some cases, the antacid is present in the pharmaceutical formulations described herein in an amount greater than about 35 mEq. In some cases, the antacid is present in the pharmaceutical formulations described herein in an amount greater than about 40 mEq.

In other cases, the antacid is present in the pharmaceutical formulations described herein in an amount from about 5 to about 50 mEq of antacid. In other cases, the antacid is present in the pharmaceutical formulations described herein in an amount from about 15 to about 40 mEq of antacid. In other cases, the antacid is present in the pharmaceutical formulations of the present invention in an amount from about 15 to about 30 mEq of antacid. In other cases, the antacid is present in the pharmaceutical formulations described herein in an amount from about 10 to about 20 mEq of antacid. In other cases, the antacid is present in the pharmaceutical formulations of the present invention in an amount from about 20 to about 30 mEq of antacid. In some cases, the antacid is present in the pharmaceutical formulations described herein in an amount of about 20 mEq to about 40 mEq.

In another case, the amount of antacid present in the pharmaceutical formulation is between 100 and 3500 mg. The amount of antacid present in the pharmaceutical formulation can be about 100 mgs, about 200 mgs, or about 300 mgs, or about 400 mgs, or about 500 mgs, or about 600 mgs, or about 700 mgs, or about 800 mgs, or about 900 mgs, or about 1000 mgs, or about 1100 mgs, or about 1200 mgs, or about 1300 mgs, or about 1400 mgs, or about 1500 mgs, or about 1600 mgs, or about 1700 mgs, or about 1800 mgs, or about 1900 mgs, or about 2000 mgs, or about 2100 mgs, or about 2200 mgs, or about 2300 mgs, or about 2400 mgs, or about 2500 mgs, or about 2600 mgs, or about 2700 mgs, or about 2800 mgs, or about 2900 mgs, or about 3000 mgs, or about 3200 mgs, or about 3500 mgs.

In various cases, the antacid is selected from sodium bicarbonate, sodium carbonate, calcium carbonate, magnesium oxide, magnesium hydroxide, magnesium carbonate, aluminum hydroxide, and mixtures thereof. In other cases, the antacid is present in the pharmaceutical formulations described herein in an amount greater than about 5 mEq of antacid. In other cases, the antacid is present in the pharmaceutical formulations described herein in an amount greater than about 7 mEq of antacid. In other cases, the antacid is present in the pharmaceutical formulations of the present invention in an amount greater than about 10 mEq of antacid. In other cases, the antacid is present in the pharmaceutical formulations described herein in an amount greater than about 15 mEq of antacid. In other cases, the antacid is present in the pharmaceutical formulations described herein in an amount greater than about 20 mEq of antacid.

In another case, the antacid comprises sodium bicarbonate in about 0.1 mEq/mg proton pump inhibitor to about 5 mEq/mg proton pump inhibitor. In yet another case, the antacid comprises a mixture of sodium bicarbonate and magnesium hydroxide, wherein the sodium bicarbonate and magnesium hydroxide are each present in about 0.1 mEq/mg proton pump inhibitor to about 5 mEq/mg proton pump inhibitor. In still another case, the antacid comprises a mixture of sodium bicarbonate, calcium carbonate, and magnesium hydroxide, wherein the sodium bicarbonate, calcium carbonate, and magnesium hydroxide are each present in about 0.1 mEq/mg proton pump inhibitor to about 5 mEq/mg of the proton pump inhibitor.

In another case, the antacid is present in the pharmaceutical formulations of the present invention in an amount of about 0.1 mEq to about 15 mEq/mg of proton pump inhibitor, or about 0.1 mEq/mg of proton pump inhibitor, or about 0.5 mEq/mg of proton pump inhibitor, or about 1 mEq/mg of proton pump inhibitor, or about 2 mEq/mg of proton pump inhibitor, or about 2.5 mEq/mg of proton pump inhibitor, or about 3 mEq/mg of proton pump inhibitor, or about 3.5 mEq/mg of proton pump inhibitor, or about 4 mEq/mg of proton pump inhibitor, or about 4.5 mEq/mg of proton pump inhibitor, or about 5 mEq/mg of proton pump inhibitor, or about 6 mEq/mg of proton pump inhibitor, or about 7 mEq/mg of proton pump inhibitor, or about 8 mEq/mg of proton pump inhibitor, or about 9 mEq/mg of proton pump inhibitor, or about 10 mEq/mg of proton pump inhibitor, or about 11 mEq/mg of proton pump inhibitor, or about 12 mEq/mg of proton pump inhibitor, or about 13 mEq/mg of proton pump inhibitor, or about 14 mEq/mg of proton pump inhibitor, or about 15 mEq/mg of proton pump inhibitor.

In one case, the antacid is present in the pharmaceutical formulations of the present invention in an amount of about 1 mEq to about 160 mEq per dose, or about 1 mEq, or about 5 mEq, or about 10 mEq, or about 15 mEq, or about 20 mEq, or about 25 mEq, or about 30 mEq, or about 35 mEq, or about 40 mEq, or about 45 mEq, or about 50 mEq, or about 60 mEq, or about 70 mEq, or about 80 mEq, or about 90 mEq, or about 100 mEq, or about 110 mEq, or about 120 mEq, or about 130 mEq, or about 140 mEq, or about 150 mEq, or about 160 mEq per dose.

In another case, the antacid is present in an amount of more than about 5 times, or more than about 10 times, or more than about 20 times, or more than about 30 times, or more than about 40 times, or more than about 50 times, or more than about 60 times, or more than about 70 times, or more than about 80 times, or more than about 90 times, or more than about 100 times the amount of the proton pump inhibiting agent on a weight to weight basis in the composition.

In various other cases described herein, the antacid is present in an amount of about 0.1 mEq/mg to about 5 mEq/mg of the proton pump inhibitor, or about 0.5 mEq/mg to about 3 mEq/mg of the proton pump inhibitor, or about 0.6 mEq/mg to about 2.5 mEq/mg of the proton pump inhibitor, or about 0.7 mEq/mg to about 2.0 mEq/mg of the proton pump inhibitor, or about 0.8 mEq/mg to about 1.8 mEq/mg of the proton pump inhibitor, or about 1.0 mEq/mg to about 1.5 mEq/mg of the proton pump inhibitor, or at least 0.5 mEq/mg of the proton pump inhibitor.

In some cases, if the antacid is a combination of two or more antacids, the combination comprises at least two non-amino acids, wherein the combination of at least two non-amino acids comprises substantially no aluminum hydroxide-sodium bicarbonate co-precipitate. In other cases, if the pharmaceutical composition comprises an amino acid, the total amount of antacid present in the pharmaceutical composition is less than about 5 mEq, or less than about 4 mEq, or less than about 3 mEq, or less than about 2 mEq. The phrase "amino acid antacid" as used herein includes amino acids, amino acid salts, and amino acid alkali salts. including: glycine, alanine, threonine, isoleucine, valine, phenylalanine, glutamic acid, asparagininic acid, lysine, aluminum glycinate and/or lysine glutamic acid salt, glycine hydrochloride, L-alanine, DL-alanine, L-threonine, DL-threonine, L-isoleucine, L-valine, L-phenylalanine, L-glutamic acid, L-glutamic acid hydrochloride, L-glutamic acid sodium salt, L-asparaginic acid, L-asparaginic acid sodium salt, L-lysine and L- lysine-L-glutamic acid salt. The term "non-amino acid antacid" as used herein includes antacids as defined herein above but does not include amino acid antacids.

Also described herein are pharmaceutical formulations comprising at least one soluble antacid. For example, in one case, the antacid is sodium bicarbonate and is present in about 0.1 mEq/mg proton pump inhibitor to about 5 mEq/mg proton pump inhibitor. In another case, the antacid is a mixture of sodium bicarbonate and magnesium hydroxide, wherein the sodium bicarbonate and magnesium hydroxide are each present in about 0.1 mEq/mg proton pump inhibitor to about 5 mEq/mg proton pump inhibitor. Yet in another case, the antacid is a mixture of sodium bicarbonate and magnesium oxide, wherein the sodium bicarbonate and magnesium oxide are each present in about 0.1 mEq/mg proton pump inhibitor to about 5 mEq/mg proton pump inhibitor. The term "soluble antacid" as used herein refers to an antacid that has a solubility of at least 25 mg/mL in water. In some cases, the solubility of the antacid is at least 50 mg/mL or 100 mg/mL in water. In other cases, the soluble antacid is sodium bicarbonate.

Also described herein are pharmaceutical formulations comprising at least one high efficiency antacid. A high efficiency antacid is one that can neutralize more than one acidic proton per molecule of antacid. Exemplary high efficiency antacids include aluminum magnesium hydroxide, aluminum hydroxide/magnesium hydroxide co-precipitate, aluminum hydroxide/sodium bicarbonate co-precipitate, aluminum glycinate, calcium acetate, calcium bicarbonate, calcium borate, calcium carbonate, calcium citrate, calcium gluconate, calcium glycerophosphate, calcium hydroxide, calcium lactate, calcium phthalate, calcium phosphate, calcium succinate, calcium tartrate, dibasic sodium phosphate, dipotassium hydrogen phosphate, dipotassium phosphate, disodium hydrogen phosphate, disodium succinate, dry aluminum hydroxide gel, , magnesium acetate, magnesium aluminate, magnesium borate, magnesium bicarbonate, magnesium carbonate, magnesium citrate, magnesium gluconate, magnesium hydroxide, magnesium lactate, magnesium metasilicate aluminate, magnesium oxide, magnesium phthalate, magnesium phosphate, magnesium silicate, magnesium succinate, magnesium tartrate, potassium carbonate, potassium borate, potassium phthalate, dibasic potassium phosphate, potassium polyphosphate, potassium pyrophosphate, potassium succinate, potassium tartrate, sodium borate, sodium citrate, sodium polyphosphate, sodium pyrophosphate, sodium sesquicarbonate, sodium tripolyphosphate, synthetic hydrotalcite, tetrapotassium pyrophosphate, tetrasodium pyrophosphate, tripotassium phosphate, and trisodium phosphate. In some cases, the high efficiency antacid is magnesium hydroxide, calcium carbonate, or magnesium oxide.

Spray dried antacids typically have a spherical particle shape. They aid with flowability and in achieving a homogeneous blend during the manufacturing process. In one case the antacid is spray dried with at least 15% of a material such as maltodextrin or starch. In still other case the antacid is spray dried with at least 10% of a material such as maltodextrin or starch. In yet another case the antacid is spray dried with at least 5% of a material such as maltodextrin or starch. In still other cases, the antacid is spray dried with between about 1% to about 10% of a material such as maltodextrin or starch. In yet other cases, the antacid is spray dried with about 5% of a material such as maltodextrin or starch.

### Particle Size of Antacids

Particle size of the antacid, especially that of an insoluble antacid, can affect the onset of in-vivo neutralization of the stomach acid. Since decreased particle size increases in surface area, the particle size reduction provides an increase in the rate of acid neutralization, leading to superior protection of PPI from gastric acid degradation. On the other hand, extremely fine particle size of the antacid will result in the powder mixture that is difficult to manufacture in commercial scale due to their poor flow and difficulties in processing (i.e., compression and encapsulation).

In some cases described herein, the antacid is a specific particle size. For example, the average particle size of the antacid may be no greater than 20 µm, or no greater than 30 µm, or no greater than 40 µm, or no greater than 50 µm, or no greater than 60 µm, or no greater than 70 µm, or no greater than 80 µm, or no greater than 90 µm or no greater than 100 µm in diameter. In various cases, at least about 70% of the antacid is no greater than 20 µm, or no greater than 30 µm, or no greater than 40 µm, or no greater than 50 µm, or no greater than 60 µm, or no greater than 70 µm, or no greater than 80 µm, or no greater than 90 µm or no greater than 100 µm in diameter. In other cases, at least about 85% of the antacid is no greater than 20 µm, or no greater than 30 µm, or no greater than 40 µm, or no greater than 50 µm, or no greater than 60 µm, or no greater than 70 µm, or no greater than 80 µm, or no greater than 90 µm or no greater than 100 µm in diameter.

In various cases described herein, some or all of the antacid is micronized. In some cases, particle size of at least 90% of antacid (D90) is less than about 300 µm, or less than about 250 µm, or less than about 200 µm, or less than about 150 µm, or less than about 100 µm. In other cases, at least 75% of the antacid (D75) has particle size of less than about 300 µm or less than about 250 µm, or less than about 200 µm, or less than about 150 µm, or less than about 100 µm. In still other cases, at least 50% of the antacid (D50) has particle size of less than about 300 µm, or less than about 250 µm, or less than about 200 µm, or less than about 150 µm, or less than about 100 µm.

### Compressible Sodium Bicarbonate

The pharmaceutical composition of the invention in tablet form, like other cases of the invention, comprises one or more antacids. A tablet *(i.e.,* caplet, chewable tablet, suspension tablet, etc.) formulation, in particular, must contain a sufficient level of antacid to neutralize stomach acid. The use of the antacid in the neutralization process prevents the degradation of omeprazole by allowing its subsequent absorption in the gastrointestinal tract.

In some cases, the formulations of the present invention contain compressible sodium bicarbonate. In certain cases, the amount of sodium bicarbonate is about 600 mg. In other cases, the amount of sodium bicarbonate is about 700 mg. In still other cases, the amount of sodium bicarbonate is about 800 mg, or about 900 mg, or about 1000 mg, or about 1100 mg, or about 1200 mg, or about 1300 mg, or about 1400 mg, or about 1500 mg, or about 1600 mg, or about 1700 mg, or about 1800 mg.

In other cases, the formulations contain a mixture of antacids, wherein one of the antacids is compressible sodium bicarbonate. In other cases, the compressible sodium bicarbonate and the second antacid are each coated with a suitable material including, but not limited to, HPC, pregelatinized starch, HPMC, etc.. In yet other cases, the sodium bicarbonate is not coated, but the second antacid is coated, for example, by using a method similar to the one described herein for coating sodium bicarbonate.

In various cases of the present invention, the compressible sodium bicarbonate, or other compressible antacid, comprises about 50% to 98% w/w of the formulation. In other cases, the compressible sodium bicarbonate makes up less than 60% w/w formulation, or about 60% w/w, or about 70% w/w, or about 80% w/w, or about 85% w/w, or about 88% w/w, or about 90% w/w, or about 93% w/w, or about 95% w/w, or about 97% w/w, or about 100% w/w formulation.

In various cases of the present invention, the sodium bicarbonate, or other antacid, is coated with an exemplary material useful for compressing the sodium bicarbonate. In these cases, the coating material may include, but is not limited to: hydroxypropyl cellulose ethers (HPC) such as Klucel® or Nisso HPC; low-substituted hydroxypropyl cellulose ethers (L-HPC); hydroxypropyl methyl cellulose ethers (HPMC) such as Seppifilm-LC, Pharmacoat®, Metolose SR, Methocel®-E, Opadry YS, PrimaFlo, Benecel MP824, and Benecel MP843; methylcellulose polymers such as Methocel®-A and Metolose®; Ethylcelluloses (EC) and mixtures thereof such as E461, Ethocel®, Aqualon®-EC, Surelease®; Polyvinyl alcohol (PVA) such as Opadry AMB; hydroxyethylcelluloses such as Natrosol®; carboxymethylcelluloses and salts of carboxymethylcelluloses (CMC) such as Aqualon®-CMC; polyvinyl alcohol and polyethylene glycol co-polymers such as Kollicoat IR®; monoglycerides (Myverol), triglycerides (KLX), polyethylene glycols, modified food starch, acrylic polymers and mixtures of acrylic polymers with cellulose ethers such as Eudragit® EPO, Eudragit® RD100, and Eudragit® EIOO; cellulose acetate phthalate; sepifilms such as mixtures of HPMC and stearic acid, cyclodextrins; and mixtures of these materials.

In other cases, the sodium bicarbonate is coated with pregelatinized starch, or hydroxypropyl methyl cellulose ethers (HPMC), or hydroxypropyl cellulose ethers (HPC), or mixtures of these materials.

In yet other cases of the present invention, the sodium bicarbonate, or other compressible antacid, is coated with an HPC solution. In certain cases described herein, the solution comprises about a 1% HPC coating, or about 2% HPC coating, or about 3% HPC coating, or about 4% HPC coating, or about 6% HPC coating, or about 7% HPC coating, or about 8% HPC coating, or about 9% HPC coating, or about 10% HPC coating, or greater than 10% HPC coating.

In other cases described herein with HPC coating, the coating solution comprises about a 0.5% to about 2.5% HPC coating, or about a a 1% to about 3% HPC coating, or about 1.5% to about 3.5% HPC coating, or about 2% to about 4% HPC coating, or about 2.5% to about 4.5% HPC coating, or about 3% to about 5% HPC coating, or about 3.5% to about 5.5% HPC coating, or about 4% to about 6% HPC coating, or about 4.5% to about 6.5% HPC coating, or about 5% to about 7% HPC coating, or about 5.5% to about 7.5% HPC coating, or about 6% to about 8% HPC coating, or about 6.5% to about 8.5% HPC coating, or about 7% to about 9% HPC coating, or about 7.5% to about 9.5% HPC coating, or about 8% to about 10% HPC coating.

Some cases of the present invention that are coated with a HPC coating derived from about a 1% w/w HPC solution, about a 2% w/w HPC solution, about a 3% w/w HPC solution, about a 4% w/w HPC solution, about a 5% w/w HPC solution, about a 6% w/w HPC solution, about a 7% w/w HPC solution, about a 8% w/w HPC solution, about a 9% w/w HPC solution, or about a 10% w/w HPC solution.

The weight percent of other suitable coating materials including, but not limited to pregelatinized starch and HPMC, can be the same as the weight percents described for HPC.

The coating of sodium bicarbonate, or other compressible antacid, with HPC or another suitable material can also be used to formulate other ingredients such as magnesium hydroxide and PPI compounds for tablet manufacture. In tablet format, as well as capsule and caplet format, the compressed granulated sodium bicarbonate provides increased compressibility to the formulation. In tablet or caplet format the compressed granulated sodium bicarbonate provides increased hardness and decreased friability without negatively impacting disintegration. The ratio of sodium bicarbonate to granulating agent can be varied to alter the desired disintegration characteristics of the final formulation.

The composition and method of coating sodium bicarbonate with HPC for use in formulations of the instant invention has the advantage of good compressibility and good disintegration characteristics. It is believed, but not relied upon, that the granulating agent, Klucel-EXF for example, augments the binding property of the composition without increasing the disintegration time.

In some cases, the compressible sodium bicarbonate makes up at least about 60 wt-% of the formulation. In other cases, the compressible sodium bicarbonate makes up at least about 70 wt-% of the formulation. In some cases, the compressible sodium bicarbonate makes up 80% of the formulation. In some cases, the compressible sodium bicarbonate makes up 90% of the formulation. In still other cases, the directly compressible sodium bicarbonate makes up 70-95 wt-% of the formulation.

### Pharmaceutical Compositions

In various cases, the pharmaceutical formulations described herein can be in any solid dosage form such as a tablet; including a suspension tablet, a chewable tablet, an effervescent tablet, or a caplet; a capsule including both soft and hard capsules (including, but not limited to, gelatin and HPMC capsules); a lozenge; pellets; or granules. These pharmaceutical formulations of the present invention can be manufactured by conventional pharmacological techniques.

The amount and types of buffers, proton pump inhibitors, and other excipients useful in each of these dosage forms are described throughout the specification and examples. It should be recognized that where a combination of buffer, proton pump inhibitor and/or excipient, including specific amounts of these components, is described with one dosage form that the same combination could be used for any other suitable dosage form. Moreover, it should be understood that one of skill in the art would, with the teachings found within this application, be able to make any of the dosage forms described herein by combining the components *(i.e.,* amounts and types of PPIs, buffers, and other excipients) described in the different sections of the specification.

Moreover, each of the dosage forms may comprise one or more additional materials such as a pharmaceutically compatible carrier, binder, filling agent, suspending agent, flavoring agent, sweetening agent, disintegrating agent, surfactant, preservative, lubricant, colorant, diluent, solubilizer, moistening agent, stabilizer, wetting agent, anti-adherent, parietal cell activator, anti-foaming agent, antioxidant, chelating agent, antifungal agent, antibacterial agent, or one or more combination thereof. In some cases the additional material is chemically compatible.

In other cases, using standard coating procedures such as those described in Remington's Pharmaceutical Sciences, 20th Edition (2000), a film coating is described around the pharmaceutical formulation. The film coating can be useful to increase stability of the composition and/or to increase swallowability of the solid dosage form.

In various cases, the proton pump inhibitor, antacid, and optionally one or more excipients are dry blended and compressed into a mass, such as a tablet, having a hardness sufficient to provide a pharmaceutical composition that substantially disintegrates within less than about 1 minute, less than about 2 minutes, less than about 5 minutes, less than about 10 minutes, less than about 20 minutes, or less than about 30 minutes upon contact with stomach acid or stimulated stomach acid in in vitro studies, thereby releasing the antacid and the proton pump inhibitor. When at least 75% of the pharmaceutical composition has disintegrated, the compressed mass has substantially disintegrated.

In other cases, the pharmaceutical composition comprises substantially no or no poly[phosphoryl/sulfon]-ated carbohydrate and is in the form of a solid dosage unit. In still another related case, if such a composition comprises a poly[phosphoryl/sulfon]-ated carbohydrate (e.g. sucralfate or sucrose octasulfate), the weight ratio of poly[phosphoryl/sulfon]-ated carbohydrate to buffering agent is less than 1:5 (0.2), less than 1:10 (0.1) or less than 1:20 (0.05). Alternatively, the poly[phosphoryl/sulfon]-ated carbohydrate is present in the composition, if at all, in an amount less than 50 mg, less than 25 mg, less than 10 mg or less than 5 mg.

### Disintegrants

Most PPIs are sparingly soluble in water and therefore exhibit a correlation of disintegration time to bioavailability. Thus, it is important to optimize the disintegration time in order to enhance in vivo dissolution of the drug. In order to release the active ingredient from a solid dosage form matrix as efficiently as possible, disintegrant is often used in the formulation, especially when the dosage forms are compressed with binder. Disintegrants help rupturing the dosage form matrix by swelling or capillary action when moisture is absorbed into the dosage form. Starch is the oldest disintegrant and 5-15% level is suggested (Remington, 20th Ed, p862). Super disintegrants such as Ac-Di-Sol® (croscarmellose sodium) or Crospovidones are effective at lower levels.

Croscarmellose sodium is effective in both direct compression and wet granulation formulations. The amount of croscarmellose sodium (or a product marketed under the trade name Ac-Di-Sol®) used in direct compression tableting may vary with typical usage levels between 1 and 3 percent. When added to granulations, the same percent is used as with a direct compression formulation. It can be added to both the wet mass and the dried granulations before compression. As with direct compression, the use level ranges from 1 to 3 percent with half of the material added to the wet mass and half added to the running powder. This promotes disintegration of both the granules and the tablet.

The amount of croscarmellose sodium used in capsule formulations ranges from 4- 6 percent. Reduced interparticle contact within a capsule facilitates the need for elevated levels of disintegrant. Capsules filled on automatic dosater types of equipment, as opposed to semi-automatic or hand-filled machines, are denser and have a harder structure due to the greater compression forces needed to form the plug and successfully transfer it into the gelatin shell. Greater plug hardness results in greater effectiveness of croscarmellose sodium.

In some cases described herein, the pharmaceutical formulation has greater than about 1 wt-% of a disintegrant. In various cases of the present invention, the pharmaceutical formulations have between about 1 wt-% to about 11 wt-% or between about 1 wt-% to about 8 wt-%, or about 1 wt-% to about 6 wt-%, or about 1 wt-% to about 4 wt-%, of a disintegrant. In some cases the disintegrant is croscarmellose sodium such as Ac-Di-Sol®. In other cases the disintegrant is sodium starch glycolated such as Promogel® or Explotab®. In still other cases, the pharmaceutical formulations have between about 2 wt-% to about 8 wt-% disintegrant, or between about 2 wt-% to about 6 wt-%, or between about 2 wt-% to about 4 wt-%. In yet other cases, the pharmaceutical formulations have greater than about 2 wt-% disintegrant.

Because sodium bicarbonate has effervescent characteristic when mixed with acid such as gastric fluid, some cases of the pharmaceutical formulations described herein can comprise at least about 400 mgs of sodium bicarbonate and greater than about 1 wt-% of a disintegrant. In some cases, the pharmaceutical formulation comprises about 2 wt-% disintegrant, or about 3 wt-% disintegrant, or about 4 wt-% disintegrant. In yet other cases, the pharmaceutical formulation comprises less than 8 wt-% disintegrant. In other cases, the pharmaceutical formulations have less than about 5 wt-% disintegrant, or less than about 4 wt-% disintegrant, or less than about 3 wt-% disintegrant, or less than about 2 wt-% disintegrant, or less than about 1 wt-% disintegrant. In other cases, the sodium bicarbonate helps facilitate the disintegration of the capsule product.

Because sodium bicarbonate has effervescent characteristic when mixed with acid such as gastric fluid, some cases of the pharmaceutical formulations of the present invention can comprise at least about 200 mgs of sodium bicarbonate and greater than about 1 wt-% of a disintegrant. In some cases, the pharmaceutical formulation comprises about 2 wt-% disintegrant, or about 3 wt-% disintegrant, or about 4 wt-% disintegrant. In yet other cases, the pharmaceutical formulation comprises less than 8 wt-% disintegrant. In other cases, the pharmaceutical formulations have less than about 5 wt-% disintegrant, or less than about 4 wt-% disintegrant, or less than about 3 wt-% disintegrant, or less than about 2 wt-% disintegrant, or less than about 1 wt-% disintegrant. In other cases, the sodium bicarbonate helps facilitate the disintegration of the capsule product.

In some cases of the present invention, the wt-% of disintegrant can be decreased and the amount of sodium bicarbonate increased to achieve the desired bioavailability of the proton pump inhibitor. In other cases, the wt-% of disintegrant can be increased and the amount of sodium bicarbonate decreased to achieve the desired bioavailability of the proton pump inhibitor.

### Lubricants

"Lubricants" are compounds added in small quantities to solid dosage formulations to improve certain processing characteristics such as preventing, reducing or inhibiting adhesion or friction of materials. Properties of a good lubricant include, low shear strength, chemical inertness, non-toxicity, ability to form a "durable layer" over the surface covered, and minimal adverse effects on the finished solid dosage formulation.

There are two major types of lubricants: hydrophobic and hydrophilic lubricants. Hydrophobic lubricants are the most widely used in tablet formulations and are usually effective at relatively low concentrations. An exemplary hydrophobic lubricant is magnesium stearate (Mg(C₁₈H₃₅O₂)₂), which contains long hydrocarbon chains that are non polar and are repelled by water. Commercial magnesium stearate consists of a mixture of several fatty acids. In some cases of the present invention, it is beneficial to use a hydrophobic lubricant.

Hydrophilic lubricants have an affinity for water and other polar solvents due to the interaction (typically hydrogen bonding) of polar groups on the lubricant with water. Sodium stearyl fumarate is a hydrophilic lubricant that combines mechanisms of action to achieve optimum lubrication and tablet performance. Because it reduces inter-particulate friction and acts as a barrier lubricant, sodium stearyl fumarate can optimize mixing times, prevent over lubrication, accelerate product development and scale-up, improve disintegration, and help produce enhanced dissolution profiles. In some cases, the hydrophilic lubricant has a solubility in water of at least about 0.05 mg/mL. In some cases, it is beneficial to use a hydrophilic lubricant.

In some cases, the amount of lubricant in the solid dosage formulation is from about 0.25 to about 5% by weight of the final solid dosage formulation. For example, a typical Sodium stearyl fumarate lubricant concentration use range may be from about 0.5 to about 2% of the final solid dosage formulation. Use of magnesium stearate as a lubricant may be from about 0.25 to about 1.5% of the solid dosage formulation.

In some cases, the composition comprises about 0.01 wt-% to about 3 wt-% of a lubricant. In other cases, the composition comprises about 0.5 wt-%, or about 1.0 wt-%, or about 1.5 wt-%, or about 2.0 wt-%, or about 2.5 wt-%, or about 3.0 wt-%, or about 3.5 wt-%, or about 4 wt-% or about 4.5 wt-%, or about 5 wt-%.

Sodium stearyl fumarate, an example of a hydrophilic lubricant, and magnesium stearate, a hydrophobic lubricant, are in the same class of pharmaceutical lubricants known as boundary lubricants. Boundary lubricants work by forming a coat around the individual particles in the blend which prevents the blend particles of the drug product to adhere to the surfaces of the processing equipment.

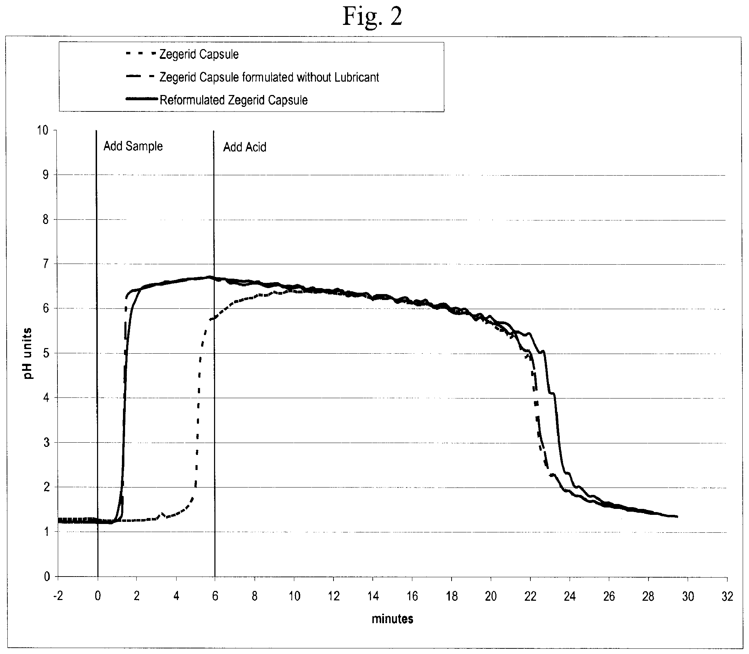

In some cases, the composition uses a hydrophilic lubricant. Specifically, in some cases where a hydrophilic lubricant is selected, the chosen hydrophilic lubricant is sodium stearyl fumarate. In certain cases described herein, a hydrophilic lubricant is desirable rather than a hydrophobic lubricant for several reasons. First, as depicted in Figure 2, the use of the hydrophilic sodium stearyl fumarate in an case described herein instead of the hydrophobic magnesium stearate increases the rate of disintegration of the solid dosage form, and vitiates the 5 minute delay in reaching maximum pH observed when magnesium stearate is used. In addition, when a hydrophilic lubricant such as sodium stearyl fumarate is utilized in an case described herein, a marked decrease in over-lubrication caused by the encapsulation process is achieved.

Second, as Figure 2 indicates, the maximum pH of cases of the present invention containing sodium stearyl fumarate was obtained rapidly and is essentially identical to that of the capsules containing no lubricant. Furthermore, cases of the present invention that incorporate a hydrophilic lubricant such as sodium stearyl fumarate will avoid the delayed disintegration of the capsule shell.

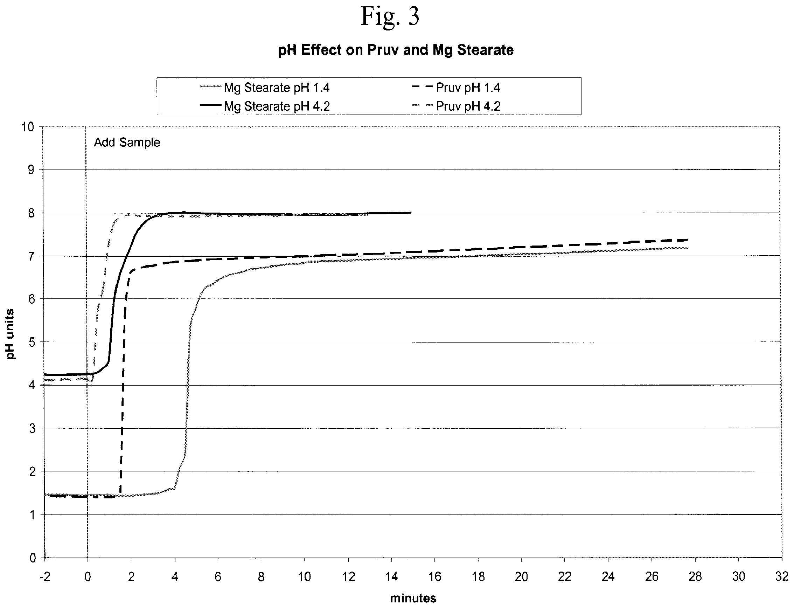

Third, hydrophobic lubricants, such as magnesium stearate, are believed to cause a coating to form around the particles in the capsule which results in an increased disintegration time and decrease in the drug dissolution rate. Referring to the experimental in vitro data in Figure 3, at both pH 1.2 and pH 4.2, the capsules with sodium stearyl fumarate dissolved more rapidly than those containing magnesium stearate. Moreover, at pH 1.4, and to a greater extent at pH 4.2, a plug was formed when the capsules with magnesium stearate dissolved. This formation of a plug can also be described as an un-dispersed or intact pocket of capsule contents following the dissolution of the capsule shell containing these contents. The presence of these plugs with the magnesium stearate lubricant is consistent with the hydrophobic coating delaying the ingress of water into the capsule. However, in cases of the invention containing the sodium stearyl fumarate lubricant, the presence of a plug is lacking.

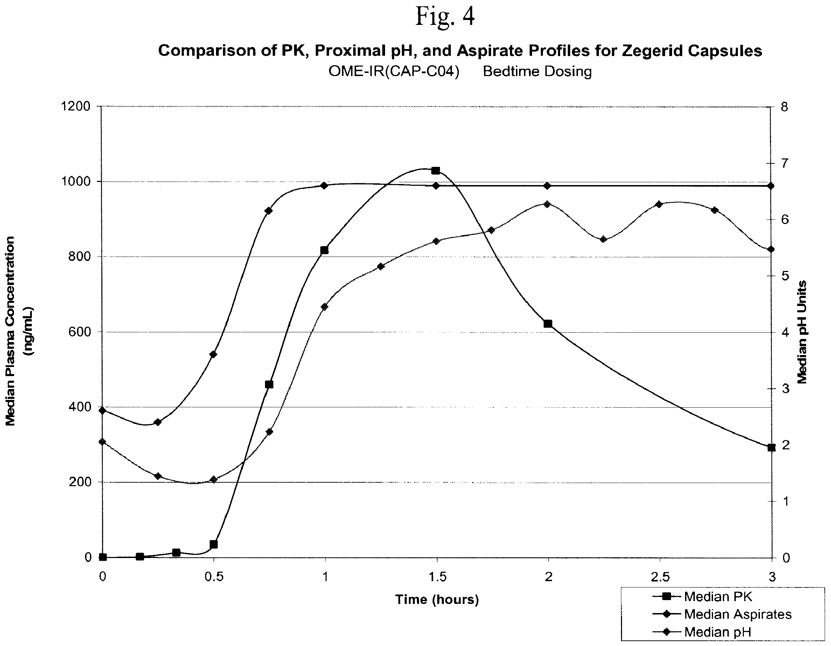

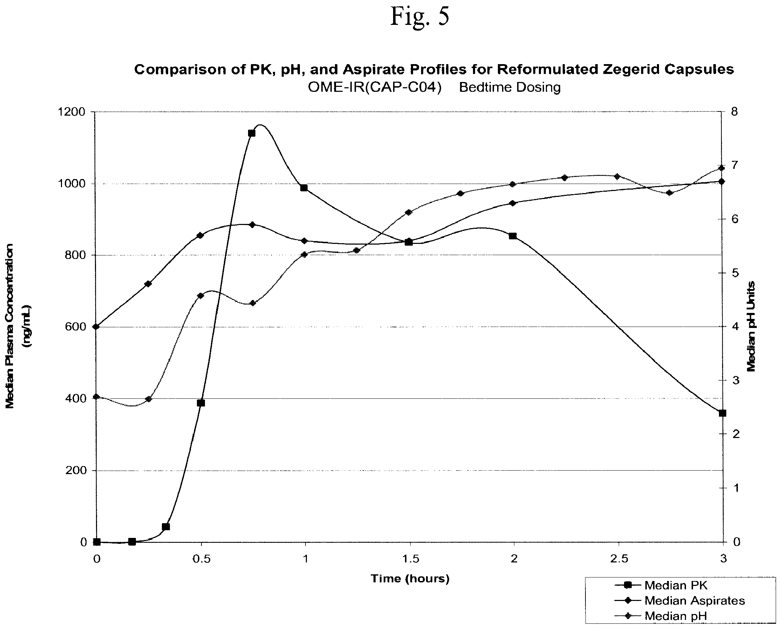

Fourth, as evidenced in Figures 4 and 5, cases of the present invention that include sodium stearyl fumarate as the lubricant achieved maximum drug concentrations in shorter times than those cases of the present invention that contain the hydrophobic magnesium stearate achieved maximum concentration much later. Specifically, the formulation with sodium stearyl fumarate achieved a maximum median pK value within 1 hour, whereas the magnesium stearate formulation achieved this within 1.5 hours. In addition, the median pH value associated with the sodium stearyl fumarate formulation was greater than pH 4 within 30 minutes, whereas the median pH value associated with the magnesium stearate formulation did not reach pH 4 until about an hour.

### Binders

Binders impart cohesiveness to solid oral dosage form formulations. For powder filled capsule formulation, they aid in plug formation that can be filled into hard shell capsules. For tablet formulation, they ensure the tablet remains intact after compression. Materials commonly used as binders include starch gelatin, and sugars such as sucrose, glucose, dextrose, molasses, and lactose. The quantity of binder used influences the characteristics of the dosage form and/or manufacturing processes. For example, dosator type encapsulators (e.g. Zanasi machine) normally requires the filling material to be mechanically strong plugs whereas dosing disc type encapsulators (e.g., H and K machine) do not require the same degree of high plug breaking force. In general, binder levels of 1-10% are used in powder-filled hard gel capsule formulations. Binder usage level in tablet formulations varies whether direct compression, wet granulation, or usage of other excipients such as fillers which itself can act as moderate binder. Formulators skilled in the art can determine the binder level for the formulations, but binder usage level of 2-25% in tablet formulations is common.

In some cases described herein, the wt-% of the disintegrant is at least equivalent to the wt-% of the binder. For example, formulations of the present invention may comprise about 5 wt-% of disintegrant and about 2 wt-% of a binder or about 3 wt-% of a disintegrant and about 3 wt-% of a binder. In other cases, the solid oral dosage form does not comprise a binder. In some cases, the solid oral dosage form comprises significantly more disintegrant than binder. For example, the binder may be present in an amount of less than 2 wt-% while the disintegrant is present in an amount of greater than 5 wt-%. In other cases, the binder and disintegrant are present in the formulation in substantially the same amount. For example, the binder may be present in an amount of about 2 wt-% and the disintegrant may be present in an amount of about 3 wt-%.

### Particle Size of Ingredients

The particle size of the proton pump inhibitor, antacid and excipients is an important factor which can effect bioavailability, blend uniformity, segregation, and flow properties. In general, smaller particle sizes of a drug increases the bioabsorption rate of the drug with substantially poor water solubility by increasing the surface area. The particle size of the drug and excipients can also affect the suspension properties of the pharmaceutical formulation. For example, smaller particles are less likely to settle and therefore form better suspensions.

In various cases, the average particle size of the dry powder (which can be administered directly, as a powder for suspension, or used in a solid dosage form) is less than about 500 microns in diameter, or less than about 450 microns in diameter, or less than about 400 microns in diameter, or less than about 350 microns in diameter, or less than about 300 microns in diameter, or less than about 250 microns in diameter, or less than about 200 microns in diameter, or less than about 150 microns in diameter, or less than about 100 microns in diameter, or less than about 75 microns in diameter, or less than about 50 microns in diameter, or less than about 25 microns in diameter, or less than about 15 microns in diameter. In other cases, the average particle size of the aggregates is between about 25 microns in diameter to about 300 microns in diameter. In still other cases, the average particle size of the aggregates is between about 25 microns in diameter to about 150 microns in diameter. And, in still further cases, the average particle size of the aggregates is between about 25 microns in diameter to about 100 microns in diameter. The term "average particle size" is intended to describe the average diameter of the particles and/or agglomerates used in the pharmaceutical formulation.

In another case, the average particle size of the insoluble excipients is between about 5 µm to about 500 µm, or less than about 400 µm, or less than about 300 µm, or less than about 200 µm, or less than about 150 µm, or less than about 100 µm, or less than about 90 µm, or less than about 80 µm, or less than about 70 µm, or less than about 60 µm, or less than about 50 µm, or less than about 40 µm, or less than about 30 µm, or less than about 25 µm, or less than about 20 µm, or less than about 15 µm, or less than about 10 µm, or less than about 5 µm.

In other cases described herein, at least about 80% of the particles have a particle size of less than about 300 µm, or less than about 250 µm, or less than about 200 µm, or less than about 150 µm, or less than about 100 µm, or less than about 50 µm. In another case, at least about 85% of the dry powder particles have a particle size of less than about 300 µm, or less than about 250 µm, or less than about 200 µm, or less than about 150 µm, or less than about 100 µm, or less than about 50 µm. In still other cases described herein, at least about 90% of the dry powder particles have a particle size of less than about 300 µm, or less than about 250 µm, or less than about 200 µm, or less than about 150 µm, or less than about 100 µm, or less than about 50 µm In yet another case, at least about 95% of the dry powder particles have a particle size of less than about 300 µm, or less than about 250 µm, or less than about 200 µm, or less than about 150 µm, or less than about 100 µm, or less than about 50 µm.

In other cases, the average particle size of the insoluble material is between about 5 *11m* to about 250 µm in diameter. In other cases, the average particle size of the insoluble excipients is between about 5 µm to about 100 µm, or between about 5 µm to about 80 µm, or between about 5 µm to about 50 µm in diameter. As used herein, the term "insoluble material," "insoluble excipient" or "insoluble antacid" refers to a solubility of less than 25 mg/mL in water. In some cases, the solubility of the insoluble material is less than 10 mg/mL, less than 5 mg/mL, less than 1 mg/mL, or less than .01 mg/mL in water.

Several factors can be considered in choosing both the proper excipient and its quantity. For example, the excipient should be pharmaceutically acceptable. Also, in some examples, rapid dissolution and neutralization of gastric acid to maintain the gastric pH at about 6.5 for at least one hour. The excipients which will be in contact with the proton pump inhibitor, if any, should also be chemically compatible with the proton pump inhibitor. "Chemically compatible" is intended to mean that the material that shows less than 5% degradation of the proton pump inhibitor when stored at room temperature for about 3 months.

### Microencapsulation and Dry Coating

In one case, the proton pump inhibitor is microencapsulated or dry coated prior to being formulated into one of the above forms. In another case, some or all of the antacid is also spray dried prior to being further formulated into one of the above forms. In these cases, the solid compositions, e.g., tablets, chewable tablets, effervescent tablets, and capsules, can be prepared by mixing the microencapsulated proton pump inhibitor or dry coated proton pump inhibitor with one or more antacid and pharmaceutical excipients to form ·a bulk blend composition. When using dry coated proton pump inhibitor, mixing with additional antacid is optional. When referring to these bulk blend compositions as homogeneous, it is meant that the microencapsulated or dry coated proton pump inhibitor and antacid are dispersed evenly throughout the composition so that the composition may be readily subdivided into equally effective unit dosage forms, such as tablets, pills, and capsules. The individual unit dosages may also comprise film coatings, which disintegrate upon oral ingestion or upon contact with diluent.

Compositions may include microencapsulation and/or dry coating of the proton pump inhibitor and/or the antacid.

Exemplary microencapsulation materials useful for enhancing the shelf life of pharmaceutical compositions comprising a proton pump inhibitor include, but are not limited to: hydroxypropyl cellulose ethers (HPC) such as Klucel® or Nisso HPC; low-substituted hydroxypropyl cellulose ethers (L-HPC); hydroxypropyl methyl cellulose ethers (HPMC) such as Seppifilm-LC, Pharmacoat®, Metolose SR, Methocel®-E, Opadry YS, PrimaFlo, Benecel MP824, and Benecel MP843; methylcellulose polymers such as Methocel®-A and Metolose®; Ethylcelluloses (EC) and mixtures thereof such as E461, Ethocel®, Aqualon®-EC, Surelease®; Polyvinyl alcohol (PVA) such as Opadry AMB; hydroxyethylcelluloses such as Natrosol®; carboxymethylcelluloses and salts of carboxymethylcelluloses (CMC) such as Aqualon®-CMC; polyvinyl alcohol and polyethylene glycol co- polymers such as Kollicoat IR®; monoglycerides (Myverol), triglycerides (KLX), polyethylene glycols, modified food starch, acrylic polymers and mixtures of acrylic polymers with cellulose ethers such as Eudragit® EPO, Eudragit® RDlOO, and Eudragit® ElOO; cellulose acetate phthalate; sepifilms such as mixtures of HPMC and stearic acid, cyclodextrins; and mixtures of these materials.

In various cases, an antacid such as sodium bicarbonate or sodium carbonate is incorporated into the microencapsulation material. In other cases, an antioxidant such as BHT is incorporated into the microencapsulation material. In still other cases, plasticizers such as polyethylene glycols, e.g., PEG 300, PEG 400, PEG 600, PEG 1450, PEG 3350, and PEG 800, stearic acid, propylene glycol, oleic acid, and triacetin are incorporated into the microencapsulation material. In other cases, the microencapsulating material useful for enhancing the shelf life of the pharmaceutical compositions is from the USP or the National Formulary (NF). In yet other cases, the microencapsulation material is Klucel. In still other cases, the microencapsulation material is Methocel.

In addition to microencapsulation, the stability of the proton pump inhibitors used in the present invention may be increased by alternative methods such as dry coating and nano-particle coating. Dry coating involves the formation of granules of a coated proton pump inhibitor which are then mixed with other components. Dry granulation is achieved by forming dense compacts which are reduced to a desired particle size and then blended with other components of the pharmaceutical composition. Dry granulation and nano-particle coating can provide enhanced stability and taste masking characteristics by diluting and isolating certain components in a granulated matrix of compatible ingredients that can enhance the shelf life of proton pump inhibitor products as well as taste mask the bitterness.

In various cases, the average particle sizes of the dry coated proton pump inhibitor ranges from submicron to less than about 1,000 microns in diameter, or less than about 900 microns in diameter, or less than about 800 microns in diameter, or less than about 700 microns in diameter, or less than about 600 microns in diameter, or less than about 500 microns in diameter, or less than about 450 microns in diameter, or less than about 400 microns in diameter, or less than about 350 microns in diameter, or less than about 300 microns in diameter, or less than about 250 microns in diameter, or less than about 200 microns in diameter, or less than about 150 microns in diameter, or less than about 100 microns in diameter, or less than about 75 microns in diameter, or less than about 50 microns in diameter, or less than about 25 microns in diameter, or less than about 15 microns in diameter.

In other cases, the average particle size of the aggregates of the dry coated proton pump inhibitor is between about 25 microns in diameter to about 300 microns in diameter. In still other cases, the average particle size of the aggregates is between about 100 microns in diameter to about 200 microns in diameter. And in still further cases, the average particle size of the aggregates is between about 25 microns in diameter to about 100 microns in diameter. The term "average particle size" is intended to describe the average diameter of the particles and/or agglomerates used in the pharmaceutical formulation.

In some cases, the dry coated proton pump inhibitor granules are less than about 2000 microns, or less than about 1500 microns, or less than about 1000 microns. In some cases, the average particle size of the dry coated proton pump inhibitor granules is between about 100 to about 2000 microns, or between about 100 to about 1000 microns, or between about 200 to about 800 microns, or between about 300 to about 600 microns.

In other cases, the dry coated proton pump inhibitor granules comprise antacid, binder, lubricant and/or sweeteners. In some cases, the antacid is sodium bicarbonate. In other cases, the binder is hydroxypropyl cellulose. In still other cases, the sweetener is sucralose and/or xylitol. In yet other cases, the lubricant is magnesium stearate. In some cases the lubricant is sodium stearyl fumarate.

In various cases, the dry coated proton pump inhibitor is combined with additional antacid. In some cases, the additional antacid is the same antacid as used in the material used to dry coat the proton pump inhibitor. In other cases, the antacid is a different antacid. In still other cases, the antacid is a combination of two or more antacids.

In yet other cases, one or more pharmaceutically acceptable excipients are mixed with the dry coated proton pump inhibitor to form the pharmaceutical composition. In some cases the additional pharmaceutical excipients include one or more flavors. In further cases, one or more other compatible materials are present in the dry coating material. Exemplary materials include, e.g., parietal cell activators, erosion facilitators, diffusion facilitators, anti-adherents, anti-foaming agents, antioxidants, flavoring agents, and carrier materials such as binders, suspending agents, disintegration agents, filing agents, surfactants, solubilizers, stabilizers, lubricants, wetting agents, diluents. In some cases, the additional compatible materials are binders, lubricants and sweeteners.

In some cases, the weight percent of the proton pump inhibitor in the dry coated granules is about 2-70%. In some cases, the weight percent of the proton pump inhibitor in the dry coated granules is about 5-50%, or about 5-30%. In other cases, the weight percent of the proton pump inhibitor in the dry coated granules is about 20% to about 40%. In other cases the weight percent of the proton pump inhibitor in the dry coated granules is about 20-40%. In yet other cases, the weight percent of the proton pump inhibitor in the granules is about 5%, or about 7%, or about 10%, or about 15%, or about 20%, or about 25%, or about 30%, or about 35%, or about 40%.

It should be noted that the compositions and methods described herein as containing microencapsulated proton pump inhibitors can, in addition to or in the alternative, contain dry coated proton pump inhibitors.

### Stability

A pharmaceutical formulation of the present invention is stable if, e.g., the proton pump inhibitor has less than about 0.5% degradation after one month of storage at room temperature, or less than about 1% degradation after one month at room temperature, or less than about 1.5% degradation after one month of storage at room temperature, or less than about 2% degradation after one month storage at room temperature, or less than about 2.5% degradation after one month of storage at room temperature, or less than about 3% degradation after one month of storage at room temperature.

In other cases, a pharmaceutical formulation of the present invention may be stable if the pharmaceutical formulation contains less than about 5% total impurities after about 3 years of storage, or after about 2.5 years of storage, or about 2 years of storage, or about 1.5 years of storage, or about 1 year of storage, or after 11 months of storage, or after 10 months of storage, or after 9 months of storage, or after 8 months of storage, or after 7 months of storage, or after 6 months of storage, or after 5 months of storage, or after 4 months of storage, or after 3 months of storage, or after 2 months of storage, or after 1 month of storage.

In further cases, pharmaceutical formulations of the present invention may contain microencapsulated omeprazole and have enhanced shelf life stability if the pharmaceutical formulation contains less degradation of the proton pump inhibitor than proton pump inhibitor in the same formulation which is not microencapsulated, or "bare". For example, if bare proton pump inhibitor in the pharmaceutical formulation degrades at room temperature by more than about 2% after one month of storage and the microencapsulated material degrades at room temperature by less than about 2% after one month of storage, then the proton pump inhibitor has been microencapsulated with a compatible material that enhances the shelf life of the pharmaceutical formulation.

### Pharmacodynamic Properties of Dosage Forms

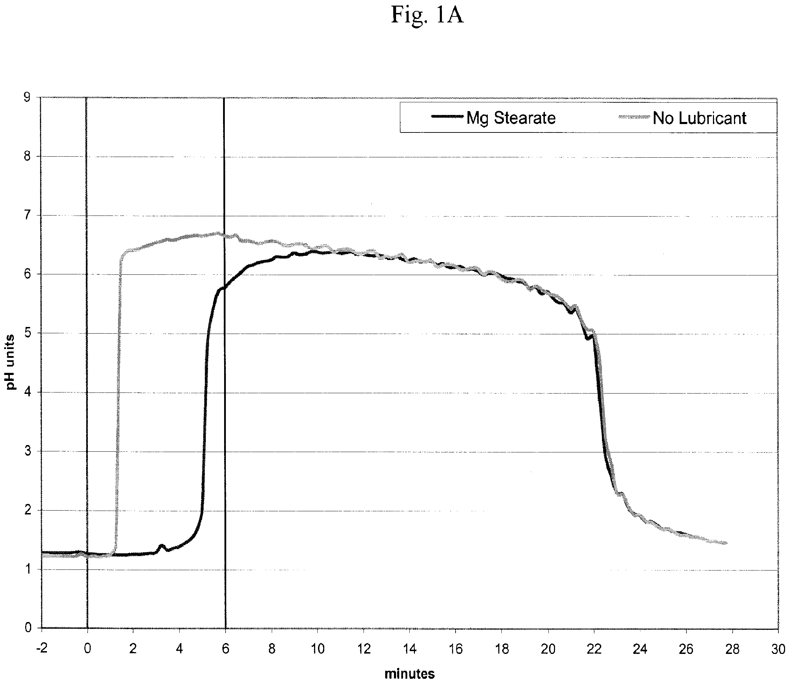

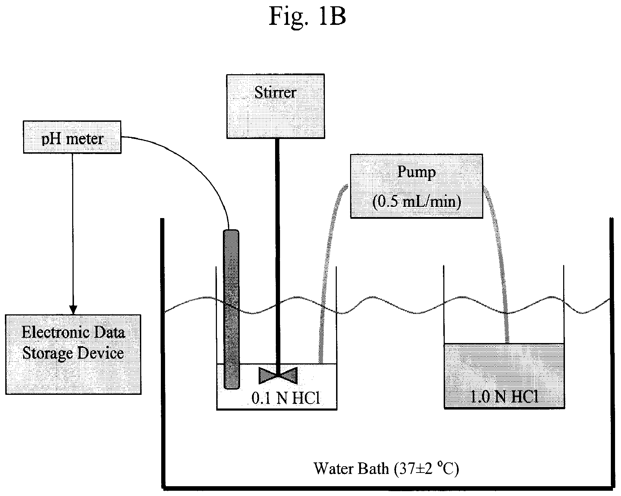

As discussed in more detail herein, small quantities of lubricant excipient can be utilized in cases of the present invention to improve certain processing characteristics of the pharmaceutical formulation. Specifically, lubricant is required to allow for the high-speed automatic encapsulation necessary to make the formulation a commercially viable product. Figure 1, representing an case of the present invention, indicates that that there is a delay of up to 5 minutes to reach the maximum pH in capsules containing the magnesium stearate lubricant vs. those containing an unlubricated formulation. In other cases described herein, there is a delay of about 4 minutes, about 3 minutes, about 2 minutes, about 1 minute, or less than one minute. In other cases, the delay is about 6 minutes, about 7 minutes, about 8 minutes, about 9 minutes, or about 10 minutes, or greater than 10 minutes. The delay in increase in pH in each of these cases is due to the time required for the capsule shell to disintegrate caused by the addition into the capsule of the hydrophobic magnesium stearate lubricant. Quick disintegration is necessary in order to expose the contents of the media for dissolution, and for the resulting pharmacological effect of the pharmaceutical formulation to occur. In addition, lubricants with very low shear strength due to their laminate structures, e.g., magnesium stearate, will be more prone to the over-lubrication effects resulting from these added shear forces of the encapsulation process. There is a need for a more efficient lubricant with a structure that will make it less prone to any shear induced over-lubrication during the encapsulation process and which will have a decreased functional disintegration time relative to the currently marketed Zegerid® formulation which contains the magnesium stearate lubricant.

Due to its effectiveness during the high speed encapsulation process, magnesium stearate is the most widely used lubricant in the pharmaceutical industry. However, as discussed above, and herein, the hydrophobic coating makes magnesium stearate less than an ideal lubricant. Alternative lubricants can be considered when magnesium stearate cannot be used. However, like magnesium stearate, most of the effective alternative lubricant options also very hydrophobic, and thus would present the same complications if exchanged with magnesium stearate of the currently marketed Zegerid® formulation. As shown herein, sodium stearyl fumarate is a relatively effective, hydrophilic (or at least not hydrophobic), alternative to magnesium stearate in tablet, caplet and capsule pharmaceutical formulations.

In certain cases of the invention, the maximum pH of the pharmaceutical formulation containing sodium stearyl fumarate is obtained in vitro in about the same amount of time as if the pharmaceutical formulation did not contain a lubricant. In one case described herein with sodium stearyl fumarate as the lubricant, the maximum pH of between 6 and 7 was obtained in vitro within about 1 minute. In another case, the same maximum pH was realized in about 1 to about 2 minutes. In yet other cases described herein, the maximum pH was realized in about 2 to about 3 minutes. In still other cases described herein, the maximum pH is realized in about 3 to about 4 minutes.

In cases of the present invention with formulations containing sodium stearyl fumarate, no plug is observed at either pH 1.4 or 4.2. In other cases of the present invention with sodium stearyl fumarate as the lubricant, no plug is observed at respective pH values of about 1.1 and about 4.1, about 1.1 and about 4.2, or about 1.1 and about 4.3, or about 1.0, and about 4.1, or about 1.0 and about 4.2, or about 1.0 and 4.3, or about 1.3 and about 4.1, or about 1.3 and 4.2, or about 1.3 and 4.4, or about 0.5 and 2.0, or about 0.5 and 2.5, or about 0.5 and 3.0, or about 0.5 and about 3.5, or about 0.5 and 4.0, or 0.5 and about 4.5, or about 0.5 and 5.0, or about 0.5 and 5.5, or about 0.5 and 6.0, or about 0.75 and 2.0, or about 0.75 and 2.5, or about 0.75 and 3.0, or about 0.75 and about 3.5, or about 0.75 and 4.0, or 0.75 and about 4.5, or about 0.75 and 5.0, or about 0.75 and 5.5, or about 0.75 and 6.0.

In some cases, the composition achieves an initial pH rise within about 4 minutes. In some cases, the initial pH rise occurs within about 3 minutes or within about 2 minutes or within about 1 minute. In some cases, the composition achieves an initial pH of at least about 4 within about 4 minutes. In some cases, the composition achieves an initial pH of at least about 4 within about 3 minutes or within about 2 minutes or within about 1 minute. In some cases, the composition achieves an initial pH of at least about 5 within about 4 minutes. In some cases, the composition achieves an initial pH of at least about 5 within about 3 minutes or within about 2 minutes or within about 1 minute. In some cases, the composition achieves an initial pH of at least about 6 within about 4 minutes. In some cases, the composition achieves an initial pH of at least about 6 within about 3 minutes or within about 2 minutes or within about 1 minute.

In some cases, the formulation is in the form of a tablet and the tablet has a hardness of about 10-20 kP. In some cases, the hardness of the tablet is about 12-20 kP, about 15-20 kP or about 17-20 kP. In some cases, the tablet achieves a hardness of about 10-20 kP with less than 10,000 pounds of force. In some cases, the tablet achieves hardness in the range described above with less than 9,000, less than 8,000, less than 7,000 less, than 6,000 or less than 5,000 pounds of force. In some cases, the formulation is in the form of a tablet comprising compressible sodium bicarbonate and hardness of 10-20 kP is achieve with less than about 7,000 pounds of force. In some cases, the desired hardness is achieved with less than about 6,000 or less than about 5,000 pounds of force.

In some cases, the formulation has a friability of less than about 0.5%. In some cases the formulation has a friability of less than about 1 %. In other cases, the formulation has a friability of between about 0.1% to about 1%. In other cases, the formulation has a friability of between about 0.1% to about 0.5%. In specific cases, the composition has a friability of between about 0.1% to about 0.5%.

In various cases of the present invention, the pharmaceutical compositions provide a release profile of the proton pump inhibitor, using USP dissolution methods, whereby greater than about 50% of the proton pump inhibitor is released from the composition within about 2 hours; or greater than 50% of the proton pump inhibitor is released from the composition within about 1.5 hours; or greater than 50% of the proton pump inhibitor is released from the composition within about 1 hour after exposure to gastrointestinal fluid. In another case, greater than about 60% of the proton pump inhibitor is released from the composition within about 2 hours; or greater than 60% of the proton pump inhibitor is released from the composition within about 1.5 hours; or greater than 60% of the proton pump inhibitor is released from the composition within about 1 hour after exposure to gastrointestinal fluid. In yet another case, greater than about 70% of the proton pump inhibitor is released from the composition within about 2 hours; or greater than 70% of the proton pump inhibitor is released from the composition within about 1.5 hours; or greater than 70% of the proton pump inhibitor is released from the composition within about 1 hour after exposure to gastrointestinal fluid.

In some cases of the present intention, the pharmaceutical compositions contain at least one sustained release proton pump inhibitor and the proton pump inhibitor in the sustained release form is less than 50% release at 2 hours. In some cases, the proton pump inhibitor in the sustained release form is less than 60%, or less than 70% or less than 80% released after 2 hours. In some cases of the present intention, the pharmaceutical compositions contain at least one sustained release proton pump inhibitor and the proton pump inhibitor in the sustained release form is less than 50% release at 4 hours. In some cases, the proton pump inhibitor in the sustained release form is less than 60%, or less than 70% or less than 80% released after 4 hours. In some cases of the present intention, the pharmaceutical compositions contain at least one sustained release proton pump inhibitor and the proton pump inhibitor in the sustained release form is less than 50% release at 6 hours. In some cases, the proton pump inhibitor in the sustained release form is less than 60%, or less than 70% or less than 80% released after 6 hours. In some cases of the present intention, the pharmaceutical compositions contain at least one sustained release proton pump inhibitor and the proton pump inhibitor in the sustained release form is less than 50% release at 8 hours. In some cases, the proton pump inhibitor in the sustained release form is less than 60%, or less than 70% or less than 80% released after 8 hours.

In some cases of the present intention, the pharmaceutical compositions contains at least one immediate release proton pump inhibitor and at least one sustained release proton pump inhibitor. In these cases, the immediate release proton pump inhibitor is substantially release within 1 hour and the sustained release proton pump inhibitor is less than 50% released at 1 hour. In some cases, the proton pump inhibitor in the sustained release form is less than 60%, or less than 70% or less than 80% released after 1 hour.

In some cases, the immediate release proton pump inhibitor is at least 80% released within 30 minutes and the sustained release proton pump inhibitor is less than 50% released at 1 hour. In some cases, the proton pump inhibitor in the sustained release form is less than 60%, or less than 70% or less than 80% released after 1 hour. In some cases, the proton pump inhibitor in the immediate release form is at least 50%, or at least 60%, or at least 70%, or at least 90% released within 30 minutes.

In some cases, the immediate release proton pump inhibitor is at least 80% released within 1 hour and the sustained release proton pump inhibitor is less than 50% released at 1 hour. In some cases, the proton pump inhibitor in the sustained release form is less than 60%, or less than 70% or less than 80% released after 1 hour. In some cases, the proton pump inhibitor in the immediate release form is at least 50%, or at least 60%, or at least 70%, or at least 90% released within 1 hour.

In some cases, the immediate release proton pump inhibitor is at least 80% released within 1 hour and the sustained release proton pump inhibitor is less than 50% released at 2 hours. In some cases, the proton pump inhibitor in the sustained release form is less than 60%, or less than 70% or less than 80% released after 2 hours. In some cases, the proton pump inhibitor in the immediate release form is at least 50%, or at least 60%, or at least 70%, or at least 90% released within 1 hour.

In some cases, the immediate release proton pump inhibitor is at least 80% released within 1 hour and the sustained release proton pump inhibitor is less than 50% released at 4 hours. In some cases, the proton pump inhibitor in the sustained release form is less than 60%, or less than 70% or less than 80% released after 4 hours. In some cases, the proton pump inhibitor in the immediate release form is at least 50%, or at least 60%, or at least 70%, or at least 90% released within 1 hour.

In some cases, the immediate release proton pump inhibitor is at least 80% released within 1 hour and the sustained release proton pump inhibitor is less than 50% released at 8 hours. In some cases, the proton pump inhibitor in the sustained release form is less than 60%, or less than 70% or less than 80% released after 8 hours. In some cases, the proton pump inhibitor in the immediate release form is at least 50%, or at least 60%, or at least 70%, or at least 90% released within 1 hour.

### Kinetic Stomach Model

The acid neutralizing capacity and pH profile of various antacid combinations can be evaluated by using an in-vitro stomach model. Several of these simulated dynamic models are known in the art. See, e.g., Smyth et al., Correlation of In-Vivo Methodology for Evaluation of Antacids, J. Pharm. Sci. Vol. 65, 1045 (1976); Hobert, Fordham et al., In-Vivo Evaluation of Liquid Antacids, New England Journal of Med. 288, 923 (1973); Johnson et al., The Chemical Testing of Antacids, Gut 5, 585 (1964); Clain et al., In-Vitro Neutralizing Capacity of Commercially Available Antacid Mixtures and Their Role in the Treatment of Peptic Ulcer, S. Afr. Med. J., 57, 158 (1980); Rossett et al., In -Vitro Evaluation of Efficacy of More Frequently Used Antacids with Particular Attention to Tablets, Gastroentrology, 26, 490; Decktor et al., Comparative Effects of Liquid Antacids on Esophageal and Gastric pH in Patients with Heartburn, Am. J. of Therapeutics, 2, 481 (1995); Charles Fuchs, Antacids: Their Function, Formulation and Evaluation, Drug and Cosmetic Industry, 49, 692; Stewart M. Beekman, Preparation and Properties of New Gastric Antacids I, Aluminum Hydroxide-Magnesium Carbonate Dried Gels, J. Am. Pharm. Assoc., 49, 191 (1960). For example, a modified Fuch's model where the continuous influx of 0.5 mEq of acid is added to initial 5.0 mEq of acid to simulate a fasting state of stomach can be used with the present invention. Reference Figure 1B.

In various cases of the present invention, the antacid increases the gastric pH to at least about 3.5 for no more than about 90 minutes as measured by a simulated stomach model such as a modified Fuch's kinetic in-vitro pH model. In other cases, the antacid increases the pH to at least about 3.5 for no more than about 60 minutes. In still other cases, the antacid increases the pH to at least about 3.5 for no more than 45 minutes. Depending on the buffer system used (i.e., type of antacid and amount) some cases described herein, the antacid increases the gastric pH to at least about 3.5 for no more than about 30 minutes as measured by a simulated stomach model such as a modified Fuchs' kinetic in-vitro pH model. In other cases, the antacid increases the gastric pH to at least about 3.5 for less than about 25 minutes as measured by a simulated stomach model such as a modified Fuch's kinetic in-vitro pH model. In yet other cases, the antacid increases the gastric pH to at least about 3.5 for less than about 20 minutes, or less than about 15 minutes, or less than about 10 minutes as measured by a stimulated stomach model such as a modified Fuch's kinetic in-vitro pH model. In each of these cases, the antacid protects at least some of the proton pump inhibitor and a therapeutically effective amount of the proton pump inhibitor is delivered to the subject.

In each of these cases, the antacid protects at least some of the proton pump inhibitor and a therapeutically effective amount of the proton pump inhibitor is delivered to the subject.

### Dosage

The proton pump inhibiting agent is administered and dosed in accordance with good medical practice, taking into account the clinical condition of the individual patient, the method of administration, scheduling of administration, and other factors known to medical practitioners. In human therapy, it is important to provide a dosage form that delivers the required therapeutic amount of the drug in vivo, and renders the drug bioavailable in a rapid manner.

The percent of intact drug that is absorbed into the bloodstream is not narrowly critical, as long as a therapeutically effective amount, e.g., a gastrointestinal-disorder-effective amount of a proton pump inhibiting agent, is absorbed following administration of the pharmaceutical composition to a subject. Gastrointestinal- disorder-effective amounts may be found in U.S. Patent No. 5,622,719. It is understood that the amount of proton pump inhibiting agent and/or antacid that is administered to a subject is dependent on a number of factors, e.g., the sex, general health, diet, and/or body weight of the subject. In addition, treatment dosages generally may be titrated to optimize safety and efficacy. Typically, dosage-effect relationships from in vitro and/or in vivo tests initially can provide useful guidance on the proper doses for subject administration. In terms of treatment protocols, it should be appreciated that the dosage to be administered will depend on several factors, including the particular agent that is administered, the route chosen for administration, and the condition of the particular subject.

Illustratively, administration of a substituted bicyclic aryl-imidazole to a young child or a small animal, such as a dog, a relatively low amount of the proton pump inhibitor, e.g., about 1 mg to about 30 mg, will often provide blood serum concentrations consistent with therapeutic effectiveness. Where the subject is an adult human or a large animal, such as a horse, achievement of a therapeutically effective blood serum concentration will require larger dosage units, e.g., about 10 mg, about 15 mg, about 20 mg, about 30 mg, about 40 mg, about 80 mg, or about 120 mg dose for an adult human, or about 150 mg, or about 200 mg, or about 400 mg, or about 800 mg, or about 1000 mg dose, or about 1500 mg dose, or about 2000 mg dose, or about 2500 mg dose, or about 3000 mg dose or about 3200 mg dose or about 3500 mg dose for an adult horse.

In various other cases of the present invention, the amount of proton pump inhibitor administered to a subject is, e.g., about 0.5-2 mg/Kg of body weight, or about 0.5 mg/Kg of body weight, or about 1 mg/Kg of body weight, or about 1.5 mg/Kg of body weight, or about 2 mg/Kg of body weight.

In various other cases of the present invention, the amount of proton pump inhibitor administered to a subject is, e.g., about 1-2 mg/Kg of body weight, or about 0.5 mg/Kg of body weight, or about 1 mg/Kg of body weight, or about 1.5 mg/Kg of body weight, or about 2 mg/Kg of body weight.

In various cases, unit dosage forms for humans contain about 1 mg to about 120 mg, or about 1 mg, or about 5 mg, or about 10 mg, or about 15 mg, or about 20 mg, or about 30 mg, or about 40 mg, or about 50 mg, or about 60 mg, or about 70 mg, or about 80 mg, or about 90 mg, or about 100 mg, or about 110 mg, or about 120 mg of a proton pump inhibitor.

In a further case described herein, the pharmaceutical formulation is administered in an amount to achieve a measurable serum concentration of the proton pump inhibiting agent greater than about 500 ng/mL within about 45 minutes after administration of the pharmaceutical formulation. In another case described herein, the pharmaceutical formulation is administered to the subject in an amount to achieve a measurable serum concentration of the proton pump inhibiting agent greater than about 500 ng/mL within about 30 minutes after administration of the pharmaceutical formulation. In yet another case, the pharmaceutical formulation is administered to the subject in an amount to achieve a measurable serum concentration of the proton pump inhibiting agent greater than about 250 ng/mL within about 15 minutes after administration of the pharmaceutical formulation.

In still another case of the present invention, the composition is administered to the subject in an amount to achieve a measurable serum concentration of a non-acid degraded or non-acid reacted proton pump inhibiting agent greater than about 500 ng/mL within about 1 hour after administration of the composition. In yet another case of the present invention, the composition is administered to the subject in an amount to achieve a measurable serum concentration of a non-acid degraded or non-acid reacted proton pump inhibiting agent greater than about 500 ng/mL within about 45 minutes after administration of the composition.

In another case of the present invention, the composition is administered to the subject in an amount sufficient to achieve a maximum serum concentration (Cmax) at a time (Tmax) that is within about 90, 70, 60, 50, 40, 30 or 20 minutes after administration of the composition according to the present invention. For example, the Tmax of the composition may be about 30 minutes after administration. In other cases, the Tmax of the composition may be about 45 minutes.

In still another case of the invention, the composition is administered to the subject in an amount sufficient to achieve a maximum serum concentration (Cmax) at a time (Tmax) that is between about 10 and about 90 minutes, between about 10 to about 60 minutes, between about 15 to about 60 minutes or between about 20 to about 60 minutes after administration of the composition according to the present invention. In some specific cases, the values of Cmax and Tmax are averages over a test population. In other specific cases, the values of Cmax and Tmax are the values for an individual. For example, the composition may exhibit a Tmax between about 15 minutes and about 30 minutes, between about 30 minutes and about 45 minutes or between about 45 minutes and about 60 minutes.

In still another case, the composition is administered in an amount sufficient to achieve a maximum serum concentration (Cmax) of from about 400 to about 3000 ng/mL, from about 400 to about 2500 ng/mL, from about 400 to about 2000 ng/mL, from about 400 to about 1500 ng/mL, from about 1000 to about 1500 ng/mL, from about 400 to about 1000 ng/mL or from about 400 to about 700 ng/mL. In some specific cases, the values of Cmax and Tmax are averages over a test population. In other specific cases, the values of Cmax and Tmax are the values for an individual.

In a further case, the composition is administered m an amount sufficient to achieve a maximum serum concentration (Cmax) of greater than 200 ng/mL, greater than 200 ng/mL, greater than 600 ng/mL, greater than 1000 ng/mL. In some specific cases, the values of Cmax and Tmax are averages over a test population. In other specific cases, the values of Cmax and Tmax are the values for an individual.

In some cases, the pharmaceutical composition comprises a sustained release proton pump inhibitor and, upon administration of the composition, a measurable serum concentration is achieved for at least about 3 to about 10 hours. In various cases, the measurable serum concentration is achieved for about 3 hours, about 4 hours, about 5 hours, about 6 hours, about 7 hours, about 8 hours, about 9 hours or about 10 hours. In some cases, a measurable serum level is achieved for about 4 to about 14 hours, about 4 to about 12 hours, about 4 to about 10 hours, about 4 to about 8 hours.

Contemplated compositions described herein can be administered once-a-day, twice-a-day, three times a day, etc. if desired.

### Administration

Described herein is a pharmaceutical composition comprising a proton pump inhibiting agent and a buffering agent for oral administration to a subject. In one case, upon administration to a subject, the composition contacts the gastric fluid of the stomach and increases the gastric pH of the stomach to a pH that prevents or inhibits acid degradation of the proton pump inhibiting agent in the gastric fluid of the stomach and allows a measurable serum concentration of the proton pump inhibiting agent to be absorbed into the blood serum of the subject, such that pharmacokinetic and pharmacodynamic parameters can be obtained using testing procedures known to those skilled in the art.

In one case, upon administration to a subject, the composition contacts the gastric fluid of the stomach and increases the gastric pH of the stomach to a pH that prevents or inhibits acid degradation of the proton pump inhibiting agent in the gastric fluid of the stomach and allows a measurable serum concentration of the proton pump inhibiting agent to be absorbed into the blood serum of the subject, such that pharmacokinetic and pharmacodynamic parameters can be obtained using testing procedures known to those skilled in the art.

Cases of the present invention also provide pharmaceutical compositions wherein a therapeutically effective dose of the proton pump inhibitor is in the blood serum of the patient within about 45 minutes, or within about 30 minutes, or within about 25 minutes, or within about 20 minutes, or within about 15 minutes, or within about 10 minutes, or within about 5 minutes after ingestion of the pharmaceutical composition.

In various cases described herein, the pH of the stomach is increased to a pH about 3, or a pH above 3.5, or a pH above 4, or a pH above 4.5, or a pH above 5, or a pH above 5.5, or a pH above 6, or a pH above 6.5, or a pH above 7 within about 45 minutes after administration of the pharmaceutical composition. In other cases described herein, the pH of the stomach is increased to a pH about 3, or a pH above 3.5, or a pH above 4, or a pH above 4.5, or a pH above 5, or a pH above 5.5, or a pH above 6, or a pH above 6.5, or a pH above 7 within about 30 minutes after administration of the pharmaceutical composition. In still other cases, the pH of the stomach is increased to a pH about 3, or a pH above 3.5, or a pH above 4, or a pH above 4.5, or a pH above 5, or a pH above 5.5, or a pH above 6, or a pH above 6.5, or a pH above 7 within about 15 minutes after administration of the pharmaceutical composition.

In one case, the pharmaceutical composition comprises an amount of buffering agent sufficient to increase the pH of the gastric fluid to a target pH for a period of time. Where the gastric fluid is the stomach of a subject, the period of time is generally sufficient for the pharmaceutical agent to be absorbed into the blood stream. Illustratively, the pH is about 3 to about 8, or greater than about 3, or about 3.5, or about 4, or about 4.5, or about 5, or about 5.5, or about 6, or about 6.5, or about 7, or about 7.5, or about 8, or about 8.5, or about 9.0, or about 9.5, or about 10. The particular target pH can depend, among other things, on the particular pharmaceutical agent utilized in the composition, and its acid labile characteristics (for example, its pKa).

Pharmacokinetic and pharmacodynamic data can be obtained by known techniques in the art. Due to the inherent variation in pharmacokinetic and pharmacodynamic parameters of drug metabolism in human subjects, appropriate pharmacokinetic and pharmacodynamic profile components describing a particular composition can vary. Typically, pharmacokinetic and pharmacodynamic profiles are based on the determination of the "mean" parameters of a group of subjects. The group of subjects includes any reasonable number of subjects suitable for determining a representative mean, for example, 5 subjects, 10 subjects, 16 subjects, 20 subjects, 25 subjects, 30 subjects, 35 subjects, or more. The "mean" is determined by calculating the average of all subject's measurements for each parameter measured.

The pharmacokinetic parameters can be any parameters suitable for describing the present composition. For example, in some cases described herein, the Cₘₐₓ can be not less than about 500 ng/ml; not less than about 550 ng/ml; not less than about 600 ng/ml; not less than about 700 ng/ml; not less than about 800 ng/ml; not less than about 850 ng/ml, not less than about 900 ng/ml; not less than about 100 ng/ml; not less than about 1250 ng/ml; not less than about 1500 ng/ml, not less than about 1700 ng/ml, or any other Cmax appropriate for describing the proton pump inhibiting agent pharmacokinetic profile.

In some cases described herein described herein, the Tmax can be, for example, not greater than about 0.5 hours, not greater than about 1.0 hours, not greater than about 1.5 hours, not greater than about 2.0 hours, not greater than about 2.5 hours, or not greater than about 3.0 hours, or any other T max appropriate for describing the proton pump inhibiting agent pharmacokinetic profile.

In some cases described herein, the AUC_{(0-inf)} can be, for example, not less than about 600 ng x hr/ml, not less than about 1500 ng x hr/ml, not less than about 2000 ng x hr/ml, not less than about 3000 ng x hr/ml, not less than about 3850 ng x hr/ml, not less than about 4000 ng x hr/ml, not less than about 5000 ng/ml, not less than about 6000 ng x hr/ml, not less than about 7000 ng x hr/ml, not less than about 8000 ng x hr/ml, not less than about 9000 ng x hr/ml, or any other AUC_{(0-inf)} appropriate for describing the proton pump inhibiting agent pharmacokinetic profile of the inventive composition. The plasma omeprazole concentration about one hour after administration can be, for example, not less than about 50 ng/ml, not less than about 100 ng/ml, not less than about 150 ng/ml, not less than about 400 ng/ml, not less than about 550 ng/ml, not less than about 650 ng/ml, not less than about 700 ng/ml, not less than about 750 ng/ml, not less than about 800 ng/ml, not less than about 900 ng/ml, not less than about 1000 ng/ml, not less than about 1200 ng/ml, or any other plasma proton pump inhibiting agent concentration suitable for describing the inventive composition.

The pharmacodynamic parameters can be any parameters suitable for describing the present composition_ For example, the pharmacodynamic profile can exhibit an integrated acidity of not greater than, for example, about 20 mmol x hr/L, about 30 mmol x hr/L, about 41.5 mmol x hr/L, about 50 mmol x hr/L, about 60 mmol x hr/L, or any other integrated acidity appropriate for describing the inventive composition. The pharmacodynamic profile can exhibit an increased pH above 4.0, e.g., for at least about 2 hours, at least about 3 hours, at least about 4 hours, at least about 4 to about 5 hours, at least about 5 hours, at least about 6 hours, at least about 7 hours, at least about 8 hours or greater, after administration of the composition.

Studies can be conducted to evaluate the bioavailability of a composition described herein using a randomized, balanced, open label, single dose, crossover design. A study, for example, can be performed using 12 healthy male and/or female volunteers between the ages of 18 and 35. Blood samples are removed at 0, 0.5, 1, 2, 3, 4, 6, 8, 10, 12, 15 and 25 hours. The data from each time point is used to derive pharmacokinetic parameters, such as, area under plasma concentration-time curve ("AUC"), including AUC₍₀₋₁₎, AUC_{(0-inf)}, mean peak plasma concentration (Cₘₐₓ) and time to mean peak plasma concentration (Tₘₐₓ)- The data can be used to confirm that the composition described herein provides the appropriate release characteristics.

In a further case described herein, the pharmaceutical composition is administered in an amount to achieve a measurable serum concentration of a non-acid degraded proton pump inhibiting agent greater than about 100 ng/mL within about 30 minutes after administration of the pharmaceutical composition. In another case described herein, the pharmaceutical composition is administered to the subject in an amount to achieve a measurable serum concentration of a non-acid degraded or non-acid reacted proton pump inhibiting agent greater than about 100 ng/mL within about 15 minutes after administration of the pharmaceutical composition. In yet another case, the pharmaceutical composition is administered to the subject in an amount to achieve a measurable serum concentration of a non-acid degraded or non-acid reacted proton pump inhibiting agent greater than about 100 ng/mL within about 10 minutes after administration of the pharmaceutical composition.

In another case of the present invention, the composition is administered to the subject in an amount to achieve a measurable serum concentration of the proton pump inhibiting agent greater than about 150 ng/ml within about 15 minutes and to maintain a serum concentration of the proton pump inhibiting agent of greater than about 150 ng/ml from about 15 minutes to about 1 hour after administration of the composition. In yet another case described herein, the composition is administered to the subject in an amount to achieve a measurable serum concentration of the proton pump inhibiting agent greater than about 250 ng/ml within about 15 minutes and to maintain a serum concentration of the proton pump inhibiting agent of greater than about 250 ng/ml from about 15 minutes to about 1 hour after administration of the composition. In another case of the present invention, the composition is administered to the subject in an amount to achieve a measurable serum concentration of the proton pump inhibiting agent greater than about 350 ng/ml within about 15 minutes and to maintain a serum concentration of the proton pump inhibiting agent of greater than about 350 ng/ml from about 15 minutes to about 1 hour after administration of the composition. In another case of the present invention, the composition is administered to the subject in an amount to achieve a measurable serum concentration of the proton pump inhibiting agent greater than about 450 ng/ml within about 15 minutes and to maintain a serum concentration of the proton pump inhibiting agent of greater than about 450 ng/ml from about 15 minutes to about 1 hour after administration of the composition.

In another case of the present invention, the composition is administered to the subject in an amount to achieve a measurable serum concentration of the proton pump inhibiting agent greater than about 150 ng/ml within about 30 minutes and to maintain a serum concentration of the proton pump inhibiting agent of greater than about 150 ng/ml from about 30 minutes to about 1 hour after administration of the composition. In yet another case described herein, the composition is administered to the subject in an amount to achieve a measurable serum concentration of the proton pump inhibiting agent greater than about 250 ng/ml within about 30 minutes and to maintain a serum concentration of the proton pump inhibiting agent of greater than about 250 ng/ml from about 30 minutes to about 1 hour after administration of the composition. In another case of the present invention, the composition is administered to the subject in an amount to achieve a measurable serum concentration of the proton pump inhibiting agent greater than about 350 ng/ml within about 30 minutes and to maintain a serum concentration of the proton pump inhibiting agent of greater than about 350 ng/ml from about 30 minutes to about 1 hour after administration of the composition. In another case described herein, the composition is administered to the subject in an amount to achieve a measurable serum concentration of the proton pump inhibiting agent greater than about 450 ng/ml within about 30 minutes and to maintain a serum concentration of the proton pump inhibiting agent of greater than about 450 ng/ml from about 30 minutes to about 1 hour after administration of the composition.

In still another case of the present invention, the composition is administered to the subject in an amount to achieve a measurable serum concentration of a non-acid degraded or non-acid reacted proton pump inhibiting agent greater than about 500 ng/mL within about 1 hour after administration of the composition. In yet another case described herein, the composition is administered to the subject in an amount to achieve a measurable serum concentration of a non-acid degraded or non-acid reacted proton pump inhibiting agent greater than about 300 ng/mL within about 45 minutes after administration of the composition.

In another case of the present invention, the composition is administered to the subject in an amount sufficient to achieve a maximum serum concentration (Cmax) at a time (Tmax) that is within about 90, 70, 60, 50, 40, 30 or 20 minutes after administration of the composition according to the present invention.

In still another case of the invention, the composition is administered to the subject in an amount sufficient to achieve a maximum serum concentration (Cmax) at a time (Tmax) that is between about 10 and about 90 minutes, between about 10 to about 60 minutes, between about 15 to about 60 minutes or between about 20 to about 60 minutes after administration of the composition according to the present invention. In some cases described herein, the Tmax is between about 10 and about 45 minutes. In some cases, the Tmax is between about 10 and 45 minutes on day 1 and day 7 after administration. In some cases, the Tmax is between about 10 and 60 minutes on day 1 and day 7 after administration. In some cases, the Tmax is between about 10 and 30 minutes on day 1 and day 7 after administration. In some cases, the Tmax is substantially the same on day 1 of administration as it is on day 7 of administration. In some cases, the Tmax on day 1 and day 7 is about 30 minutes. In some cases, the Tmax on day 1 and day 7 is about 45 minutes. In some cases, the Tmax on day 1 and day 7 is about 60 minutes. In some specific cases, the values of Cmax and Tmax are averages over a test population. In other specific cases, the values of Cmax and Tmax are the values for an individual.

In still another case, the composition is administered in an amount sufficient to achieve a maximum serum concentration (Cmax) of from about 400 to about 2000 ng/mL, from about 400 to about 1500 ng/mL, from about 1000 to about 1500 ng/mL, from about 400 to about 1000 ng/mL or from about 400 to about 700 ng/rnL. In some specific cases, the values of Cmax and Tmax are averages over a test population. In other specific cases, the values of Cmax and Tmax are the values for an individual.

In a further case, the composition is administered in an amount sufficient to achieve a maximum serum concentration (Cmax) of greater than 400 ng/mL, greater than 600 ng/mL, greater than 1000 ng/mL. In some specific cases, the values of Cmax and Tmax are averages over a test population. In other specific cases, the values of Cmax and Tmax are the values for an individual.

In one case of the present invention, the composition is administered to a subject in a gastrointestinal-disorder-effective amount, that is, the composition is administered in an amount that achieves a therapeutically-effective dose of a proton pump inhibiting agent in the blood serum of a subject for a period of time to elicit a desired therapeutic effect. Illustratively, in a fasting adult human (fasting for generally at least 10 hours) the composition is administered to achieve a therapeutically-effective dose of a proton pump inhibiting agent in the blood serum of a subject within about 45 minutes after administration of the composition. In another case of the present invention, a therapeutically-effective dose of the proton pump inhibiting agent is achieved in the blood serum of a subject within about 30 minutes from the time of administration of the composition to the subject. In yet another case, a therapeutically-effective dose of the proton pump inhibiting agent is achieved in the blood serum of a subject within about 20 minutes from the time of administration to the subject. In still another case of the present invention, a therapeutically-effective dose of the proton pump inhibiting agent is achieved in the blood serum of a subject at about 15 minutes from the time of administration of the composition to the subject.

In further cases, the oral bioavailability of the proton pump inhibitor is at least about 25%. In other cases, the oral bioavailability of the proton pump inhibitor is at least about 30%. In still other cases, the oral bioavailability of the proton pump inhibitor is at least 35%, or at least 40%, or at least 45%, or at least 50%, or at least 55% bioavailable, or at least 60%.

In alternative cases, the pharmaceutical composition comprises at least about 5 mEq of antacid and is bioequivalent to a proton pump inhibitor product such as Prilosec®, Nexium®, Prevacid®, Protonix®, or Aciphex®. In other cases, the pharmaceutical composition comprises between about 5 mEq to about 30 mEq of antacid and is bioequivalent to a proton pump inhibitor product such as Prilosec®, Nexium®, Prevacid®, Protonix®, or Aciphex®. In still other cases, the pharmaceutical composition comprises between about 5 mEq to about 30 mEq, or about 5 mEq, or about 7 mEq, or about 10 mEq, or about 13 mEq, or about 15 mEq, or about 17 mEq, or about 20 mEq, or about 22 mEq, or about 25 mEq, or about 27 mEq, or about 30 mEq of antacid and is bioequivalent to a proton pump inhibitor product such as Prilosec®, Nexium®, Prevacid®, Protonix®, or Aciphex®. "Bioequivalent" is intended to mean that the area under the serum concentration time curve (AUC) and the peak serum concentration (Cmax) are each within 80% and 125%.

In alternative cases, the pharmaceutical composition comprises at least about 5 mEq of antacid and is bioequivalent to a proton pump inhibitor product such as Prilosec®, Nexium®, Prevacid®, Protonix®, or Aciphex®. In other cases, the pharmaceutical composition comprises between about 5 mEq to about 11 mEq of antacid and is bioequivalent to a proton pump inhibitor product such as Prilosec®, Nexium®, Prevacid®, Protonix®, or Aciphex®. In still other cases, the pharmaceutical composition comprises between about 5 mEq to about 11 mEq, or about 5 mEq, or about 6 mEq, or about 7 mEq, or about 8 mEq, or about 9 mEq, or about 10 mEq, or about 11 mEq of antacid and is bioequivalent to a proton pump inhibitor product such as Prilosec®, Nexium®, Prevacid®, Protonix®, or Aciphex®.

In other cases, when administered to a subject, the pharmaceutical composition has an area under the serum concentration time curve (AUC) for the proton pump inhibitor that is equivalent to the area under the serum concentration time curve (AUC) for the proton pump inhibitor when the enteric form of the proton pump inhibitor is delivered without antacid. "Equivalent" is intended to mean that the area under the serum concentration time curve (AUC) for the proton pump inhibitor is within 30% of the area under the serum concentration time curve (AUC) when the same dosage amount of the proton pump inhibitor is enterically coated and delivered to the subject with less than 1 mEq of antacid. The "enteric form of the proton pump inhibitor" is intended to mean that some or most of the proton pump inhibitor has been enterically coated to ensure that at least some of the drug is released in the proximal region of the small intestine (duodenum), rather than the acidic environment of the stomach.

Compositions contemplated by the present invention provide a therapeutic effect as proton pump inhibiting agent medications over an interval of about 5 minutes to about 24 hours after administration, enabling, for example, once-a-day, twice-a-day, or three times a day administration if desired. Generally speaking, one will desire to administer an amount of the compound that is effective to achieve a serum level commensurate with the concentrations found to be effective in vivo for a period of time effective to elicit a therapeutic effect. Determination of these parameters is well within the skill of the art.

The compositions of the present invention can also be evaluated under a variety of dissolution conditions to determine the effects of pH, media, agitation and apparatus. For example, dissolution tests can be performed using a USP type apparatus. Effects of pH, agitation, polarity, enzymes and bile salts can also be evaluated.

### Treatment

Initial treatment of a subject suffering from a disease, condition or disorder where treatment with an inhibitor of H⁺/K⁺-ATPase is indicated can begin with the dosages indicated above. Treatment is generally continued as necessary over a period of hours, days, or weeks to several months or years until the disease, condition or disorder has been controlled or eliminated. Subjects undergoing treatment with the compositions disclosed herein can be routinely monitored by any of the methods well known in the art to determine the effectiveness of therapy. Continuous analysis of such data permits modification of the treatment regimen during therapy so that optimal effective amounts of compounds described herein are administered at any point in time, and so that the duration of treatment can be determined as well. In this way, the treatment regimen/dosing schedule can be rationally modified over the course of therapy so that the lowest amount of an inhibitor of H⁺1K⁺-ATPase exhibiting satisfactory effectiveness is administered, and so that administration is continued only so long as is necessary to successfully treat the disease, condition or disorder.

Besides being useful for human treatment, the present invention is also useful for other subjects including veterinary animals, reptiles, birds, exotic animals and farm animals, including mammals, rodents, and the like. Mammals include primates, e.g., a monkey, or a lemur, horses, dogs, pigs, or cats. Rodents includes rats, mice, squirrels, or guinea pigs.

In one case, the pharmaceutical formulations are useful for treating a condition, disease or disorder where treatment with a proton pump inhibitor is indicated. In other cases, the treatment method comprises oral administration of one or more compositions of the present invention to a subject in need thereof in an amount effective at treating the condition, disease, or disorder. In another case, the disease, condition or disorder is a gastrointestinal disorder.

Also described are methods of treating, preventing, reversing, halting or slowing the progression of a gastrointestinal disorder once it becomes clinically evident, or treating the symptoms associated with, or related to the gastrointestinal disorder, by administering to the subject a composition of the present invention. The subject may already have a gastrointestinal disorder at the time of administration, or be at risk of developing a gastrointestinal disorder. The symptoms or conditions of a gastrointestinal disorder in a subject can be determined by one skilled in the art and are described in standard textbooks. The method comprises the oral administration a gastrointestinal-disorder-effective amount of one or more compositions of the present invention to a subject in need thereof.

Gastrointestinal disorders include, e.g., duodenal ulcer disease, gastrointestinal ulcer disease, gastroesophageal reflux disease, erosive esophagitis, poorly responsive symptomatic gastroesophageal reflux disease, pathological gastrointestinal hypersecretory disease, Zollinger Ellison Syndrome, acid dyspepsia nighttime gastric acidity, and nocturnal acid breakthrough. In one case described herein, the gastrointestinal disorder is heartburn.

### Nocturnal Acid Breakthrough

Gastroesophageal reflux disease (GERD) is a condition in which acid reflux irritates the esophageal walls, which thereby induces peristaltic contraction of the esophageal smooth muscle. A subject suffering from GERD may experience discomfort and even pain (commonly referred to as "heartburn") depending on the severity of the irritation and subsequent contraction to clear the refluxed acid. GERD is commonly treated by administering a compound to reduce the production of gastric acid (e.g., a proton pump inhibitor). Typically GERD episodes occur during the early daytime hours, but some GERD sufferers experience reflux during the night despite treatment with compounds to reduce the production of gastric acid. These nighttime episodes of reflux are referred to as nocturnal acid breakthrough ("NAB").

As used herein, the term "nocturnal acid breakthrough" or "NAB" refers to a nocturnal gastric pH less than 4 for greater than one hour in a subject treated with a compound to reduce the production of gastric acid. The compositions and methods described herein are useful for the treatment of NAB. In some cases, the compositions described herein are more effective at treating and/or preventing NAB than enteric coated PPI formulations.

In some cases, the compositions of the present invention are more effective at treating/preventing nocturnal acid breakthrough than enteric coated and/or delayed-release formulations. A composition is more effective at treating/preventing nocturnal acid breakthrough if the percent of patients that have NAB are lower than the percent of patients that have NAB after treatment with the control compound. In one case, the compositions of the present invention are more effective at treating NAB than enteric coated formulations *(e.g,* Prevacid and/or Nexium) in the first two hours of the night. In other cases, the compositions described herein are more effective at treating NAB than enteric coated formulations *(e.g,* Prevacid and/or Nexium) in the first four hours of the night. In still other cases, the compositions described herein are more effective at treating NAB than enteric coated formulations *(e.g,* Prevacid and/or Nexium) in the first six hours of the night. In yet other cases, the compositions described herein are more effective at treating NAB than enteric coated formulations (e.g, Prevacid and/or Nexium) in the first eight hours of the night.

In some cases, the compositions described herein are at least about 5%, or about 10%, or about 15%, or about 20%, or about 25%, or about 30%, or about 35%, or about 40%, or about 50%, or about 60%, or about 65%, or about 70% more effective at treating NAB than enteric coated formulations (e.g, Prevacid and/or Nexium) in the first two hours of the night. In other cases, the compositions of the present invention are at least about 5%, or about 10%, or about 15%, or about 20%, or about 25%, or about 30%, or about 35%, or about 40%, or about 50% more effective at treating NAB than enteric coated formulations (e.g, Prevacid and/or Nexium) in the first four hours of the night. In still other cases, the compositions described herein are at least about 5%, or about 10%, or about 15%, or about 20%, or about 25%, or about 30%, or about 35%, or about 40%, or about 50% more effective at treating NAB than enteric coated formulations (e.g, Prevacid and/or Nexium) in the first six hours of the night. In yet other cases, the compositions described herein are at least about 5%, or about 10%, or about 15%, or about 20%, or about 25%, or about 30%, or about 35%, or about 40%, or about 50% more effective at treating NAB than enteric coated formulations *(e.g,* Prevacid and/or Nexium) throughout the night.

A composition is "more effective" at treating and or preventing nighttime gastric acidity than an enteric coated or delayed release formulation if: (1) the percent of time the gastric pH is greater than 4 is higher than the reference compound; (2) the median gastric pH is higher than the reference compound; and/or (3) the cumulative Integrated Gastric Acidity "IGA" is lower than the reference compound. In some cases, the compositions of the present invention are more effective than Prevacid capsules (40 mg). In other cases, the compositions described herein are more effective than Nexium capsules (40 mg).

In some cases, the compositions described herein are at least about 5%, or about 10%, or about 15%, or about 20%, or about 25%, or about 30%, or about 35%, or about 40%, or about 50%, or about 60%, or about 65%, or about 70% more effective at treating nighttime gastric acidity than enteric coated formulations (e.g, Prevacid and/or Nexium) in the first two hours of the night. In other cases, the compositions described herein are at least about 5%, or about 10%, or about 15%, or about 20%, or about 25%, or about 30%, or about 35%, or about 40%, or about 50% more effective at treating nighttime gastric acidity than enteric coated formulations *(e.g,* Prevacid and/or Nexium) in the first four hours of the night. In still other cases, the compositions described herein are at least about 5%, or about 10%, or about 15%, or about 20%, or about 25%, or about 30%, or about 35%, or about 40%, or about 50% more effective at treating nighttime gastric acidity than enteric coated formulations (e.g, Prevacid and/or Nexium) in the first six hours of the night. In yet other cases, the compositions of the present invention are at least about 5%, or about 10%, or about 15%, or about 20%, or about 25%, or about 30%, or about 35%, or about 40%, or about 50% more effective at treating nighttime gastric acidity than enteric coated formulations *(e.g,* Prevacid and/or Nexium) throughout the night.

In some cases, the compositions of the present invention are administered less than one hour before retiring to bed and maintain the gastric pH above 4 for about 50% of the next 8 hours. In other cases, the compositions of the present invention are administered less than one hour before retiring to bed and maintain the gastric pH about 4 for about 60% of the next 8 hours. In some cases, the compositions described herein are administered less than one hour before retiring to bed and maintain the gastric pH about 4 for about 70% of the next 8 hours. In other cases, the compositions described herein are administered less than one hour before retiring to bed and maintain the gastric pH about 4 for about 80% of the next 8 hours. In yet other cases, the compositions of the present invention are administered at bedtime and maintain the gastric pH above 4 for about 90% of the next 8 hours. In yet other cases, the compositions described herein are administered at bedtime and maintain the gastric pH above 4 for about 95% of the next 8 hours.

In other cases, the compositions of the present invention are administered at bedtime and maintain the gastric pH above 4 between about 50% and about 60% of the next 8 hours. In some cases, the compositions of the present invention are administered at bedtime and maintain the gastric pH above 4 between about 60% and about 70% of the next 8 hours. In other cases, the compositions described herein are administered at bedtime and maintain the gastric pH above 4 between about 70% and about 80% of the next 8 hours. In other cases, the compositions of the present invention are administered at bedtime and maintain the gastric pH above 4 between about 80% and about 90% of the next 8 hours. In yet other cases, the compositions described herein are administered at bedtime and maintain the gastric pH above 4 between about 90% and about 98% of the next 8 hours.

In some cases, the compositions of the present invention are administered at bedtime and the median gastric pH over the next 8 hours is greater than about 4. In some cases, the compositions described herein are administered at bedtime and the median gastric pH over the next 8 hours is about 4.5. In some cases, the compositions of the present invention are administered at bedtime and the median gastric pH over the next 8 hours is about 5. In other cases, the compositions described herein are administered at bedtime and the median gastric pH over the next 8 hours is greater than about 5.5. In some cases, the compositions described herein are administered at bedtime and the median gastric pH over the next 8 hours is about 6.

In some cases, the compositions of the present invention are administered at bedtime for seven consecutive days and the percentage of patients with nocturnal acid breakthrough during the 2 hours following administration is less than about 80%. In some cases, the compositions described herein are administered at bedtime for seven consecutive days and the percentage of patients with nocturnal acid breakthrough during the 2 hours following administration is less than about 70%. In some cases, the percentage of patients with nocturnal acid breakthrough during the 2 hours following administration is less than about 60%. In some cases, the compositions of the present invention are administered at bedtime for seven consecutive days and the percentage of patients with nocturnal acid breakthrough during the 2 hours following administration is less than about 50%. In still other cases, the percentage of patients with nocturnal acid breakthrough during the 2 hours following administration is less than about 40%. In some cases, the compositions described herein are administered at bedtime for seven consecutive days and the percentage of patients with nocturnal acid breakthrough during the 2 hours following administration is less than about 30%. In other cases, the percentage of patients with nocturnal acid breakthrough during the 2 hours following administration is less than about 20%.

In some cases, the compositions of the present invention are administered at bedtime for seven consecutive days and the percentage of patients with nocturnal acid breakthrough during the 4 hours following administration is less than about 80%. In some cases, the compositions described herein are administered at bedtime for seven consecutive days and the percentage of patients with nocturnal acid breakthrough during the 4 hours following administration is less than about 70%. In some cases, the percentage of patients with nocturnal acid breakthrough during the 4 hours following administration is less than about 60%. In some cases, the compositions of the present invention are administered at bedtime for seven consecutive days and the percentage of patients with nocturnal acid breakthrough during the 4 hours following administration is less than about 50%. In still other cases, the percentage of patients with nocturnal acid breakthrough during the 4 hours following administration is less than about 40%. In some cases, the compositions described herein are administered at bedtime for seven consecutive days and the percentage of patients with nocturnal acid breakthrough during the 4 hours following administration is less than about 30%. In other cases, the percentage of patients with nocturnal acid breakthrough during the 4 hours following administration is less than about 20%.

In some cases, the compositions of the present invention are administered at bedtime for seven consecutive days and the percentage of patients with nocturnal acid breakthrough during the 6 hours following administration is less than about 80%. In some cases, the compositions described herein are administered at bedtime for seven consecutive days and the percentage of patients with nocturnal acid breakthrough during the 6 hours following administration is less than about 70%. In some cases, the percentage of patients with nocturnal acid breakthrough during the 6 hours following administration is less than about 60%. In some cases, the compositions of the present invention are administered at bedtime for seven consecutive days and the percentage of patients with nocturnal acid breakthrough during the 6 hours following administration is less than about 50%. In still other cases, the percentage of patients with nocturnal acid breakthrough during the 6 hours following administration is less than about 40%. In some cases, the compositions of the present invention are administered at bedtime for seven consecutive days and the percentage of patients with nocturnal acid breakthrough during the 6 hours following administration is less than about 30%. In other cases, the percentage of patients with nocturnal acid breakthrough during the 6 hours following administration is less than about 20%.

### Dosage Forms

The pharmaceutical compositions of the present invention contain desired amounts of proton pump inhibitor and antacid and can be in the form of: a tablet, (including a suspension tablet, a chewable tablet, a fast- melt tablet, a bite-disintegration tablet, a rapid-disintegration tablet, an effervescent tablet, or a caplet), a pill, a powder (including a sterile packaged powder, a dispensable powder, or an effervescent powder) a capsule (including both soft or hard capsules, e.g., capsules made from animal-derived gelatin or plant-derived HPMC) a lozenge, a sachet, a troche, pellets, granules, or an aerosol. The pharmaceutical compositions described herein can be manufactured by conventional pharmacological techniques.

In some cases, the pharmaceutical compositions of the present invention contain desired amounts of proton pump inhibiting inhibitor and antacid and are in a solid dosage form. In other cases, the pharmaceutical compositions of the present invention contain desired amounts of proton pump inhibitor and antacid and are administered in the form of a capsule (including both soft and hard capsules, e.g., capsules made from animal-derived gelatin or plant-derived HPMC). The pharmaceutical compositions described herein can be manufactured by conventional pharmacological techniques.

Conventional pharmacological techniques include, e.g., one or a combination of methods: (1) dry mixing, (2) direct compression, (3) milling, (4) dry or non-aqueous granulation, (5) wet granulation, or (6) fusion. See, e.g., Lachman et al., The Theory and Practice of Industrial Pharmacy (1986). Other methods include, e.g., prilling, spray drying, pan coating, melt granulation, granulation, wurster coating, tangential coating, top spraying, extruding, coacervation and the like.

In one case, the proton pump inhibitor is microencapsulated prior to being formulated into one of the above forms. In another case, some of the proton pump inhibitor is microencapsulated prior to being formulated. In another case, some or all of the antacid is microencapsulated prior to being formulated. In still another case, some or most of the proton pump inhibitor is coated prior to being further formulated by using standard coating procedures, such as those described in Remington's Pharmaceutical Sciences, 20th Edition (2000). In yet other cases contemplated by the present invention, a film coating is described around the pharmaceutical composition.

In other cases, the pharmaceutical compositions further comprise one or more additional materials such as a pharmaceutically compatible carrier, binder, filling agent, suspending agent, flavoring agent, sweetening agent, disintegrating agent, surfactant, preservative, lubricant, colorant, diluent, solubilizer, moistening agent, stabilizer, wetting agent, anti-adherent, parietal cell activator, anti-foaming agent, antioxidant, chelating agent, antifungal agent, antibacterial agent, or one or more combination thereof.

In other cases, one or more layers of the pharmaceutical formulation are plasticized. Illustratively, a plasticizer is generally a high boiling point solid or liquid. Suitable plasticizers can be added from about 0.01% to about 50% by weight (w/w) of the coating composition. Plasticizers include, e.g., diethyl phthalate, citrate esters, polyethylene glycol, glycerol, acetylated glycerides, triacetin, polypropylene glycol, polyethylene glycol, triethyl citrate, dibutyl sebacate, stearic acid, stearol, stearate, and castor oil.

In some cases, some or all of the proton pump inhibitor is in a sustained release form. In some of these cases, the sustained release form comprises about 10-80 wt-% of a slowly soluble polymer. In some cases the sustained release form comprises about 10-20 wt-%, or about 20-30 wt-%, or about 30-40 wt-%, or about 40-50 wt-%, or about 50-60 wt-%, or about 60-70 wt-%, or about 70-80 wt-% of a polymer. In some cases, the polymer is a solowly soluble polyer or a combination of slowly soluble polymers. In various cases, the polymer is a cellulose ether polymer, including but not limited to HPC, HPMC or HEC. In various other cases, the polyer is a polyethylene oxide. As used herein, the term "slowly soluble polyer" refers to a polymer that releases less than about 50% of the proton pump inhibitor within 2 hours.

### Exemplary Solid Oral Dosage Forms

Solid oral dosage compositions, e.g., tablets (such as chewable tablets, effervescent tablets and caplets), and capsules, can be prepared, for example, by mixing the proton pump inhibitor, one or more antacid, and pharmaceutical excipients to form a bulk blend composition. When referring to these bulk blend compositions as homogeneous, it is meant that the proton pump inhibitor and antacid are dispersed evenly throughout the composition so that the composition may be readily subdivided into equally effective unit dosage forms, such as tablets, pills, and capsules. The individual unit dosages may also comprise film coatings, which disintegrate upon oral ingestion or upon contact with diluent.

Compressed tablets are solid dosage forms prepared by compacting the bulk blend compositions described above. In various cases, compressed tablets of the present invention will comprise one or more functional excipients such as binding agents and/or disintegrants. In other cases, the compressed tablets will comprise a film surrounding the final compressed tablet. In other cases, the compressed tablets comprise one or more excipients and/or flavoring agents.

A chewable tablet may be prepared by compacting bulk blend compositions, described above. In one case, the chewable tablet comprises a material useful for enhancing the shelf life of the pharmaceutical composition. In another case, the microencapsulated material has taste-masking properties. In various other cases, the chewable tablet comprises one or more flavoring agents and one or more taste-masking materials. In yet other cases the chewable tablet comprised both a material useful for enhancing the shelf life of the pharmaceutical formulation and one or more flavoring agents.

In various cases, the proton pump inhibitor, antacid, and optionally one or more excipients, are dry blended and compressed into a mass, such as a tablet or caplet, having a hardness sufficient to provide a pharmaceutical composition that substantially disintegrates within less than about 30 minutes, less than about 35 minutes, less than about 40 minutes, less than about 45 minutes, less than about 50 minutes, less than about 55 minutes, or less than about 60 minutes, after oral administration, thereby releasing the antacid and the proton pump inhibitor into the gastrointestinal fluid. When at least 50% of the pharmaceutical composition has disintegrated, the compressed mass has substantially disintegrated.

A capsule may be prepared by placing any of the bulk blend compositions described above, into a capsule. In some cases of the present invention, the therapeutic dose is split into multiple (e.g., two, three, or four) capsules. In some cases, the entire dose of the proton pump inhibitor and antacid are delivered in a capsule form. For example, the capsule may comprise between about 10 mg to about 120 mg of a proton pump inhibitor and between about 5 mEq to about 30 mEq of antacid. In some cases, the antacid may be selected from sodium bicarbonate, magnesium hydroxide, calcium carbonate, magnesium oxide, and mixtures thereof. In alternative cases the capsule comprises 5 mEq to about 30 mEq of sodium bicarbonate.

### Exemplary Powder Compositions

A powder for suspension may be prepared by combining at least one acid labile proton pump inhibitor and between about 5 mEq to about 50 mEq of antacid. In various cases, the powder may comprise one more pharmaceutical excipients and flavors. A powder for suspension may be prepared, for example, by mixing the proton pump inhibitor, one or more antacids, and optional pharmaceutical excipients to form a bulk blend composition. This bulk blend is uniformly subdivided into unit dosage packaging or multi-dosage packaging units. The term "uniform" means the homogeneity of the bulk blend is substantially maintained during the packaging process.

In some cases, some or all of the proton pump inhibitor is micronized. Additional cases described herein also comprise a suspending agent and/or a wetting agent.

Effervescent powders are also prepared in accordance with the present invention. Effervescent salts have been used to disperse medicines in water for oral administration. Effervescent salts are granules or coarse powders containing a medicinal agent in a dry mixture, usually composed of sodium bicarbonate, citric acid and/or tartaric acid. When salts of the present invention are added to water, the acids and the base react to liberate carbon dioxide gas, thereby causing "effervescence." Examples of effervescent salts include, e.g., the following ingredients: sodium bicarbonate or a mixture of sodium bicarbonate and sodium carbonate, citric acid and/or tartaric acid. Any acid-base combination that results in the liberation of carbon dioxide can be used in place of the combination of sodium bicarbonate and citric and tartaric acids, as long as the ingredients were suitable for pharmaceutical use and result in a pH of about 6.0 or higher.

The method of preparation of the effervescent granules of the present invention employs three basic processes: wet granulation, dry granulation and fusion. The fusion method is used for the preparation of most commercial effervescent powders. It should be noted that, although these methods are intended for the preparation of granules, the formulations of effervescent salts of the present invention could also be prepared as tablets, according to known technology for tablet preparation.

### Powder for Suspension

In some cases, compositions are described comprising a pharmaceutical at least one proton pump inhibitor, about 5 mEq to about 50 mEq of an antacid, in some cases between about 5 mEq to about 11 mEq antacid, and at least one suspending agent for oral administration to a subject. The composition may be a powder for suspension, and upon admixture with water, a substantially uniform suspension is obtained.

A suspension is "substantially uniform" when it is mostly homogenous, that is, when the suspension is composed of approximately the same concentration of proton pump inhibitor at any point throughout the suspension. A suspension is determined to be composed of approximately the same concentration of proton pump inhibitor throughout the suspension when there is less than about 20%, less than about 15%, less than about 13%, less than about 11%, less than about 10%, less than about 8%, less than about 5%, or less than about 3% variation in concentration among samples taken from various points in the suspension.

The concentration at various points throughout the suspension can be determined by any suitable means known in the art. For example, one suitable method of determining concentration at various points involves dividing the suspension into three substantially equal sections: top, middle and bottom. The layers are divided starting at the top of the suspension and ending at the bottom of the suspension. Any number of sections suitable for determining the uniformity of the suspension can be used, such as for example, two sections, three sections, four sections, five sections, or six or more sections.

In one case, the composition comprises at least one proton pump inhibitor, between about 5 mEq to about 50 mEq antacid, in some cases between about 5 mEq to about 11 mEq of antacid, and a gum suspending agent, wherein the average particle size of the insoluble material is less than about 200 µm. In some cases, the average particle size of the insoluble material is less than about 100 µm. In other cases, the average particle size of the insoluble material is less than about 50 µm. The composition is a powder for suspension, and upon admixture with water, a first suspension is obtained that is substantially more uniform when compared to a second suspension comprising the proton pump inhibitor, the antacid, and suspending agent, wherein the suspending agent is not xanthan gum.

In another case, the composition comprises omeprazole, sodium bicarbonate and xanthan gum. The composition is a powder for suspension, and upon admixture with water, a substantially uniform suspension is obtained. In yet another case, the composition is a powder for suspension and comprises omeprazole, about 5 mEq to about 50 mEq sodium bicarbonate, in some case between about 5 mEq to about 11 mEq sodium bicarbonate, xanthan gum, and at least one sweetener or flavoring agent.

### Combination Therapy

The present pharmaceutical compositions can also be used in combination ("combination therapy") with another pharmaceutical agent.

In some cases, the second pharmaceutical agent is one that is indicated for treating or preventing a gastrointestinal disorder, such as, e.g., an anti-bacterial agent, an alginate, a prokinetic agent, or an H2 antagonist which are commonly administered to minimize the pain and/or complications related to this disorder. In other cases, the pharmaceutical formulations of the present invention are administered with low strength enteric coated Aspirin or another NSAID. In another case, the second active pharmaceutical, e.g., Aspirin or an NSAID, used in combination with the pharmaceutical formulations of the present invention, is enteric coated. In other cases, antacid present in the pharmaceutical formulations of the present invention increase the pH level of the gastrointestinal fluid, thereby allowing part or all of the enteric coating on the second active pharmaceutical to dissolve in the stomach. In yet another case, the second pharmaceutical agent is a sleep aid.

### EXAMPLES

Understanding of the present invention is further aided by the following examples.

### Example 1A: Capsule Formulations

### Capsulated Omeprazole Formulations with Two Different Lubricants

The following specific formulations and examples are described by way of illustrating the present invention and are not intended to be limiting.

40 mg capsules were prepared by blending the indicated amount of micronized omeprazole and about half the indicated amount of sodium bicarbonate according to the ingredients listed in Table 1A1. After blending the omeprazole and sodium bicarbonate, the remaining sodium bicarbonate was added along with the indicated amount of croscarmellose sodium and magnesium stearate. Once the omeprazole was homogeneously blended with the excipients, the appropriate weight of composition was filled into hard gelatin capsules using a tamping pin-type automatic encapsulator.

**Table 1A1: 40 mg formulation with omeprazole and magnesium stearate**

| | | |
|---|---|---|
| **Component** | **%** | **mg/cap** |

20 mg capsules were prepared in the same manner as the 40 mg capsules, pursuant to the ingredients depicted in Table 1A2.

**Table 1A2: 20 mg formulation with omeprazole and magnesium stearate**

| **Component** | **%** | **Mg/cap** |
|---|---|---|
| Omeprazole USP | 1.8 | 20 |
| Sodium Bicarbonate USP # 2 | 94.8 | 1100 |
| Croscarmellose Sodium, NF | 2.6 | 30 |
| Magnesium Stearate, NF | 0.9 | 10 |
| Totals | 100 | 1160 |

### Magnesium Stearate Lubricant v. No Lubricant

Several in vitro studies were performed to evaluate the effect of using different lubricants in the omeprazole pharmaceutical formulations. These studies utilized the developmental in vitro gastric acid Kinetic Stomach Model illustrated in Fig. 1B. First, an in vitro study analyzing two different formulations was performed. One formulation included all of the ingredients listed in Table 1A1, including the magnesium stearate lubricant. The second formulation included all of the same ingredients except omitted the magnesium stearate lubricant. Both of these samples were then evaluated in a 150 mL dissolution vessel that contained 50 mL of 0.1 N HCl, maintained at constant temperature of about 37°C and stirred at 200 rpm. Stomach acid secretion was simulated through the use of a pump, delivering 1.0 N HCl at a rate of 0.5 rnL/min. The pH was monitored using a probe inserted directly into the dissolution vessel. Antacid performance comparisons were then performed.

Fig. 1A illustrates the comparison in pH profiles between omeprazole formulations lubricated with magnesium stearate and those that are unlubricated. As shown in this figure, a delay of up to five minutes to reach maximum pH is observed with formulation capsules containing magnesium stearate.

### Magnesium Stearate Lubricant v. Sodium Stearyl Fumarate

40 mg and 20 mg capsules of omeprazole with sodium stearyl fumarate were also prepared in the same manner as the omeprazole/magnesium stearate capsules, but pursuant to the ingredients listed in Table 1A3 and Table 1A4.

**Table 1A3: 40 mg formulation with omeprazole and sodium stearyl fumarate**

| **Component** | **%** | **mg/cap** |
|---|---|---|
| Omeprazole USP | 3.5 | 40 |
| Sodium Bicarbonate USP # 2 | 93.2 | 1100 |
| Croscarmellose Sodium, NF | 2.5 | 30 |
| Sodium Stearyl Fumarate, NF | 0.8 | 10 |
| Totals | 100 | 1180 |

**Table 1A4: 20 mg formulation with omeprazole and sodium stearyl fumarate**

| **Component** | **%** | **Mg/cap** |
|---|---|---|
| Omeprazole USP | 1.8 | 20 |
| Sodium Bicarbonate USP # 2 | 94.8 | 1100 |
| Croscarmellose Sodium, NF | 2.6 | 30 |
| Sodium Stearyl Fumarate, NF | 0.9 | 10 |
| Totals | 100 | 1160 |

An in vitro study was performed, using the same steps, methodology and equipment used for the previous Kinetic Stomach model as discussed directly above, to evaluate the impact of replacing magnesium stearate with sodium stearyl fumarate on drug product dissolution and pH. In this study, three encapsulated formulations were prepared. The first formulation was prepared pursuant to Table 1A1 and included magnesium stearate as the lubricant. The second formulation was prepared pursuant to Table 1A3 and included sodium stearyl fumarate as the lubricant. The third formulation included no lubricant, but included all of the ingredients common to both Table 1A1 and Table 1A3. As Fig. 2 indicates, the capsules formulated without lubricant immediately dissolved and demonstrated a rise in pH within the first 2 minutes of sample addition. Samples containing sodium stearyl fumarate as a lubricant also demonstrated an immediate rise in pH and have a nearly identical neutralization profile to samples without lubricant. However, dissolution is delayed by 4 minutes in samples containing the more hydrophobic lubricant magnesium stearate. All of these results of this in vitro study are illustrated in Figure 2.

### Sodium Stearyl Fumarate and Magnesium Stearate at pH 1.4 v. 4.2

An in vitro Kinetic Stomach Model study was performed, using the steps, methodology, and equipment as discussed previously. However, the initial pH of the media was set to pH values of 1.4 and 4.2 to simulate the in-vivo pH conditions of patient's stomach at day 1 dosing and day 7 dosing. Two formulations of omeprazole were subjected to the study, one with magnesium stearate produced pursuant to Table 1A1, and a formulation with sodium stearyl fumarate pursuant to Table 1A3. Figure 3 illustrates the pH profile for both lubricants.

### Physicochemical and Biological Properties of Sodium Stearyl Fumarate Compared to Magnesium Stearate

A multipoint in vitro dissolution study was performed utilizing capsule formulations prepared pursuant to Table 1A1 including magnesium stearate, and capsule formulations prepared pursuant to Table 1A3 with sodium stearyl fumarate. The study was performed with a paddle apparatus and time points of 15, 30, 45, 60, and 75 minutes, utilizing a 75 rpm paddle speed. Table 1A5 provides the corresponding data for capsules with 40 mg omeprazole, and Table 1A6 provides the data for the 20 mg omeprazole capsules.

**Table 1A5: Dissolution Results of 40 mg omeprazole capsules with two different lubricants**

| | **Omeprazole w/ Magnesium Stearate** | | | | | **Omeprazole w/ Sodium Stearyl Fumarate** | | | | |
|---|---|---|---|---|---|---|---|---|---|---|
| | **% Omeprazole Released** | | | | | **% Omeprazole Released** | | | | |
| Time Point (minutes) | 15 | 30 | 45 | 60 | 75 | 15 | 30 | 45 | 60 | 75 |
| Dosage Unit 1 | 91 | 97 | 97 | 96 | 96 | 87 | 93 | 94 | 98 | 97 |
| Dosage Unit 2 | 87 | 95 | 96 | 94 | 95 | 87 | 96 | 96 | 93 | 93 |
| Dosage Unit 3 | 93 | 98 | 98 | 98 | 97 | 81 | 91 | 93 | 95 | 93 |
| Dosage Unit 4 | 92 | 95 | 95 | 94 | 95 | 83 | 91 | 92 | 95 | 95 |
| Dosage Unit 5 | 89 | 97 | 97 | 95 | 96 | 84 | 92 | 94 | 95 | 95 |
| Dosage Unit 6 | 93 | 98 | 97 | 97 | 97 | 87 | 94 | 94 | 95 | 92 |
| Dosage Unit 7 | 91 | 96 | 96 | 95 | 94 | 88 | 94 | 95 | 95 | 97 |
| Dosage Unit 8 | 93 | 98 | 97 | 97 | 96 | 84 | 93 | 94 | 93 | 92 |
| Dosage Unit 9 | 88 | 96 | 96 | 96 | 93 | 83 | 91 | 91 | 93 | 92 |
| Dosage Unit 10 | 93 | 98 | 97 | 96 | 95 | 85 | 91 | 93 | 93 | 93 |
| Dosage Unit 11 | 94 | 98 | 97 | 96 | 96 | 86 | 93 | 95 | 93 | 93 |
| Dosage Unit 12 | 90 | 96 | 95 | 95 | 95 | 87 | 94 | 95 | 97 | 93 |
| Average | 91 | 97 | 97 | 96 | 95 | 85 | 93 | 94 | 95 | 94 |
| %RSD | 2.5 | 1.2 | 0.9 | 1.3 | 1.2 | 2.5 | 1.7 | 1.5 | 1.8 | 1.9 |

**Table 1A6: Dissolution Results of 20 mg omeprazole capsules with two different lubricants**

| | **zegerid® Capsules With Magnesium Stearate** | | | | | **Reformulated zegerid® Capsules With Sodium Stearyl Fumarate** | | | | |
|---|---|---|---|---|---|---|---|---|---|---|
| | % **Omeprazole Released** | | | | | **% Omeprazole Released** | | | | |
| Time Point (minutes) | 15 | 30 | 45 | 60 | 75 | 15 | 30 | 45 | 60 | 75 |
| Dosage Unit 1 | 66 | 100 | 100 | 99 | 99 | 82 | 89 | 91 | 91 | 91 |
| Dosage Unit 2 | 80 | 98 | 98 | 98 | 98 | 80 | 89 | 90 | 92 | 92 |
| Dosage Unit 3 | 55 | 100 | 102 | 102 | 101 | 78 | 85 | 88 | 90 | 93 |
| Dosage Unit 4 | 81 | 101 | 101 | 101 | 100 | 79 | 88 | 90 | 92 | 92 |
| Dosage Unit 5 | 82 | 100 | 100 | 100 | 99 | 79 | 88 | 89 | 90 | 91 |
| Dosage Unit 6 | 70 | 100 | 101 | 100 | 100 | 82 | 90 | 90 | 91 | 91 |
| Dosage Unit 7 | 63 | 100 | 101 | 101 | 100 | 79 | 86 | 88 | 89 | 89 |
| Dosage Unit 8 | 81 | 98 | 99 | 98 | 98 | 78 | 85 | 87 | 88 | 89 |
| Dosage Unit 9 | 77 | 99 | 99 | 98 | 98 | 77 | 84 | 86 | 86 | 87 |
| Dosage Unit 10 | 70 | 98 | 99 | 98 | 98 | 80 | 87 | 88 | 89 | 89 |
| Dosage Unit 11 | 78 | 101 | 101 | 101 | 100 | 77 | 86 | 88 | 88 | 89 |
| Dosage Unit 12 | 79 | 99 | 99 | 98 | 97 | 78 | 87 | 88 | 89 | 89 |
| Average | 74 | 99 | 100 | 100 | 99 | 79 | 87 | 89 | 90 | 90 |
| % RSD | 11. | 1.1 | 1.2 | 1.5 | 1.2 | 2.1 | 2.1 | 1.6 | 2.0 | 1.9 |

### Clinical Trial Study

A small clinical study was performed using capsules formulated with sodium stearyl fumarate and capsules formulated with magnesium stearate. The encapsulated process of method was the same as previously discussed in Example 1A. pH data was collected via probes located in the proximal and distal regions of the stomach and from aspirates. PK data was also collected from blood plasma samples to determine the maximum concentration in the blood (Cmax) as well as the amount of time necessary to achieve maximum concentration (Tmax).

Figure 4 demonstrates the in-vivo PK, pH, and aspirate profile for omeprazole capsules containing magnesium stearate. Figure 5 demonstrates an in vivo profile of the PK, pH, and aspirate profiles for omeprazole capsules containing sodium stearyl fumarate. These clinical results indicate that the use of sodium stearyl fumarate in place of magnesium stearate improves the relative Tmax for the reformulated capsule.

### pH Profile from Clinical Trial Study

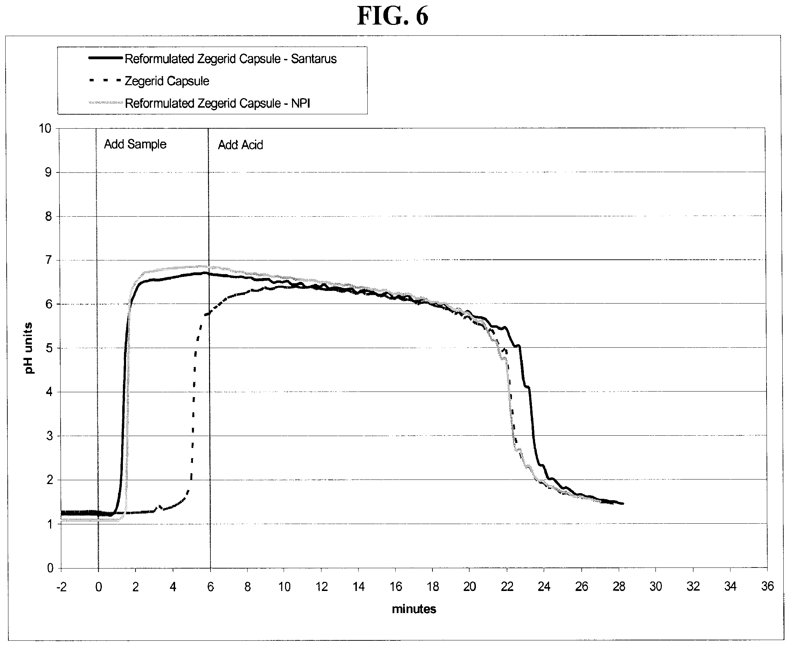

A 110 kg feasibility batch of 40 mg capsules of omeprazole with the proportionate quantity of ingredients listed in Table 1A3, including sodium stearyl fumarate, was prepared and encapsulated on a high speed encapsulator. A pH profile generated for samples from this trial is illustrated in Figure 6.

The pH profile generated in Fig. 6 matches the original feasibility profile depicted in Fig. 2. In both cases, the samples with sodium stearyl fumarate show a more rapid dissolution and sustained pH profile when compared to omeprazole capsule formulations containing magnesium stearate.

### Example lB: Capsule Formulations

The following specific formulations are described by way of reference only and are not intended to limit the scope described herein. Each formulation contains therapeutically effective doses of PPI as well as sufficient buffering agent to prevent acid degradation of at least some of the PPI by raising the pH of gastric fluid. Amounts of buffer (i.e. antacid) are expressed in weight as well as in molar equivalents (mEq). The capsules are prepared by blending the PPI with one or more antacids, and homogeneously blending with excipients, including sodium stearyl fumarate as the lubricant. The appropriate weight of bulk blend composition is filled into a hard gelatin capsule *(e.g.,* size 00) using an automatic encapsulator. The PPI can be in a micronized form.

**Table 1B1: 40 mg omeprazole formulation with 10.5 mEq sodium bicarbonate and sodium stearyl**

| **PPI·** | **Antacid** | **Excipient** |
|---|---|---|
| 40 mg omeprazole | 10.5 mEq or 880 mg NaHCO₃ | 30 mg HPC |
| | | 20 mg Crospovidone |
| | | 10 mg sodium stearyl fumarate |

**Table 1B2: 60 mg omeprazole formulation with 11.4 mEq sodium bicarbonate and sodium stearyl**

| **PPI·** | **Antacid** | **Excipient** |
|---|---|---|
| 60 mg omeprazole | 11.4 mEq or 960 mg NaHCO₃ | 20 mg MCC |
| | | 25 mg croscarmellose sodium |
| | | 10 mg sodium stearyl fumarate |

**Table 1B3: 40 mg lansoprazole formulation with 23.6 mEq total buffer and sodium stearyl fumarate**

| **PPI·** | **Antacid** | **Excipient** |
|---|---|---|
| 40 mg lansoprazole | 20.6 mEq or 600 mg Mg(OH)₂ | 20 mg MCC |
| | 3.0 mEq or 250 mg NaHCO₃ | 50 mg croscarmellose sodium |
| | | 10 mg sodium stearyl fumarate |
| | 23.6 mEq or 850 mgs total buffer | |

**Table 1B4: 40 mg esomeprazole formulation with 23.6 mEq total buffer and sodium stearyl fumarate**

| **PPI·** | **Antacid** | **Excipient** |
|---|---|---|
| 40 mg esomeprazole | 20.6 mEq or 600 mg Mg(OH)₂ | 100 mg MCC |
| | 3.0 mEq or 250 mg NaHCO₃ | 50 mg croscarmellose sodium |
| | | 10 mg sodium stearyl fumarate |
| | 23.6 mEq or 850 mgs total buffer | |

**Table 1B5: 40 mg tenatoprazole formulation with 23.6 mEq total buffer and sodium stearyl fumarate**

| **PPI·** | **Antacid** | **Excipient** |
|---|---|---|
| 40 mg tenatoprazole | 20.6 mEq or 600 mg Mg(OH)₂ | 30 mg MCC |
| | 3.0 mEq or 250 mg NaHCO₃ | 100 mg sodium starch glycolate (Primojel®) |
| | 23.6 mEq or 850 mgs total buffer | 10 mg sodium stearyl fumarate |

**Table 1B6: 40 mg tenatoprazole formulation with 23.6 mEq total buffer and sodium stearyl fumarate**

| **PPI·** | **Antacid** | **Excipient** |
|---|---|---|
| 40 mg omeprazole | 20.6 mEq or 600 mg Mg(OH)₂ | 50 mg HPC |
| | 3.0 mEq or 250 mg NaHCO₃ | 50 mg croscarmellose sodium |
| | | 10 mg sodium stearyl fumarate |
| | 23.6 mEq or 850 mgs total buffer | |

**Table 1B7: 40 mg tenatoprazole formulation with 23.6 mEq total buffer and sodium stearyl fumarate**

| **PPI·** | **Antacid** | **Excipient** |
|---|---|---|
| 40 mg tenatoprazole | 20.6 mEq or 600 mg Mg(OH)₂ | 30 mg HPC |
| | 3.0 mEq or 250 mg NaHCO₃ | 30 mg croscarmellose sodium |
| | | 10 mg sodium stearyl fumarate |
| | 23.6 mEq or 850 mgs total buffer | |

**Table 1B8: 20 mg pariprazole formulation with 23.6 mEq total buffer and sodium stearyl fumarate**

| **PPI·** | **Antacid** | **Excipient** |
|---|---|---|
| 20 mg pariprazole | 20.6 mEq or 600mg Mg(OH)₂ | 75 mg HPC |
| | 3.0 mEq or 250 mg NaHCO₃ | 30 mg croscarmellose sodium |
| | | 10 mg sodium stearyl fumarate |
| | 23.6 mEq or 950 mgs total buffer | |

**Table 1B9: 20 mg omeprazole formulation with 23.6 mEq total buffer and sodium stearyl fumarate**

| **PPI·** | **Antacid** | **Excipient** |
|---|---|---|
| 20 mg omeprazole | 20.6 mEq or 600 mg Mg(OH)₂ | 30 mg HPC |
| | 3.0 mEq or 250 mg NaHCO₃ | 70 mg Crospovidone |
| | | 10 mg sodium stearyl fumarate |
| | 23.6 mEq or 850 mgs total buffer | |

**Table 1B10: 20 mg pantoprazole formulation with 23. mEq total buffer and sodium stearyl fumarate**

| **PPI·** | **Antacid** | **Excipient** |
|---|---|---|
| 20 mg pantoprazole | 206 mEq or 600mg Mg(OH)₂ | 50 mg croscarmellose sodium |
| | 3.0 mEq or 250 mg NaHCO₃ | 30 mg HPC |
| | | 10 mg sodium stearyl fumarate |
| | 23.6 mEq or 850 mgs total buffer | |

**Table 1B11: 20 mg omeprazole formulation with 24.7 mEq total buffer and sodium stearyl fumarate**

| **PPI·** | **Antacid** | **Excipient** |
|---|---|---|
| 20 mg omeprazole | 20.6 mEq or 600 mg Mg(OH)₂ | 40 mg croscarmellose sodium |
| | 4.2 mEq or 250 mg NaHCO₃ | 35 mg HPC |
| | | 10 mg sodium stearyl fumarate |
| | 24.7 mEq or 950 mg total buffer | |

**Table 1B11: 30 mg esomeprazole formulation with 21.3 mEq total buffer and sodium stearyl fumarate**

| **PPI·** | **Antacid** | **Excipient** |
|---|---|---|
| 30 mg esomeprazole | 17.1 mEq or 500 mg Mg(OH)₂ | 40 mg croscarmellose sodium |
| | 4.2 mEq or 350mg NaHCO₃ | 30 mg HPC |
| | | 10 mg sodium stearyl fumarate |
| | 21.3 mEq or 850 mg total buffer | |

**Table 1B12: 30 mg esomeprazole formulation with 20.1 mEq total buffer and sodium stearyl fumarate**

| **PPI·** | **Antacid** | **Excipient** |
|---|---|---|
| 60 mg esomeprazole | 17.1 mEq or 500 mg Mg(OH)₂ | 30 mg Crospovidone |
| | 3.0 mEq or 250 mg NaHCO₃ | 15 mg croscarmellose sodium |
| | | 7 mg sodium stearyl fumarate |
| | 20.1 mEq or 750 mg total buffer | |

**Table 1B13: 10 mg pantoprazole formulation with 20.1 mEq total buffer and sodium stearyl fumarate**

| **PPI·** | **Antacid** | **Excipient** |
|---|---|---|
| 10 mg pantoprazole | 17.1 mEq or 500 mg Mg(OH)₂ | 30 mg sodium starch glycolate |
| | 3.0 mEq or 250 mg NaHCO₃ | (Explotab®) |
| | | 15 mg HPC |
| | 20.1 mE1 or 750 mg total buffer | 7 mg sodium stearyl fumarate |

**Table 1B14: 40 mg omeprazole formulation with 21.3 mEq total buffer and sodium stearyl fumarate**

| **PPI·** | **Antacid** | **Excipient** |
|---|---|---|
| 40 mg omeprazole | 17.1 mEq or 500 mg Mg(OH)₂ | 40 mg croscarmellose sodium |
| | 4.2 mEq or 350 mg NaHCO₃ | 45 mg HPC |
| | | 10 mg sodium stearyl fumarate |
| | 21.3 mEq or 850 mg total buffer | |

**Table 1B15: 15 mg lansoprazole formulation with 20.1 mEq total buffer and sodium stearyl fumarate**

| **PPI·** | **Antacid** | **Excipient** |
|---|---|---|
| 15 mg lansoprazole | 17.1 mEq or 500 mg Mg(OH)₂ | 30 mg Crospovidone |
| | 3.0 mEq or 250 mg NaHCO₃ | 15 mg HPC |
| | | 7 mg sodium stearyl fumarate |
| | 20.1 mEq or 750 mg total buffer | |

**Table 1B16: 20 mg omeprazole formulation with 20.1 mEq total buffer and sodium stearyl fumarate**

| **PPI·** | **Antacid** | **Excipient** |
|---|---|---|
| 20 mg omeprazole | 17.1 mEq or 500 mg Mg(OH)₂ | 50 mg croscarmellose sodium |
| | 3.0 mEq or 250 mg NaHCO₃ | 30 mg HPC |
| | | 10 mg sodium stearyl fumarate |
| | 20.1 mEq or 750 mg total buffer | |

**Table 1B17: 40 mg rabeprazole formulation with 24.7 mEq total buffer and sodium stearyl fumarate**

| **PPI·** | **Antacid** | **Excipient** |
|---|---|---|
| 40 mg rabeprazole | 20.6 mEq or 600 mg Mg(OH)₂ | 40 mg croscarmellose sodium |
| | 4.2 mEq or 350 mg NaHCO₃ | 35 mg HPC |
| | | 10 mg sodium stearyl fumarate |
| | 24.7 mEq or 950 mg total buffer | |

**Table 1B18: 60 mg pariprazole formulation with 20.1 mEq total buffer and sodium stearyl fumarate**

| **PPI·** | **Antacid** | **Excipient** |
|---|---|---|
| 60 mg pariprazole | 17.1 mEq or 500 mg Mg(OH)₂ | 30 mg croscarmellose sodium |
| | 3.0 mEq or 250 mg NaHCO₃ | 15 mg HPC |
| | | 7 mg sodium stearyl fumarate |
| | 20.1 mEq or 750 mg total buffer | |

**Table 1B19: 20 mg omeprazole formulation with 10.8 mEq total buffer and sodium stearyl fumarate**

| **PPI·** | **Antacid** | **Excipient** |
|---|---|---|
| 20 mg omeprazole | 6.9 mEq or 200 mg Mg(OH)₂ | 30 mg croscarmellose sodium |
| | 3.9 mEq or 330 mg NaHCO₃ | 35 mg HPC |
| | | 6 mg sodium stearyl fumarate |
| | 10.8 mEq or 530 mg total buffer | |

**Table 1B20: 30 mg pantoprazole formulation with 7.2 mEq total buffer and sodium stearyl fumarate**

| **PPI·** | **Antacid** | **Excipient** |
|---|---|---|
| 30 mg pantoprazole | 3.4 mEq or 100 mg Mg(OH)₂ | 20 mg croscarmellose sodium |
| | 3.8 mEq or 315 mg NaHCO₃ | 30 mg HPC |
| | | 5 mg sodium stearyl fumarate |
| | 7.2 mEq or 415 mg total buffer | |

**Table 1B21: 60 mg omeprazole formulation with 8.1 mEq total buffer and sodium stearyl fumarate**

| **PPI·** | **Antacid** | **Excipient** |
|---|---|---|
| 60 mg omeprazole | 5.1mEq or 150 mg Mg(OH)₂ | 20 mg croscarmellose sodium |
| | 3.0 mEq or 250 mg NaHCO₃ | 10 mg HPC |
| | | 4 mg sodium stearyl fumarate |
| | 8.1 mEq or 400 mg total buffer | |

**Table 1B22: 120 mg esomeprazole formulation with 11.0 mEq total buffer and sodium stearyl fumarate**

| **PPI·** | **Antacid** | **Excipient** |
|---|---|---|
| 120 mg esomeprazole | 8.6 mEq or 250 mg Mg(OH)₂ | 30 mg croscarmellose sodium |
| | 2.4 mEq or 200 mg NaHCO₃ | 30 mg HPC |
| | | 8 mg sodium stearyl fumarate |
| | 11.0 mEq or 450 mg total buffer | |

**Table 1A23: 10 mg rabeprazole formulation with 6.4 mEq total buffer and sodium stearyl fumarate**

| **PPI·** | **Antacid** | **Excipient** |
|---|---|---|
| 10 mg rabeprazole | 3.4 mEq or 100 mg Mg(OH)₂ | 18 mg croscarmellose sodium |
| | 3.0 mEq or 250 mg NaHCO₃ | 15 mg HPC |
| | | 7 mg sodium stearyl fumarate |
| | 6.4 mEq or 350 mg total buffer | |

**Table 1B24: 40 mg tenatoprazole formulation with 23.6 mEq total buffer and sodium stearyl fumarate**

| **PPI·** | **Antacid** | **Excipient** |
|---|---|---|
| 40 mg tenatoprazole | 20.6 mEq or 600 mg Mg(OH)₂ | 50 mg croscarmellose sodium |
| | 3.0 mEq or 250 mg Na∼HCO₃ | 10 mg sodium stearyl fumarate |
| | | |
| | 23.6 mEq or 850 mgs total buffer | |

**Table 1B25: 40 mg omeprazole formulation with 10.5 mEq total buffer and sodium stearyl fumarate**

| **PPI·** | **Antacid** | **Excipient** |
|---|---|---|
| 40 mg omeprazole | 10.5 mEq or 880 mg NaHCO₃ | 20 mg croscarmellose sodium |
| | | 9 mg sodium stearyl fumarate |
| | 10.5 mEq or 880 mg total buffer | |

### Example 1C: Capsule Formulations with Compressible Sodium Bicarbonate

The following specific formulations are described by way of reference only and are not intended to limit the scope described herein. Each formulation contains therapeutically effective doses of PPI as well as coated, compressible buffering agent (i.e. an antacid) to prevent acid degradation of at least some of the PPI by raising the pH of gastric fluid. Amounts of antacid are expressed in molar equivalents (mEq). The capsules are prepared by blending the PPI with one or more compressible buffering agents, and homogeneously blending with excipients, including one of two types of lubricants: sodium stearyl fumarate or magnesium stearate. The appropriate weight of bulk blend composition is filled into a hard gelatin capsule (e.g., size 00) using an automatic encapsulator. The PPI can be in a micronized form.

**Table 1C1: 40 mg omeprazole formulation with 10.5 mEq compressible antacid and sodium stearyl fumarate**

| **PPI·** | **Antacid** | **Excipient** |
|---|---|---|
| 40mg omeprazole | 10.5 mEq of compressible NaHCO₃ | 30 mg HPC |
| | 97% / 3% HPC | 25 mg Crospovidone |
| | | 10 mg sodium stearyl fumarate |

**Table 1C2: 60 mg omeprazole formulation with 10.5 mEq compressible antacid and magnesium stearate**

| **PPI·** | **Antacid** | **Excipient** |
|---|---|---|
| 60mg omeprazole | 10.5 mEq of compressible NaHCO₃ | 20 mg MCC |
| | 97% / HPC 3% | 25 mg croscarmellose sodium |
| | | 10 mg magnesium stearate |

**Table 1C3: 40 mg lansoprazole formulation with 23.6mEq compressible antacid and sodium stearyl**

| **PPI·** | **Antacid** | **Excipient** |
|---|---|---|
| 40 mg lansoprazole | 20.6 mEq of Mg(OH)₂ | 20 mg MCC |
| | 3.0 mEq of compressible NaHCO₃ | 50 mg croscarmellose sodium |
| | 95% / HPMC 5% | 10 mg sodium stearyl fumarate |
| | | |
| | 23.6 mEq or 850 mgs total buffer | |

**Table 1C4: 40mg esomeprazole formulation with 23.6 mEq compressible antacid and magnesium stearate**

| **PPI·** | **Antacid** | **Excipient** |
|---|---|---|
| 40 mg esomeprazole | 20.6 mEq of Mg(OH)₂ | 100mg MCC |
| | 3.0 mEq of compressible NaHCO₃ | 50 mg croscarmellose sodium |
| | 95% / Pregelatinized starch 5% | 10 mg magnesium stearate |
| | | |
| | 23.6 mEq total buffer | |

**Table 1C5: 40 mg tenatoprazole formulation with 23.6 mEq compressible antacid and sodium stearyl fumarate**

| **PPI·** | **Antacid** | **Excipient** |
|---|---|---|
| 40 mg tenatoprazole | 20.6 mEq of Mg(OH)₂ | 30mg MCC |
| | 3.0 mEq of compressible NaHCO₃ | 100 mg sodium starch glycolate (Primojel®) |
| | 97%/HPMC3% | |
| | | 10 mg sodium stearyl fumarate |
| | 23.6 mEq total buffer | |

**Table 1C6:40 mg omeprazole formulation with 23.6 mEq compressible antacid and sodium stearyl fumarate**

| **PPI·** | **Antacid** | **Excipient** |
|---|---|---|
| 40 mg omeprazole | 20.6 mEq of Mg(OH)₂ | 50mg HPC |
| | 3.0 mEq of compressible NaHCO₃ | 50 mg croscarmellose sodium |
| | 97%/HPC3% | 10 mg sodium stearyl fumarate |
| | | |
| | 23.6 mEq total buffer | |

**Table 1C7:40 mg tenatoprazole formulation with 23.6 mEq compressible antacid and sodium stearyl fumarate**

| **PPI·** | **Antacid** | **Excipient** |
|---|---|---|
| 40 mg tenatoprazole | 20.6 mEq of Mg(OH)₂ | 30mg HPC |
| | 3.0 mEq of compressible NaHCO₃ | 30 mg croscarmellose sodium |
| | 95%/HPC 5% | 10 mg sodium stearyl fumarate |
| | | |
| | 23.6 mEq total buffer | |

**1C8: 20 mg pariprazole formulation with 23.6mq compressible antacid and magnesium stearate**

| **PPI·** | **Antacid** | **Excipient** |
|---|---|---|
| 20 mg pariprazole | 20.6 mEq of Mg(OH)₂ | 75mg HPC |
| | 3.0 mEq of compressible NaHCO₃ | 30 mg croscarmellose sodium |
| | 95% / HPC | 10 mg sodium stearyl fumarate |
| | | |
| | 23.6 mEq total buffer | |

**Table 1C9:20 mg omeprazole formulation with 23.6 mEq compressible antacid and sodium stearyl**

| **PPI·** | **Antacid** | **Excipient** |
|---|---|---|
| 20 mg omeprazole | 20.6 mEq of Mg(OH)₂ | 30mg HPC |
| | 3.0 mEq of compressible NaHCO₃ | 70 mg Crospovidone |
| | 95% / Pregelatinized Starch 5% | 10 mg sodium stearyl fumarate |
| | | |
| | 23.6 mEq total buffer | |

**Table 1C10:20 mg pantoprazole formulation with 23.6 mEq compressible antacid and sodium stearyl fumarate**

| **PPI·** | **Antacid** | **Excipient** |
|---|---|---|
| 20 mg pantoprazole | 20.6 mEq of Mg(OH)₂ | 50 mg croscarmellose sodium |
| | 3.0 mEq of compressible NaHCO₃ | 30mg HPC |
| | 95%/HPC 5% | 10 mg sodium stearyl fumarate |
| | | |
| | 23.6 mEq total buffer | |

**Table 1C11: 20 mg pantoprazole formulation with 23.6 mEq compressible antacid and magnesium stearate**

| **PPI·** | **Antacid** | **Excipient** |
|---|---|---|
| 20 mg pantoprazole | 20.6 mEq of Mg(OH)₂ | 50 mg croscarmellose sodium |
| | 3.0 mEq of compressible NaHCO₃ | 30mg HPC |
| | 97%/HPMC 3% | 10 mg magnesium stearate |
| | | |
| | 23.6 mEq total buffer | |

**Table 1C12: 20 mg omeprazole formulation with 24.7 mEq compressible antacid and magnesium stearate**

| **PPI·** | **Antacid** | **Excipient** |
|---|---|---|
| 20 mg omeprazole | 20.6 mEq of Mg(OH)₂ | 40 mg croscarmellose sodium |
| | 4.2 mEq of compressible NaHCO₃ | 35mg HPC |
| | 97%/HPC 3% | 10 mg magnesium stearate |
| | | |
| | 24.7 mEq total buffer | |

**Table 1C13:30 mg esomeprazole formulation with 21.3 mEq compressible antacid and magnesium stearate**

| **PPI·** | **Antacid** | **Excipient** |
|---|---|---|
| 30 mg esomeprazole | 17.1 mEq of Mg(OH)₂ | 40 mg croscarmellose sodium |
| | 4.2 mEq of compressible NaHCO₃ | 30mg HPC |
| | 97%/HPMC 3% | 10 mg magnesium stearate |
| | | |
| | 21.3 mEq total buffer | |

**Table 1C14: 60 mg omeprazole formulation with 20.1 mEq compressible antacid and sodium stearyl fumarate**

| **PPI·** | **Antacid** | **Excipient** |
|---|---|---|
| 60 mg omeprazole | 17.1 mEq of Mg(OH)₂ | 30 mg Crospovidone |
| | 3.0 mEq of compressible NaHCO₃ | 15 mg HPC |
| | 95%/HPC 5% | 7 mg sodium stearyl fumarate |
| | | |
| | 20.1 mEq total buffer | |

**Table 1C15:10 mg pantoprazole formulation with 20.1 mEq compressible antacid and sodium stearyl fumarate**

| **PPI·** | **Antacid** | **Excipient** |
|---|---|---|
| 10 mg pantoprazole | 17.1 mEq of Mg(OH)₂ | 30 mg sodium starch glycolate |
| | 3.0 mEq of compressible NaHCO₃ | (Explotab®) |
| | 97% /Pregelatinized Starch 3% | 15 mg HPC |
| | | 7 mg sodium stearyl fumarate |
| | 20.1 mEq total buffer | |

**Table 1C16: 10 mg pantoprazole formulation with 20.1 mEq compressible antacid and magnesium**

| **PPI·** | **Antacid** | **Excipient** |
|---|---|---|
| 10 mg pantoprazole | 17.1 mEq of Mg(OH)₂ | 30 mg sodium starch glycolate |
| | 3.0 mEq of compressible NaHCO₃ | (Explotab®) |
| | 95%/HPC 5% | 15 mg HPC |
| | | 7 mg magnesium stearate |
| | 20.1 mEq total buffer | |

**Table 1C17: 40 mg omeprazole formulation with 21.3 mEq compressible antacid and magnesium stearate**

| **PPI·** | **Antacid** | **Excipient** |
|---|---|---|
| 40 mg omeprazole | 17.1 mEq of Mg(OH)₂ | 40 mg croscarmellose sodium |
| | 4.2 mEq of compressible NaHCO₃ | 45 mg HPC |
| | 95%/HPMC 5% | 10 mg magnesium stearate |
| | | |
| | 21.3 mEq total buffer | |

**Table 1C18: 15 mg lansoprazole formulation with 20.1 mEq compressible antacid and sodium stearyl fumarate**

| **PPI·** | **Antacid** | **Excipient** |
|---|---|---|
| 15 mg lansoprazole | 17.1 mEq of Mg(OH)₂ | 30 mg Crospovidone |
| | 3.0 mEq of compressible NaHCO₃ | 15 mg HPC |
| | 95%/HPC 5% | 7 mg sodium stearyl fumarate |
| | | |
| | 20.1 mEq total buffer | |

**Table 1C19:20 mg omeprazole formulation with 20.1 mEq compressible antacid and magnesium stearate**

| **PPI·** | **Antacid** | **Excipient** |
|---|---|---|
| 20 mg omeprazole | 17.1 mEq of Mg(OH)₂ | 50 mg croscarmellose sodium |
| | 3.0 mEq of compressible NaHCO₃ | 30mg HPC |
| | 95%/HPMC 5% | 10 mg magnesium stearate |
| | | |
| | 20.1 mEq total buffer | |

**Table 1C20: 30 mg rabeprazole formulation with 24.7 mEq compressible antacid and sodium stearyl fumarate**

| **PPI·** | **Antacid** | **Excipient** |
|---|---|---|
| 40 mg rabeprazole | 20.6 mEq of Mg(OH)₂ | 40 mg croscarmellose sodium |
| | 4.2 mEq of compressible NaHCO₃ | 35 mg HPC |
| | 95%/HPC 5% | 10 mg sodium stearyl fumarate |
| | | |
| | 24.7 mEq total buffer | |

**Table 1C21: 60 mg pariprazole formulation with 20.1 mEq compressible antacid and magnesium stearate**

| **PPI·** | **Antacid** | **Excipie** |
|---|---|---|
| 60 mg pariprazole | 17.1 mEq of Mg(OH)₂ | 30 mg croscarmellose sodium |
| | 3.0 mEq of compressible NaHCO₃ | 15mg HPC |
| | 97% /Pregelatinized Starch 3% | 7 mg magnesium stearate |
| | | |
| | 20.1 mEq total buffer | |

**Table 1C22: 20 mg omeprazole formulation with 10.8 mEq compressible antacid and magnesium stearate**

| **PPI·** | **Antacid** | **Excipient** |
|---|---|---|
| 20 mg omeprazole | 6.9 mEq of Mg(OH)₃ | 30 mg croscarmellose sodium |
| | 3.9 mEq of compressible NaHCO₃ | 35 mg HPC |
| | 95% / Pregelatinized Starch 5% | 6 mg magnesium stearate |
| | | |
| | 10.8 mEq total buffer | |

**Table 1C23: 30 mg pantoprazole formulation with 7.2 mEq compr3essible antacid and sodium stearyl fumarate**

| **PPI** | **Antacid** | **Excipient** |
|---|---|---|
| 30 mg pantoprazole | 3.4 mEq of Mg(OH)₂ | 20 mg croscarmellose sodium |
| | 3.8 mEq of compressible NaHCO₃ | 30mg HPC |
| | 97%/HPMC3% | 5 mg sodium stearyl fumarate |
| | | |
| | 7.2 mEq total buffer | |

**Table 1C24: 60 mg omeprazole formulation with 8.1 mEq compressible antacid and sodium stearyl fumarate**

| **PPI** | **Antacid** | **Excipient** |
|---|---|---|
| 60 mg omeprazole | 5.1mEq of Mg(OH)₂ | 20 mg croscarmellose sodium |
| | 3.0 mEq of compressible NaHCO₃ | 10 mg HPC |
| | 95%/HPC 5% | 4 mg sodium stearyl fumarate |
| | | |
| | 8.1 mEq total buffer | |

**Table 1C25: 120 mg esomeprazole formulation with 11.0 mEq compressible antacid and sodium stearyl fumarate**

| **PPI** | **Antacid** | **Excipient** |
|---|---|---|
| 120 mg esomeprazole | 8.6 mEq of Mg(OH)₂ | 30 mg croscarmellose sodium |
| | 2.4 mEq of compressible NaHCO₃ | 30mg HPC |
| | 95%/HPMC 5% | 8 mg sodium stearyl fumarate |
| | | |
| | 11.0 mEq total buffer | |

**Table 1C26:120 mg esomeprazole formulation with 11.0 mEq compressible antacid and magnesium stearate**

| **PPI** | **Antacid** | **Excipient** |
|---|---|---|
| 120 mg esomeprazole | 8.6 mEq of Mg(OH)₂ | 30 mg croscarmellose sodium |
| | 2.4 mEq of compressible NaHCO₃ | 30mg HPC |
| | 97% /Pregelatinized Starch 3% | 8 mg magnesium stearate |
| | | |
| | 11.0 mEq total buffer | |

**Table 1C27:10 mg rabeprazole formulation with 6.4 mEq compressible antacid and sodium stearyl fumarate**

| **PPI** | **Antacid** | **Excipien** |
|---|---|---|
| 10 mg rabeprazole | 3.4 mEq of Mg(OH)₂ | 18 mg croscarmellose sodium |
| | 3.0 mEq of compressible NaHCO₃ | 15 mg HPC |
| | 95%/HPC 5% | 7 mg sodium stearyl fumarate |
| | 6.4 mEq total buffer | |

**Table 1C28: 60 mg tenatoprazole formulation with 23.6 mEq compressible antacid and sodium stearyl fumarate**

| **PPI** | **Antacid** | **Excipient** |
|---|---|---|
| 40 mg tenatoprazole | 20.6 mEq of Mg(OH)₂ | 50 mg croscarmellose sodium |
| | 3.0 mEq of compressible NaHCO₃ | 10 mg sodium stearyl fumarate |
| | 95%/HPMC 5% | |
| | | |
| | 23.6 mEq total buffer | |

**Table 1C29: 40 mg tenatoprazole formulation with 23.6 mEq compressible antacid and magnesium stearate**

| **PPI** | **Antacid** | **Excipient** |
|---|---|---|
| 40 mg tenatoprazole | 20.6 mEq of Mg(OH)₂ | 50 mg croscarmellose sodium |
| | 3.0 mEq of compressible NaHCO₃ | 10 mg magnesium stearate |
| | 97%/HPMC3% | |
| | | |
| | 23.6 mEq total buffer | |

**Table 1C30: 40 mg omeprazole formulation with 10.5 mEg compressible antacid and magnesium stearate**

| **PPI** | **Antacid** | **Excipient** |
|---|---|---|
| 40 mg omeprazole | 10.5 mEq of compressible NaHCO₃ | 20 mg croscarmellose sodium |
| | 95%/HPC 5% | 9 mg magnesium stearate |
| | | |
| | 10.5 mEq total buffer | |

### Example 2A: Caplet Formulations

The following specific formulations are described by way of illustrating the present invention and are not intended to be limiting. The caplets were prepared by blending the indicated amount of micronized omeprazole and about half the indicated amount of sodium bicarbonate. After blending the omeprazole and sodium bicarbonate, the remaining sodium bicarbonate was added along with the indicated amount of magnesium hydroxide, hydroxypropyl cellulose, croscarmellose sodium, and magnesium stearate from Table 2A1 and Table 2A2.

### Preparation of Omeprazole Caplet Formulations in Three Antacid Forms

**Table 2A1: 20 mg omeprazole caplet formulation with sodium bicarbonate and magnesium stearate**

| **Material** | **Caplet Formulation** | |
|---|---|---|
| | **Mg/tab** | **%** |
| Omeprazole | 20 | 1.6 |
| Sodium Bicarbonate #2 | 1100 | 88.2 |
| Hydroxypropylcellulose EXF | 76 | 6.1 |
| Croscarmellose Sodium | 38.2 | 3.1 |
| Magnesium Stearate | 12.0 | 1.0 |
| **Totals:** | **1247** | **100** |
| **Theoretical ANC (mEq):** | **13.1** | |

Magnesium hydroxide in combination with sodium bicarbonate was selected as an alternative to the use of pure sodium bicarbonate as the antacid according to Table 2A2.

**Table 2A2: 40 mg omeprazole caplet formulation with 95% magnesium hydroxide, sodium bicarbonate and magnesium stearate**

| | **CTM Formulation** | |
|---|---|---|
| **Material** | **mg/tab** | **%** |
| Omeprazole | 40 | 3.2 |
| Sodium Bicarbonate #2 | 750 | 58.9 |
| Magnesium Hydroxide, 95% | 368.5 | 28.9 |
| Hydroxypropylcellulose EXF | 69 | 5.4 |
| Croscarmellose Sodium | 38 | 3.0 |
| Magnesium Stearate | 7.5 | 0.6 |
| **Totals:** | **1273** | **100** |
| **Theoretical ANC** | **20.9** | |

Three caplet formulations were prepared, one without any lubricant (denoted as the control trial common blend), one containing a magnesium stearate lubricant, and the other containing sodium stearyl fumarate. The ingredients of the formulation without a lubricant are listed in Table 2A3. This common unlubricated blend was then divided into two formulations, with two different lubricants listed in Table 2A4 and Table 2A5.

**Table 2A3: Control Trial Common Blend Formulation**

| **Material** | **Caplet Formulation** | |
|---|---|---|
| | **mg/tab** | **%** |
| Omeprazole | 20.4 | 1.7 |
| Sodium Bicarbonate #2 | 1100 | 89.1 |
| Hydroxypropylcellulose EXF | 76 | 6.2 |
| Croscarmellose Sodium | 38.2 | 3.1 |
| **Totals:** | **1234.6** | **100** |
| **Theoretical ANC (mEq):** | **13.1** | |

**Table 2A4: Lubricated Control Blends**

| **Material** | **Caplet Formulation** | | | |
|---|---|---|---|---|
| | **Magnesium Stearate Sodium Stearyl Fumarate** | | | |
| | mg;/tab | % | mg/tab | % |
| Unlubricated Common Blend | 1234.6 | 99.4 | 1234.6 | 98.5 |
| Magnesium Stearate | 8 | 0.6 | -- | -- |
| Sodium Stearyl Fumarate | -- | -- | 19 | 1.5 |
| **Totals:** | **1242.6** | **100** | **1253.6** | **100** |
| **Theoretical ANC (mEq):** | **13.1** | | | |

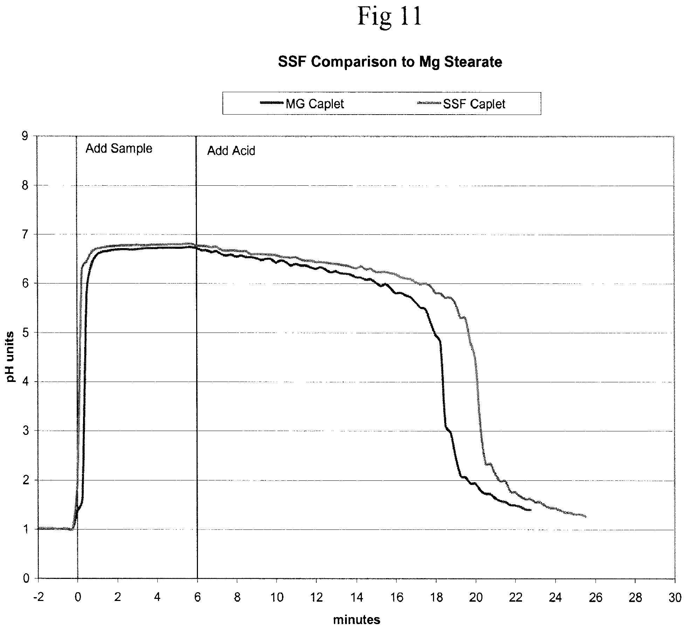

Each control blend was then compressed using a rotary station tablet press. Figure 11 illustrates a comparison of the pH profiles between capsules with the two different lubricants in capslets formulated pursuant to Table 2A4 and Table 2A5.

Table 2A5 provides a summary of the compression characteristics of the two lubricated control blends.

**Table 2 A5: Summary of Caplet Compression Characteristics with sodium stearyl fumarate and magnesium stearate**

| **Parameter/Characteristic** | **Lubricated Formulations** | |
|---|---|---|
| | **0.6%w/w Magnesium** | **1.5% w/w Sodium Stearyl** |
| Average Compression | 9000lbs | 9000 lbs |
| Average Observed Ejection Force | 65 lbs | 55 lbs |
| Ave. Tab Weight (mg) | 1242.1 | 1251.2 |
| Average Hardness (kP) | 13.1 | 12.7 |
| Average Thickness (mm) | 6.13 | 6.16 |
| Friability(%) | 0.20 | 0.22 |
| Disintegration (first tablet/last tablet) | 25 s/35 s | 23 s/25 s |

### Preparation of Omeprazole Caplet Formulations with HPC Coated Sodium Bicarbonate

Four formulations with sodium bicarbonate and sodium stearyl fumarate were prepared as listed in Tables 2A9 and 2A10. In all of these formulations, the sodium bicarbonate was coated with a 10% w/w HPC in a fluidized bed, but with differing concentrations of HPC relative to sodium bicarbonate. Two formulations utilized sodium bicarbonate coated with 5% Hydroxypropylcellulose (HPC) and 95% sodium bicarbonate. The other two formulations utilized sodium bicarbonate coated with 3% HPC. These formulations were prepared by blending the compressible sodium bicarbonate with the omeprazole for 5 minutes, adding the hydroxypropylcellulose (if applicable) and croscarmellose sodium and mixing for 20 minutes. To that mixture sodium stearyl fumarate was added and that mixture was mixed for 10 minutes. The blended mixture was then compressed into caplets.

The coating process entailed loading 4,750 g of sodium bicarbonate into a fluid bed dryer. Prior to spraying, the sodium bicarbonate was heated to 45°C. The level of fluidization was adjusted to provide sufficient movement of sodium bicarbonate into the bowl granulator, while keeping the bulk of the material within the spray zone. Following the spray application of HPC, the coated material was allowed to dry by letting the product temperature rise to 50°C.

Table 2A6 depicts the operating parameters for the feasibility test of the 95% sodium bicarbonate, 5% hydroxypropylcellulose (HPC) formulation. Table 2A7 depicts the target operating parameters utilized for the coating with 97% sodium bicarbonate and 3% HPC.

**Table 2A6: Process Operating Parameters for the Preparation of 5% HPC and 95% Sodium Bicarbonate**

| Inlet Temp (°C) | Product Temperature (°C) | Air Volume (PSI) | Atomization Pressure (PSI) | Spray Rate (g/rnin) |
|---|---|---|---|---|
| **75** | **45** | **25-30** | **20** | **18** |

**Table 2A7: Process Operating Parameters for the Preparation of 3% HPC and 97% Sodium Bicarbonate**

| Inlet Temp (°C) | Product Temperature (°C) | Air Volume (PSI) | Atomization Pressure (PSI) | Spray Rate (g/rnin) |
|---|---|---|---|---|
| **75** | **45** | **25** | **20** | **18** |

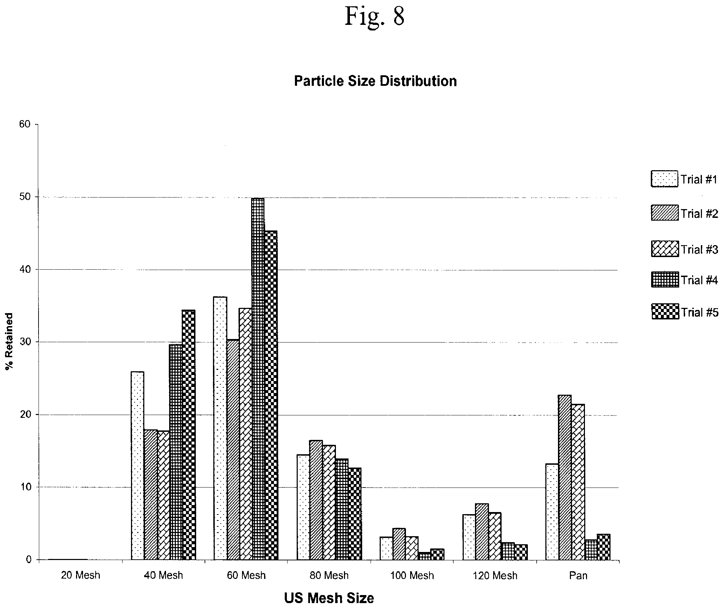

With both the 5% HPC coating (95% sodium bicarbonate) and the 3 % HPC coating (97% sodium bicarbonate), the bulk and tapped density of the coated sodium bicarbonate decreases as the amount of HPC coating applied increases. Although the bulk density of sodium bicarbonate coated with 5% HPC (the 95% sodium bicarbonate) is less than that coated with 3% HPC (the 97% sodium bicarbonate), the respective Carr Index values for each as listed in Table 2A8 indicate that the flow of both is excellent. In addition, as Figure 8 illustrates, the particle size distribution shows that the particle size of the coated sodium bicarbonate increases significantly as the amount of coating solution applied increases.

### Density, Flow Rate, Particle Size Distribution. Compressibility, Hardness and Other Physical Characteristics of Omeprazole Caplets Coated With Sodium Bicarbonate

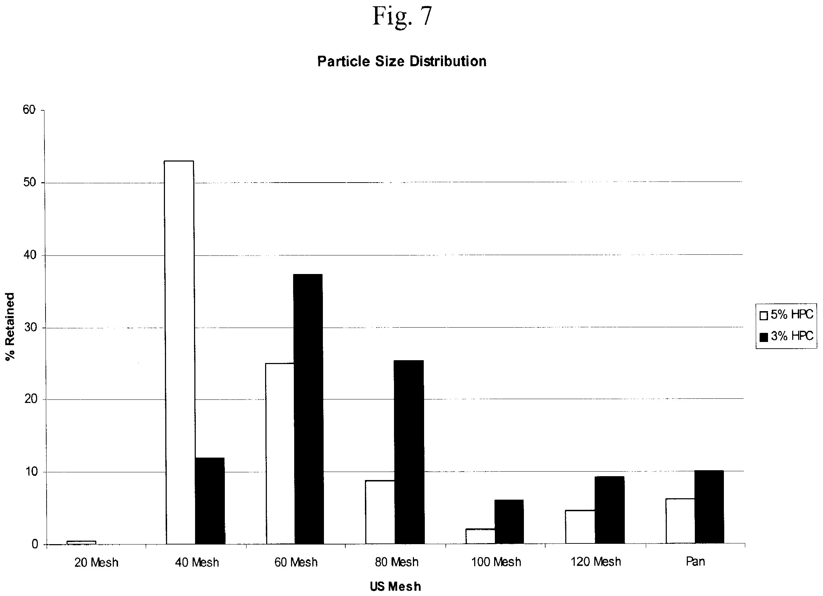

Coated sodium bicarbonate was prepared in the same manner described above in Example 2A6. A sample of this formulation was then taken for physical testing in regards to bulk density, loss of drying (L.O.D.) and particle size distribution. A summary of the results is described in Table 2A8. Figure 7 illustrates the comparative particle size distribution of sodium bicarbonate coated with 5% HPC and 3% HPC drawn from a 10% w/w HPC solution.

**Table 2A8: Physical Test Result Summary for 5% HPC and 3% HPC Solutions**

| **Test** | **5% HPC** | **3% HPC** |
|---|---|---|
| Bulk Density (g/ml) | 0.58 | 0.75 |
| Tapped Density (g/ml) | 0.64 | 0.85 |
| Carr's Index (TD-BD)/TD x 100 | 9.3 | 11.9 |
| L.O.D. (%) | 0.64 | 0.72 |

| Particle Size Distribution (%Retained) | | |
|---|---|---|
| #20US Mesh | 0.4 | 0.0 |
| #40US Mesh | 52.93 | 11.9 |
| #60 US Mesh | 25.01 | 37.32 |
| #80 US mesh | 8.8 | 25.33 |
| #100 US Mesh | 2.1 | 6.12 |
| #120 US Mesh | 4.54 | 9.19 |
| Pan | 6.27 | 10.01 |

To evaluate the compressibility of the HPC coated sodium bicarbonate, four separate caplet formulations were prepared, two of which were coated with 5% HPC sodium bicarbonate, and two of which were coated with 3% sodium bicarbonate. The exact composition of these trial caplet formulations are described in Table 2A9 and Table 2A10. All four formulations were prepared by blending the compressible sodium bicarbonate with the omeprazole for 5 minutes, adding the hydroxypropylcellulose and croscarmellose sodium and mixing for 20 minutes. To that mixture sodium stearyl fumarate was added and mixed for 10 minutes. The blended mixture was then compressed into caplets.

**Table 2A9: Caplet trial formulations using Sodium Bicarbonate coated with 5% HPC**

| **Ingredients** | **Formulation #1** | | **Formulation #2** | |
|---|---|---|---|---|
| | **mg/tab** | **%** | **Mg/ta** | **%** |
| Omeprazole | 20.4 | 1. | 2 | 1.6 |
| Sodium Bicarbonate 95% /HPC 5% | 1158 | 93.9 | 1158 | 92.5 |
| Klucel EXF (HPC) | --- | --- | 1 | 1.4 |
| Croscarmellose Sodium | 37 | 3. | 3 | 3.0 |
| Sodium Stearyl Fumarate | 18 | 1. | 1 | 1.5 |
| **Totals:** | **1233.4** | **10** | **1252.4** | **100** |

**Table 2A10: Caplet trial formulations using Sodium Bicarbonate coated with 3% HPC**

| **Ingredients** | **Formulation #3** | | **Formulation #4** | |
|---|---|---|---|---|
| | **mg/tab** | **%** | **mg/tab** | **%** |
| Omeprazole | 20.4 | 1.7 | 20.4 | 1.6 |
| Sodium Bicarbonate 97% /HPC 3% | 1134 | 93.8 | 1134 | 90.6 |
| Klucel EXF (HPC) | --- | --- | 41 | 3.3 |
| Croscarmellose Sodium | 37 | 3.1 | 37 | 3.0 |
| Sodium Stearyl Fumarate | 18 | 1.5 | 19 | 1.5 |
| **Totals:** | **1209.4** | **100** | **1251.4** | **100** |

Whether coated with 3% HPC or 5% HPC, the coated sodium bicarbonate formulations demonstrate a significant improvement in compressibility and friability compared to essentially the same formulations using uncoated sodium bicarbonate as determined by binder level required, compression forces required, and resulting tablet hardness.

Specifically, the compressibility of formulations with 5% HPC coating is greater than those formulations coated with 3% HPC. Summaries of the data illustrating the greater relative effectiveness of the 5% HPC over the 3% HPC are depicted in their respective parameter tables, Table 2A11 and Table 2A12. In both situations, however, the friability of the caplet formulations is very good and tablet chipping is minimal. Further analysis of the data in these tables indicates that the compression force exerted on both the 5% and 3% formulations was well below the maximum that can be applied to the compression tooling, and thus it is possible to increase the caplet hardness further.

**Table 2A11: Summary of Formulations #1 and #2 Caplet Compression Characteristics (5% HPC Coating)**

| | **5% HPC Formulations** | |
|---|---|---|
| **Parameter/Characteristic** | **Formulation #1** | **Formulation #2** |
| Average Compression Force | 4700 lbs | 4500 lbs |
| Average Observed Ejection Force | 40 lbs | 35 lbs |
| Average Weight (mg) | 1230.3 | 1232.4 |
| Average Hardness (kp) | 16.6 | 15.8 |
| Average Thickness (mm) | 6.23 | 6.31 |
| Friability (%) | 0.12 | 0.13 |
| Disintegration Time in Water (first caplet/last caplet) | 40 s/60 s | 50 s/62 s |

**Table 2A12: Summary of Formulations #3 and #4 Caplet Compression Characteristics (3% HPC Coating)**

| **Parameter/Characteristic** | **3% HPC Formulations** | |
|---|---|---|
| | **Formulation #3** | **Formulation #4** |
| Average Compression Force | 7000 lbs | 5500 lbs |
| Average Observed Ejection Force | 50 lbs | 35 lbs |
| Average Weight (mg) | 1206.3 | 1248.3 |
| Average Hardness (kp) | 16.5 | 17.0 |
| Average Thickness (rnm) | 5.95 | 6.24 |
| Friabilit | 0.15 | 0.10 |
| Disintegration Time in Water | 35 s/43 s | 50s/52 s |

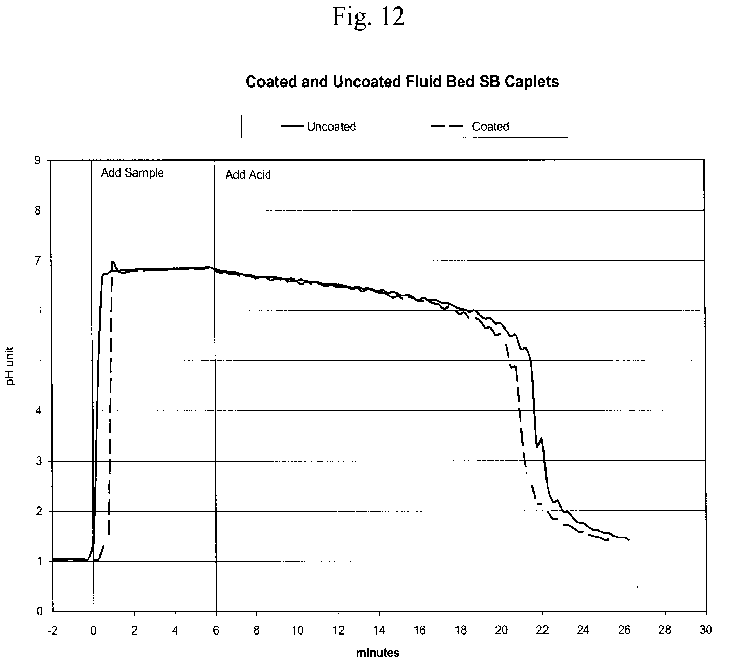

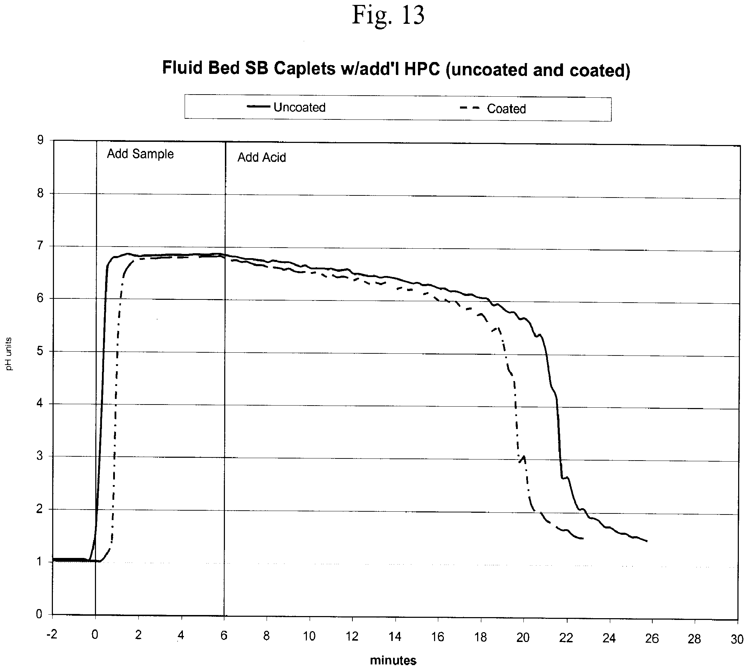

Figure 12 Illustrates a comparison in the pH profiles of film coated caplets with Opadry II 57U18539 or non-film coated caplets prepared using compressible sodium bicarbonate pursuant to the formulation #3 depicted in Table 2A10, and Figure 13 illustrates this comparison but pursuant to formulation #4 depicted in Table 2A10.

Five additional trials of formulations of sodium bicarbonate coated with 3% HPC drawn from a 7.5% w/w HPC solution were prepared in a fluid bed drying process with a target inlet temperature of about 85°C, a target product temperature of about 40°C to 45°C, air volume target of about 25PSI to 30 PSI and an atomization pressure of about 30 PSI,. The blended mixture was then compressed into caplets. The 7.5% w/w HPC solution represents a reduction in solids by 25% over that used in earlier feasibility trials with HPC coating drawn from a 10% w/w solution. The physical test result summary for each of the five trials is depicted in Table 2A13.

**Table 2A13: Physical Test Result Summary for Trials 1-5**

| **Test** | **Trial 1** | **Trial 2** | **Trial 3** | **Trial 4** | **Trial 5** |
|---|---|---|---|---|---|
| Bulk Density (g/ml) | 0.70 | 0.76 | 0.68 | 0.58 | 0.68 |
| Tapped Density (g/ml) | 0.78 | 0.82 | 0.76 | 0.64 | 0.73 |
| Carr's Index* (TD-BD)/TD X | 10.3 | 7.32 | 10.5 | 9.4 | 6.9 |
| L.O.D. (%) | 0.38 | 0.56 | 0.53 | 0.62 | 0.30 |

| | Particle Size Distribution(% Retained) | | | | |
|---|---|---|---|---|---|
| #20USMesh | 0.04 | 0.06 | 0.05 | 0.01 | 0.01 |
| #40USMesh | 25.88 | 17.94 | 17.76 | 29.62 | 34.38 |
| #60 US mesh | 36.25 | 30.31 | 34.66 | 49.83 | 45.33 |
| #80US Mesh | 14.49 | 16.46 | 15.80 | 13.90 | 12.64 |
| #100 US Mesh | 3.16 | 4.36 | 3.23 | 1.02 | 1.53#120 US Mesh#120 US Mesh |
| #120 US Mesh | | | | | |
| Pan | 13.26 | 22.70 | 21.41 | 2.81 | 3.60 |

Figure 8 demonstrates the comparative particle size distribution for five fluid bed trials with 3% HPC coated sodium bicarbonate, prepared from a 7.5 wt-% HPC solution.

### Direct Blending Omeprazole Capsule Formulations

Two direct blending trials were prepared to investigate the feasibility of using direct compression excipients and regular sodium bicarbonate, rather than HPC coated sodium bicarbonate. The two excipients used during the trial were pregelatinized starch and Xylitab®. These direct compression formulations were prepared by blending the compressible sodium bicarbonate with the omeprazole for 5 minutes, adding the hydroxypropylcellulose and croscarmellose sodium and mixing for 20 minutes. The excipients were incorporated into the mixture at the same time the HPC was added. Sodium stearyl fumarate was then added and mixed for 10 minutes. The blended mixture was then compressed into caplets. The test formulations for these two trials are described in Table 2A14. A summary of the caplet compression characteristics with each excipient is described for in Table 2A15.

**Table 2A14: Trial formulation with alternate direct compression excipients**

| **Ingredients** | **Pregelatinized Starch** | | **Xylitab®** | |
|---|---|---|---|---|
| | **mg/tab** | **%** | **mg/tab** | **%** |
| Omeprazole | 40.8 | 2.7 | 20.4 | 1.4 |
| Sodium Bicarbonate | 1100 | 72.3 | 1100 | 73.3 |
| Klucel EXF (HPC) | 90 | 5.9 | 90 | 6.0 |
| Pregelatinized Starch | 222.6 | 14.6 | -- | -- |
| Xylitab 100 | -- | -- | 222.6 | 14.8 |
| Croscarmellose Sodium | 45 | 3.0 | 45 | 3.0 |
| Sodium Stearyl Fumarate | 22 | 1.4 | 22 | 1.5 |
| **Totals:** | **1520.4** | **100** | **1500.0** | **100** |

**Table 2A15: Summary of the caplet compression characteristics using excipients**

| **Parameter/Characteristic** | **Caplet Trial Lot Number** | | | | | |
|---|---|---|---|---|---|---|
| | **Pregelatinized Starch** | | | **Xylitab** | | |
| Average Weight (mg) | * | | | 1500.9 | | |
| Max Tab Weight (mg) | * | | | 1526.0 | | |
| Min Tab Weight (mg) | * | | | 1483.0 | | |
| %RSD | * | | | 0.99 | | |

| Average Compression Force | 5000lb | 7000 lb | 9000 lb | 5000 lb | 7000 lb | 9000lb |
|---|---|---|---|---|---|---|
| Average Hardness (kp) | 11.2 | 16.7 | 20.3 | 15.4 | 21.1 | 23.8 |
| Max Hardness (kp) | 12.0 | 17.3 | 21.3 | 16.1 | 22.9 | 25.4 |
| Min Hardness (kp) | 10.5 | 15.5 | 19.4 | 14.3 | 18.6 | 19.5 |
| %RSD | 5.17 | 3.47 | 3.49 | 3.99 | 5.83 | 7.00 |
| Friability (%) | Fails | Fails | Fails | 0.3 Significant edge chipping observed | | |
| Average Thickness (mm) | 7.82 | | | 7.51 | | |
| Max Thickness (mm) | 7.87 | | | 7.58 | | |
| Min Thickness (mml | 7.77 | | | 7.47 | | |
| %RSD | 0.39 | | | 0.46 | | |
| Disintegration Time in Water (first caplet/last caplet) | Not tested due to poor compressibility results | | | 6 min 43 s/10 mins 5 s | | |

| | | | | | | |
|---|---|---|---|---|---|---|
| *Individual weights not measured | | | | | | |

Of the two excipients, Xylitab® produced a relatively better caplet compression compared to pregelatinized starch. However, both formulations had poor friability results. The use of coated sodium bicarbonate without these two excipients results in a more compressible, robust caplet formulation.

### Specific Caplet Formulations

**Table 2A16**

| **Component** | **mg/tab** | **Conc.** | **mEq** |
|---|---|---|---|
| Omeprazole | 40.8 | 3.2 | |
| Sodium bicarbonate 97%/HPC 3% | 1134 | 89.0 | 13.1 |
| Hydroxypropyl cellulose-EXF | 42 | 3.3 | |
| Croscarmellose sodium | 38 | 3.0 | |
| Sodium stearyl fumarate | 19 | 1.5 | |

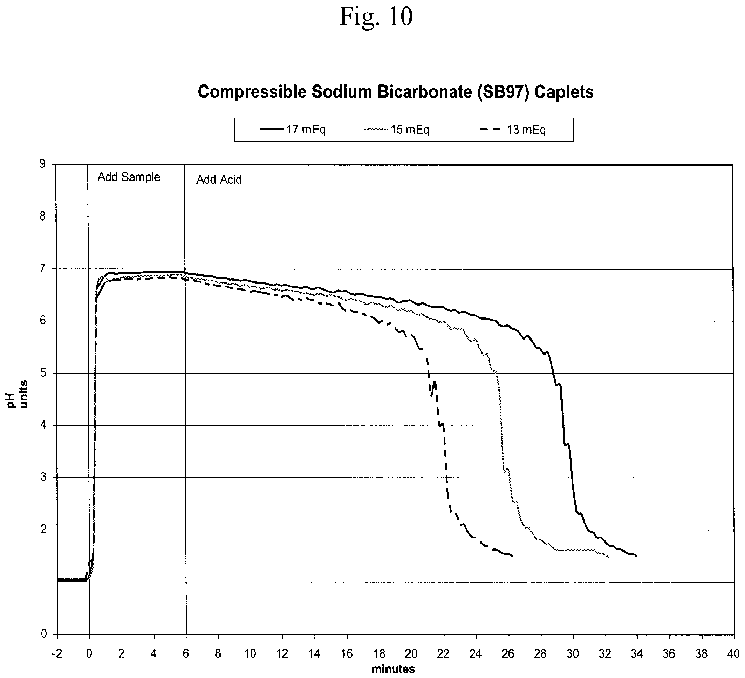

The formulation was prepared by blending the indicated amount of omeprazole with sodium bicarbonate, homogeneously blending with excipients, followed by a final blend after the addition of the lubricant. The blend was then compressed into caplets. The caplet was film coated with Colorcon film coating formula # 57U18539 at 3% weight gain. Sodium stearyl fumarate was utilized as the lubricant. Caplets without coating were also prepared. The compression force used was 4300 lb_{f}, the friability 0.1%, the hardness of the caplet was determined to be 17.6 kP, and the disintegration in water results were as follows: first tablet 55s, last tablet 65s. Figure 10 illustrates the pH profile for this formulation as well.

**Table 2A17**

| **Component** | **Mg/tab** | **Conc.** | **mEq** |
|---|---|---|---|
| Omeprazole | 46.7 | 3.2 | |
| Sodium bicarbonate 97%/HPC 3% | 1299 | 89.0 | 15.0 |
| Hydroxypropyl cellulose-EXF | 48 | 3.3 | |
| Croscarmellose sodium | 44 | 3.0 | |
| Sodium stearyl fumarate | 22 | 1.5 | |

The formulation was prepared in a manner analogous to Example 2A16. The hardness was measured to be 25 kP. Figure 10 illustrates the pH profile for this formulation.

**Table 218**

| **Component** | **mg/tab** | **Conc.** | **mEq** |
|---|---|---|---|
| Omeprazole | 53.5 | 3.2 | |
| Sodium bicarbonate 97%/HPC 3% | 1488 | 89.0 | 17.2 |
| Hydroxypropyl cellulose-EXF | 55 | 3.3 | |
| Croscarmellose sodium | 50 | 3.0 | |
| Sodium stearyl fumarate | 25 | 1.5 | |

The formulation was prepared in a manner analogous to Example 2A16. The hardness was measured to be 27 kP. Figure 10 also illustrates the pH profile for this formulation.

**Table 2A19**

| **Component** | **mg/tab** | **Conc.** | **mEq** |
|---|---|---|---|
| Omeprazole | 40.8 | 3.2 | |
| Sodium bicarbonate | 750 | 58.3 | 8.9 |
| Magnesium hydroxide 95% | 368.5 | 28.6 | 12.0 |
| Hydroxypropyl cellulose-EXF | 71 | 5.5 | |
| Croscarmellose sodium | 38 | 3.0 | |
| Sodium stearyl fumarate | 19 | 11.5 | |

The formulation was prepared in a manner analogous to Example 2A16. The caplet was film coated with Colorcon film coating formula# 57U18539 at 3% weight gain and 80W68912 at 4% weight gain. Caplets without coating were also prepared. Using a compression force of 4700 lb_{f}, produced caplets with a friability 0.1%, hardness of 18.4 kP, and disintegration in water of: 33s (first tablet)/42s (last tablet).

**Table 2A20**

| **Component** | **mg/tab** | **Conc.** | **mEq** |
|---|---|---|---|
| Omeprazole | 20.4 | 1.6 | |
| Sodium bicarbonate | 1100 | 88.2 | 13.1 |
| HPC | 76 | 6.1 | |
| Croscarmellose sodium | 38.2 | 3.1 | |
| Magnesium stearate | 12 | 1.0 | |

The formulation was prepared in a manner analogous to Example 2A16. Using a compression force of 11,000 lb_{f}, produced caplets with a hardness 10.9 kP and a friability of 0.7%.

**Table 2A21**

| **Component** | **mg/tab** | **Cone.** | **mEq** |
|---|---|---|---|
| Omeprazole | 20.4 | 1.6 | |
| Sodium bicarbonate | 850 | 67.5 | 10.1 |
| Magnesium hydroxide, 95% | 263.2 | 20.9 | 8.6 |
| Hydroxypropyl cellulose-EXF | 77 | 6.1 | |
| Croscarmellose sodium | 38 | 3.0 | |
| Magnesium stearate | 11 | 0.9 | |

The formulation was prepared in a manner analogous to Example 2A16. Using a compression force of 8,000 lb_{f}, produced caplets with a hardness of 15.8 kP and a friability of 0.7%.

**Table 2A22**

| **Component** | **mg/tab** | **Conc.** | **mEq** |
|---|---|---|---|
| Omeprazole | 20.4 | 1.6 | |
| Sodium bicarbonate | 750 | 59.3 | 8.9 |
| Magnesium hydroxide, 95% | 368.5 | 29.1 | 12.0 |
| Hydroxypropyl cellulose-EXF | 77 | 6.1 | |
| Croscarmellose sodium | 38 | 3.0 | |
| Magnesium stearate | 11.7 | 0.9 | |

The formulation was prepared in a manner analogous to Example 2A16. Using a compression force of 8,600 lb_{f}, produced caplets with a hardness of 20.1 kP, a friability of 0.4%, and disintergration in water of 4 minutes.

**Table 2A23**

| **Component** | **mg/tab** | **Conc.** | **mEq** |
|---|---|---|---|
| Omeprazole | 20.4 | 1.6 | |
| Sodium bicarbonate | 750 | 60.0 | 8.9 |
| Magnesium hydroxide, 95% | 368.5 | 29.5 | 12.0 |
| Hydroxypropyl cellulose-EXF | 63 | 5.0 | |
| Croscarmellose sodium | 38 | 3.0 | |
| Magnesium stearate | 11 | 0.9 | |

The formulation was prepared in a manner analogous to Example 2A16. Using a compression force was of 8,500 lb_{f}, produced caplets with a hardness of 18.8kP, friability of 0.5%, and disintergration in water of: 1.5 minutes (first tablet)/ 4 minutes (last tablet).

**Table 2A24**

| **Component** | **mg/tab** | **Conc.** | **mEq** |
|---|---|---|---|
| Omeprazole | 20.4 | 1.6 | |
| Sodium bicarbonate | 750 | 59.7 | 8.9 |
| Magnesium hydroxide, 95% | 368.5 | 29.3 | 12.0 |
| Hydroxypropyl cellulose-EXF | 69 | 5.5 | |
| Croscarmellose sodium | 38 | 3.0 | |
| Magnesium stearate | 11 | 0.9 | |

The formulation was prepared in a manner analogous to Example 2A16. Using a compression force of 8,500 lb_{f}, produced caplets with a hardness of 19.7 kP, a friability of 0.4%, and disintergration in water of: 2.5 minutes (first tablet)/ 4.8 minutes (last tablet).

**Table 2A25**

| **Component** | **mg/tab** | **Conc.** | **mEq** |
|---|---|---|---|
| Omeprazole | 20.4 | 1.7 | |
| Sodium bicarbonate | 750 | 60.9 | 8.9 |
| Magnesium hydroxide, 95% | 368.5 | 29.9 | 12.0 |
| Hydroxypropyl cellulose-EXF | 69 | 5.6 | |
| Croscarmellose sodium | 12 | 1.0 | |
| Magnesium stearate | 11 | 0.9 | |

The formulation was prepared in a manner analogous to Example Example 2A16. This formulation was rejected because the disintegration time was too long. Using a compression force of 8,500 lb_{f}, produced tablets with a hardness of 18.7kP, a friability of 0.4%, and disintergration in water of 21 minutes (last caplet).

**Table 2A26**

| **Component** | **mg/tab** | **Conc.** | **mEq** |
|---|---|---|---|
| Omeprazole | 20.4 | 1.6 | |
| Sodium bicarbonate | 750 | 59.7 | 8.9 |
| Magnesium hydroxide, 95% | 368.5 | 29.3 | 12.0 |
| Hydroxypropyl cellulose-EXF | 69 | 5.5 | |
| Croscarmellose sodium | 38 | 3.0 | |
| Magnesium stearate | 11 | 0.9 | |

The formulation was prepared in a manner analogous to Example Example 2A16. Using a compression force of 8,500 lbf, produced caplets with a hardness of 19.0 kP, a friability of 0.4%, and disintegration in water of 3 minutes (last caplet).

**Table 2A27**

| **Component** | **Mg/tab** | **Conc.** | **mEq** |
|---|---|---|---|
| Omeprazole | 20.4 | 1.6 | |
| Sodium bicarbonate | 750 | 58.5 | 8.9 |
| Magnesium hydroxide, 95% | 368.5 | 28.7 | 12.0 |
| Hydroxypropyl cellulose-EXF | 69 | 5.4 | |
| Croscarmellose sodium | 64 | 5.0 | |
| Magnesium stearate | 11 | 0.9 | |

The formulation was prepared in a manner analogous to Example 2A16. Using a compression force of 8,500 lb_{f}, produced caplets with ahardness of 18.3 kP, a friability of 0.5%, and disintegration in water of 3.7 minutes (last caplet).

**Table 2A28**

| **Component** | **mg/tab** | **Conc.** | **mEq** |
|---|---|---|---|
| Omeprazole | 20.4 | 1.6 | |
| Sodium bicarbonate | 750 | 59.9 | 8.9 |
| Magnesium hydroxide, 95% | 368.5 | 29.4 | 12.0 |
| Hydroxypropyl cellulose-EXF | 69 | 5.5 | |
| Croscarmellose sodium | 38 | 3.0 | |
| Magnesium stearate | 6 | 0.5 | |

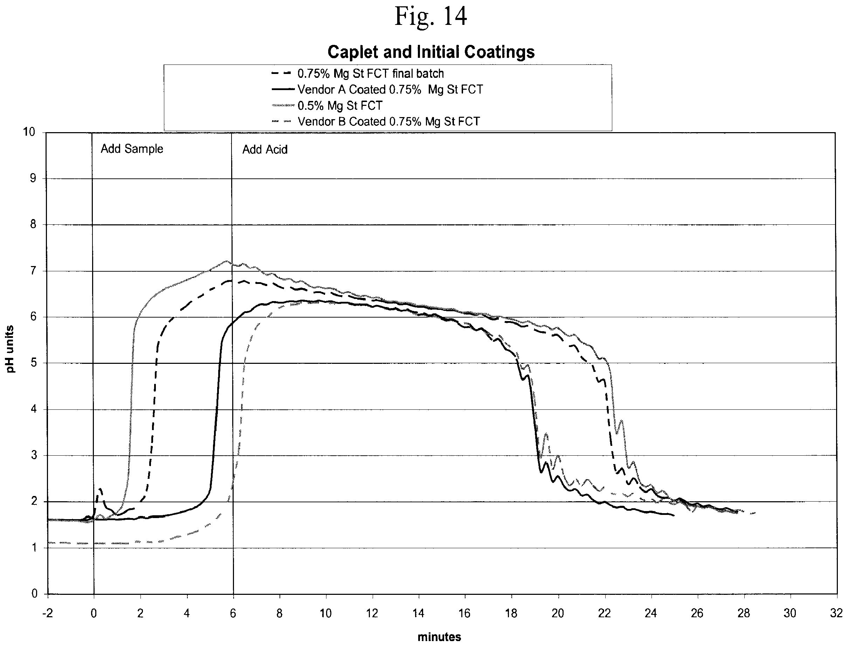

The formulation was prepared in a manner analogous to Example 2A16. Using a compression force of 8,500 lb_{f}, produced caplets with a hardness of 21.8 kP, a friability of 0.4%, and the disintegration in water of 2.6 minutes (last caplet). Figure 14 illustrates a compasion in the pH profiles of omeprazole caplets with magnesium stearate lubricant formulated pursuant to Tables 2A28 and 2A29.

**Table 2A29**

| **Component** | **mg/tab** | **Conc.** | **mEQ** |
|---|---|---|---|
| Omeprazole | 20.4 | 1.6 | |
| Sodium bicarbonate | 750 | 59.7 | 8.9 |
| Magnesium hydroxide, 95% | 368.5 | 29.4 | 12.0 |
| Hydroxypropyl cellulose-EXF | 69 | 5.5 | |
| Croscarmellose sodium | 38 | 3.0 | |
| Magnesium stearate | 9.4 | 0.75 | |

The formulation was prepared in a manner analogous to Example 2A16. The caplets were film coated with HPC or HPMC and HPC based coatings. Caplets without coating were also prepared. Using a compression force of 8,500 lb_{f}, produced caplets with a hardness of 19.8 kP, a friability of 0.4%, and disintegration in water of 4.5 minutes (last caplet).

**Table 2A30**

| **Component** | **mg/tab** | **Conc.** | **mEq** |
|---|---|---|---|
| Omeprazole | 20.4 | 1.6 | |
| Sodium bicarbonate | 750 | 59.6 | 8.9 |
| Magnesium hydroxide, 95% | 368.5 | 29.3 | 12.0 |
| Hydroxypropyl cellulose-EXF | 69 | 5.5 | |
| Croscarmellose sodium | 38 | 3.0 | |
| Magnesium stearate | 12 | 1.0 | |

The formulation was prepared in a manner analogous to Example 2A16. Using a compression force of 8,500 lb_{f}, produced caplets with a hardness of 18.5 kP, a friability of 0.4%, and the disintegration in water of 6 minutes (last caplet).

**Table 2A31**

| **Component** | **Mg/tab** | **Conc.** | **mEq** |
|---|---|---|---|
| Omeprazole | 20.4 | 1.6 | |
| Sodium bicarbonate | 750 | 59.4 | 8.9 |
| Magnesium hydroxide, 95% | 368.5 | 29.2 | 12.0 |
| Hydroxypropyl cellulose-EXF | 69 | 5.5 | |
| Croscarmellose sodium | 38 | 3.0 | |
| Magnesium stearate | 15.8 | 1.25 | |

The formulation was prepared in a manner analogous to Example 2A16. Using a compression force of 8,500 lb_{f}, produced caplets with a hardness of 16.4 kP, a friability of 0.5%, and disintegration in water of 13.5 minutes (last caplet).

**Table 2A32**

| **Component** | **mg/tab** | **Conc.** | **mEq** |
|---|---|---|---|
| Omeprazole | 40.8 | 3.2 | |
| Sodium bicarbonate | 750 | 58.9 | 8.9 |
| Magnesium hydroxide, 95% | 368.5 | 28.9 | 12.0 |
| Hydroxypropyl cellulose-EXF | 69 | 5.4 | |
| Croscarmellose sodium | 38 | 3.0 | |
| Magnesium stearate | 7.5 | 0.6 | |

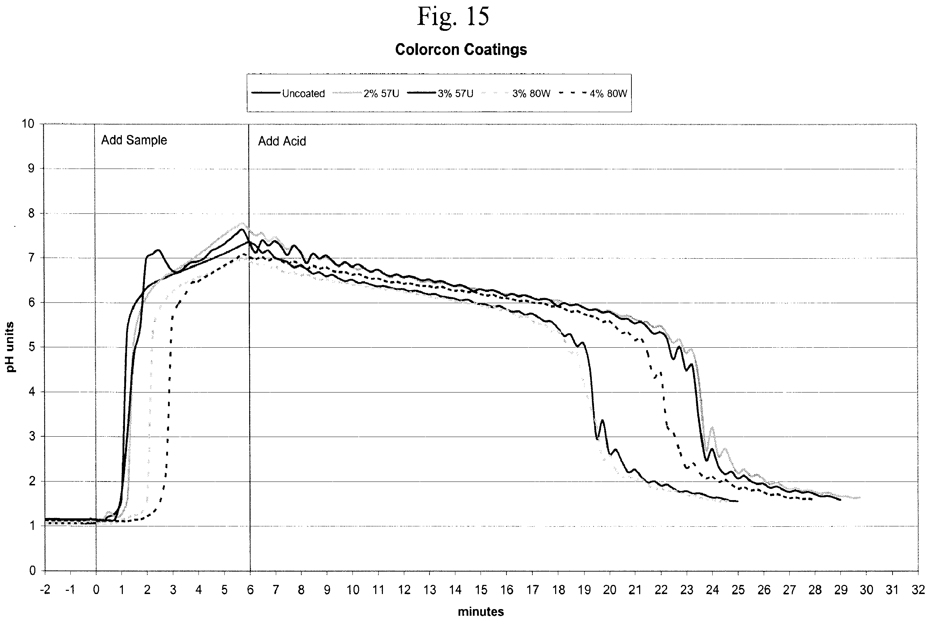

The formulation was prepared in a manner analogous to Example 2A16. The hardness was determined to be 18.5 kP, and the friability 0.4%. Figure 15 illustrates a comparison of the pH profiles of omeprazole caplets with magnesium stearate lubricant formulated pursuant to Table 2A32 with and without film coating.

**Table 2A33**

| **Component** | **mg/tab** | **Conc.** | **mEq** |
|---|---|---|---|
| Omeprazole | 20.4 | 2.3 | |
| Sodium bicarbonate | 500 | 55.3 | 6.0 |
| Magnesium Oxide, Heavy 310-GR | 280 | 31.0 | 13.9 |
| Hydroxypropyl cellulose-EXF | 30 | 3.3 | |
| Croscarmellose sodium | 65 | 7.2 | |
| Magnesium stearate | 8 | 0.9 | |

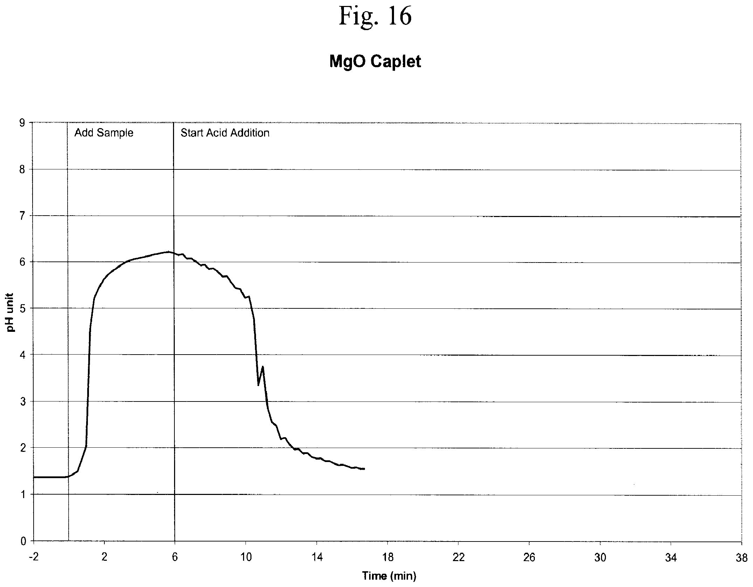

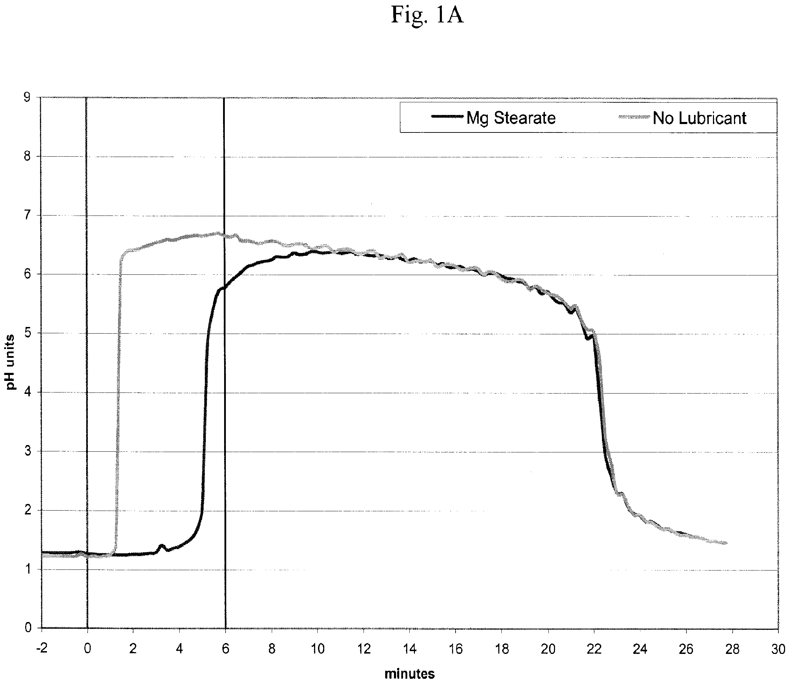

The formulation was prepared in a manner analogous to Example 2A16. The formulation was rejected because the hardness in the range of 5 - 7 kP was determined to be unacceptable. Figure 16 illustrates a comparison in the pH profiles of omeprazole caplets with magnesium stearate lubricant formulated pursuant to Table 2A33.

**Table 2A34**

| **Component** | **Mg/tab** | **Conc.** | **mEq** |
|---|---|---|---|
| Omeprazole | 20.4 | 2.2 | |
| Sodium bicarbonate | 500 | 55.0 | 6.0 |
| Magnesium Oxide, Heavy 310-GR | 280 | 30.8 | 13.9 |
| Hydroxypropyl cellulose-EXF | 36 | 4.0 | |
| Croscarmellose sodium | 65 | 7.1 | |
| Magnesium stearate | 8 | 0.9 | |

The formulation was prepared in a manner analogous to Example 2A16. The formulation was rejected because the hardness in the range of 7.5 - 8.5 kP was determined to be unacceptable since the caplet could not coat due to erosion. The friability was 0.3%.

### Example 2B: Caplet Formulations

All ingredients are mixed well to achieve a homogenous bulk blend which is then compressed into caplets.

**Table 2B1**

| **PPI** | **Antacid** | **Excipient** |
|---|---|---|
| 40 mg omeprazole | 17.1 mEq or 500 mg Mg(OH)₂ | 20 mg Croscarmellose sodium |
| | 3.0 mEq or 250 mg NaHCO₃ | 80mg HPC |
| | | 10 mg sodium stearyl fumarate |
| | 20.1 mEq or 750 mg total buffer | |

**Table 2B2**

| **PPI** | **Antacid** | **Excipient** |
|---|---|---|
| 15 mg lansoprazole | 17.1 mEq or 500 mg Mg(OH)₂ | 20 mg Croscarmellose sodium |
| | 3.0 mEq or 250 mg NaHCO₃ | 80mg HPC |
| | | 10 mg sodium stearyl fumarate |
| | 20.1 mEq or 750 mg total buffer | |

**Table 2B3**

| **PPI** | **Antacid** | **Excipient** |
|---|---|---|
| 10 mg tenatoprazole | 13.7 mEq or 400 mg Mg(OH)₂ | 20 mg Croscarmellose sodium |
| | 3.0 mEq or 250 mg NaHCO₂ | 80mg HPC |
| | | 10 mg sodium stearyl fumarate |
| | 16.7 mEq or 650 mg total buffer | |

**Table 2B4**

| **PPI** | **Antacid** | **Excipient** |
|---|---|---|
| 40 mg omeprazole | 20.6 mEq or 600 mg Mg(OH)₂ | 20 mg Croscarmellose sodium |
| | 3.0 mEq or 250 mg NaHCO₃ | 80mg HPC |
| | | 10 mg sodium stearyl fumarate |
| | 23.6 mEq or 850 mg total buffer | |

**Table 2B5**

| **Table 2B5PPI** | **Antacid** | **Excipient** |
|---|---|---|
| 40 mg rabeprazole | 17.1 mEq or 500 mg Mg(OH)₂ | 20 mg Croscarmellose sodium |
| | 3.0 mEq or 250 mg NaHCO₃ | 80mg HPC |
| | | 10 mg sodium stearyl fumarate |
| | 20.1 mEq or 750 mg total buffer | |

**Table 2B6**

| **PPI** | **Antacid** | **Excipient** |
|---|---|---|
| 15 mg lansoprazole | 17.1 mEq or 500 mg Mg(OH)₂ | 20 mg Croscarmellose sodium |
| | 3.0 mEq or 250 mg NaHCO₃ | 80mgHPC |
| | | 10 mg sodium stearyl fumarate |
| | 20.1 mEq or 750 mg total buffer | |

**Table 2B7**

| **PPI** | **Antacid** | **Excipient.** |
|---|---|---|
| 10 mg pariprazole | 13.7 mEq or 400 mg Mg(OH)₂ | 20 mg Croscarmellose sodium |
| | 3.0 mEq or 250 mg NaHCO₃ | 80mg HPC |
| | | 10 mg sodium stearyl fumarate |
| | 16.7 mEq or 650 mg total buffer | |

**Table 2B8**

| **PPI** | **Antacid** | **Excipient** |
|---|---|---|
| 40 mg omeprazole | 20.6 mEq or 600 mg Mg(OH)₂ | 20 mg Croscarmellose sodium |
| | 3.0 mEq or 250 mg NaHCO₃ | 80 mg HPC |
| | | 10 mg sodium stearyl fumarate |
| | 23.6 mEq or 80 mg total buffer | |

**Table 2B9**

| **PPI** | **Antacid** | **Excipient** |
|---|---|---|
| 20 mg esomeprazole | 20.6 mEq or 600 mg Mg(OH)₂ | 30 mg Croscarmellose sodium |
| | 5.0 mEq or 420 mg NaHCO₃ | 100 mg HPC |
| | | 15 mg sodium stearyl fumarate |
| | 25.6 mEq or 1020 mg total buffer | 3 mg Red #40 Lake |

**Table 2B10**

| **PPI** | **Antacid** | **Excipient** |
|---|---|---|
| 40 mg omeprazole | 24.0 mEq or 700 mg Mg(OH)₂ | 30 mg Croscarmellose sodium |
| | 7.1 mEq or 600 mg NaHCO₃ | 120 mg HPC |
| | | 15 mg sodium stearyl fumarate |
| | 31.1 mEq or 1300 mg total buffer | 1 mg Blue #2 Lake |

**Table 2B11**

| **PPI** | **Antacid** | **Excipient** |
|---|---|---|
| 30 mg lansoprazole | 24.0 mEq or 700 mg Mg(OH)₂ | 30 mg Croscarmellose sodium |
| | 50 mEq or 420 mg NaHCO₃ | 100 mg HPC 1 |
| | | 15 mg sodium stearyl fumarate |
| | 29.0 mEq or 1300 mg total buffer | |

**Table 2B12**

| **PPI** | **Antacid** | **Excipient** |
|---|---|---|
| 60 mg omeprazole | 15.0 mEq or 750 mg Ca(OH)₂ | 30 mg Croscarmellose sodium |
| | 5.4 mEq or 450 mg NaHCO₃ | 100 mg HPC |
| | | 15 mg sodium stearyl fumarate |
| | 20.3 mEq or 1200 mg total buffer | |

**Table 2B13**

| **PPI** | **Antacid** | **Excipient** |
|---|---|---|
| 40 mg pariprazole | 14.0mEq or 700 mg Ca(OH)₂ | 30 mg Croscarmellose sodium |
| | 6.0 mEq or 500 mg NaHCO₃ | 75 mg HPC |
| | | 15 mg sodium stearyl fumarate |
| | 20 mEq or 1200 mg total buffer | |

**Table 2B14**

| **PPI** | **Buffering Agent** | **Excipient** |
|---|---|---|
| 40 mg esomeprazole | 24.0 mEq or 700 mg Mg(OH)₂ | 60 mg Croscarmellose sodium |
| | 7.1 mEq or 600 mg NaHCO₃ | 60 mg pregelatinized starch |
| | | 30mg HPC |
| | 31.1 mEq or 1300 mg total buffer | 15 mg sodium stearyl fumarate |

**Table 2B15**

| **PPI** | **Antacid** | **Excipient** |
|---|---|---|
| 30 mg lansoprazole | 17.1 mEq or 500 mg Mg(OH)₂ | 60 mg Croscarmellose sodium |
| | 5.0 mEq or 420 mg NaHCO₃ | 70 mg pregelatinized starch |
| | | 30mg HPC |
| | 22.1 mEq or 920 mg total buffer | 15 mg sodium stearyl fumarate |

**Table 2B16**

| **PPI** | **Antacid** | **Excipient** |
|---|---|---|
| 60 mg tenatoprazole | 15.0 mEq or 750 mg Ca(OH)₂ | 60 mg Croscarmellose sodium |
| | 5.4 mEq or 450 mg NaHCO₃ | 60 mg pregelatinized starch |
| | | 30mg HPC |
| | 20.3 mEq or 1200 mg total buffer | 15 mg sodium stearyl fumarate |

**Table 2 B17**

| **PPI** | **Antacid** | **Excipient** |
|---|---|---|
| 40 mg pantoprazole | 14.0 mEq or 700 mg Ca(OH)₂ | 60 mg Croscarmellose sodium |
| | 6.0 mEq or 500 mg NaHCO₃ | 60 mg pregelatinized starch |
| | | 30mg HPC |
| | 20 mEq or 1200 mg total buffer | 15 mg sodium stearyl fumarate |

### Example 3A: Chewable Tablet Formulations

The following specific formulation is described by way of illustrating the present invention and is not intended to be limiting.

### Microencapsulation of Omeprazole with 37 w/w%

The process of microencapsulating omeprazole with 37 w/w % Klucel EF (HPC) requires the preparation of a suspension containing Klucel EF, sodium bicarbonate and omeprazole (total solid content of 16.23%) and spray dried using a rotary atomizer. The pH of the suspension was 8.1. Spray rate was 35 Kg/hour and the resulting outlet temperature was 70-85°C. Atomizer speed was 22,000 rpm. The viscosity of the suspension was 680 cps and the pumping system had no difficulty in delivering the suspension to the atomizer. White, fine particles were collected. The median particle size of sample was approximately 80 - 110 -tm. USP No. 2 *in vitro* dissolution test showed drug release of >90% in 15 minutes. The amounts of each component are shown below:

**Table 3A1**

| **Ingredient'** | **Amount Weighed Out (kg)·** | **Calculated *Wt. % in Dried Sample** |
|---|---|---|
| Klucel EF, NF (HPC) | 50 | 61.6 |
| Omeprazo | 30 | 36.9 |
| Sodium Bicarbonate (NaHCO₃) | 1.15 | 1.4 |
| USP Purified Water | 418.85 | -- |

An omeprazole pre-blend containing microencapsulated omeprazole, antacid excipients and other formulation components was prepared. A flavor pre-blend containing sensory components was then prepared. The main blend was then prepared by combining the omeprazole and flavor pre-blends. Magnesium stearate was then added to the main blend and mixed to form a final blend. All blending operations were carried out in appropriately sized V-blenders. Blend uniformity was ensured by testing at various stages of blending. The final blend was then compressed on a high speed rotary tablet press to form the final tablets. The tablet press was a rotary tablet press using ¾" round FFBE tooling gave an acceptable tablet harness and friability in all prototype batches. The amount of each component is listed below in Tables 1.B and 1.C.

**Table 3A2**

| **Ingredient** | **Quantity / 20 mg Tablet** |
|---|---|
| Microencapsulated Omeprazole | 55.1 mg* |
| Sodium Bicarbonate | 600mg |
| Magnesium Hydroxide (95% w/w) | 736.8 mg** |
| Hydroxypropyl Cellulose | 90mg |
| Croscarmellose Sodium | 33mg |
| Xylitol | 200mg |
| Sucralose | 80mg |
| Peach Durarome | 52mg |
| Peppermint Durarome | 10mg |
| Masking Flavor | 27mg |
| Magnesium Stearate | 17mg |
| Red #40 Lake Dye | 2mg |

| | |
|---|---|
| *Spray-dried omeprazole (37% w/w) includes a 2% omeprazole overage in the blend manufacture that helps ensure label claim amount of omeprazole in the final product. **Spray-dried magnesium hydroxide (95% w/w with 5% starch) equivalent to 700 mg of active magnesium hydroxide | |

**Table 3A3**

| **Ingredient** | **Quantity / 40 mgTablet** |
|---|---|
| Microencapsulated Omeprazole | 110.3* |
| Sodium Bicarbonate | 600mg |
| Magnesium Hydroxide (95% w/w) | 736.8 mg** |
| Hydroxypropyl Cellulose | 90mg |
| Croscarmellose Sodium | 33 mg |
| Xylitol | 200mg |
| Sucralose | 80mg |
| Peach Durarome | 52mg |
| Peppermint Durarome | 10mg |
| Masking Flavor | 27mg |
| Magnesium Stearate | 17mg |
| Red #40 Lake Dye | 2mg |

| | |
|---|---|
| *Spray-dried omeprazole (37% w/w) includes a 2% omeprazole overage in the blend manufacture that helps ensure label claim amount of omeprazole in the final product. **Spray-dried magnesium hydroxide (95% w/w with 5% starch) equivalent to 700 mg of active magnesium hydroxide | |

### Example 3B: Chewable Tablet Formulations

All ingredients are mixed well to achieve a homogenous bulk blend which is then compressed into chewable tablets.

**Table 3B1**

| **PPI** | **Antacid** | **Excipient** |
|---|---|---|
| 20 mg omeprazole | 20.6 mEq or 600 mg Mg(OH)₂ | 170 mg Xylitab |
| | 5.0 mEq or 420 mg NaHC)₃ | 30 mg Croscarmellose sodium |
| | 25.6 mEq or 1020 mg total buffer | 100mg HPC |
| | | 25 mg cherry flavor |
| | | 15 mg sodium stearyl fumarate |
| | | 3 mg Red #40 Lake |

**Table 3B2**

| **PPI** | **Antacid** | **Excipient** |
|---|---|---|
| 40 mg tenatoprazole | 24.0 mEq or 700 mg Mg(OH)₂ | 170 mg Dipac sugar |
| | 7.1 mEq or 600 mg NaHCO₃ | 30 mg Croscarmellose sodium |
| | 31.1 mEq or 1300 mg total buffer | 120mg HPC |
| | | 27 mg grape flavor |
| | | 15 mg sodium stearyl fumarate |
| | | 1 mg Red #40 Lake |
| | | 1 mg Blue #2 Lake |

**Table 3B3**

| **PPI** | **Antacid** | **Excipient** |
|---|---|---|
| 15 mg pantoprazole | 17.1 mEq or 500 mg Mg(OH)₂ | 170 mg Dipac sugar |
| | 3.0 mEq or 250 mg NaHC0₃ | 30 mg Croscarmellose sodium |
| | | 120mg HPC |
| | 20.1 mEq or 750 mg total buffer | 27 mg grape flavor |
| | | 15 mg sodium stearyl fumarate |
| | | 1 mg red #40 lake |
| | | 1 mg blue #2 lake |

**Table 3B4**

| **PPI** | **Antacid** | **Excipient** |
|---|---|---|
| 30 mg lansoprazole | 24.0 mEq or 700 mg Mg(OH)₂ | 170 mg Xylitab |
| | 5.0 mEq or 420 mg NaHCO₃ | 30 mg Croscarmellose sodium |
| | 29.0 mEq or 1120 mg total buffer | 100mg HPC |
| | | 25 mg cherry flavor |
| | | 15 mg sodium stearyl fumarate |
| | | 3 mg Red #40 Lake |

**Table 3B5**

| **PPI** | **Antacid** | **Excipient** |
|---|---|---|
| 20 mg omeprazole | 20.6 mEq or 600 mg Mg(OH)₂ | 170 mg Xylitab |
| | 5.0 mEq or 420 mg NaHC0₃ | 30 mg Croscarmellose sodium |
| | | 100mgHPC |
| | 25.6 mEq or 1020 mg total buffer | 40mg Sucralose |
| | | 25 mg cherry flavor |
| | | 15 mg sodium stearyl fumarate |
| | | 3 mg Red #40 Lake |

**Table 3B6**

| **PPI** | **Antacid** | **Excipient** |
|---|---|---|
| 40 mg omeprazole | 24.0 mEq or 700 mg Mg(OH)₂ | 170 mg Dipac sugar |
| | 7.1 mEq or 600 mg NaHCO₃ | 30 mg Croscarmellose sodium |
| | | 120 mg HPC |
| | 31.1 mEq or 1300 mg total buffer | 27 mg grape flavor |
| | | 15 mg sodium stearyl fumarate |
| | | 1mg Red #40 Lake |
| | | 1mg Blue #2 Lake |

**Table 3B7**

| **PPI** | **Antacid** | **Excipient** |
|---|---|---|
| 30 mg paripazole | 24.0 mEq or 700 mg Mg(OH)₂ | 170 mg Xylitab |
| | 5.0 mEq or 420 mg NaHCO₃ | 30 mg Croscarmellose sodium |
| | | 100 mg HPC |
| | 29.0 mEq or 1120 mg total buffer | 15 mg cherry flavor |
| | | 15 mg sodium stearyl fumarate |
| | | 3 mg Red #40 Lake |

**Table 3B8**

| **PPI** | **Antacid** | **Excipient** |
|---|---|---|
| 60 mg omeprazole | 15.0 mEq or 750 mg Ca(OH)₂ | 170 mg Xylitab |
| | 15.0 mEq or 1260 mg NaHCO₃ | 30 mg Croscarmellose sodium |
| | | 100 mg HPC |
| | 30.0 mEq or 2010 mg total buffer | 15 mg cherry flavor |
| | | 15 mg sodium stearyl fumarate |
| | | 3 mg Red #40 Lake |

**Table 3B9**

| **PPI** | **Antacid** | **Excipient** |
|---|---|---|
| 40 mg tenatoprazole | 15.0 mEq or 750 mg Ca(OH)₂ | 170 mg Xylitab |
| | 10.0 mEq or 840 mg NaHCO₃ | 30 mg Croscarmellose sodium |
| | | 100mg HPC |
| | 25.0 mEq or 1590 mg total buffer | 15 mg mint flavor |
| | | 15 mg sodium stearyl fumarate |

**Table 3B10**

| **PPI** | **Antacid** | **Excipient** |
|---|---|---|
| 40 mg rabeprazole | 24.0 mEq or 700 mg Mg(OH)₂ | 60 mg sucralose |
| | 7.1 mEq or 600 mg NaHCO₃ | 60 mg Croscarmellose sodium |
| | | 60 mg pregelatinized starch |
| | 31.1 mEq or 1300 mg total buffer | 30mg HPC |
| | | 27 mg grape flavor |
| | | 15 mg sodium stearyl fumarate |
| | | 1 mg Red #40 Lake |
| | | 1 mg Blue #2 Lake |

**Table 3B11**

| **PPI** | **Antacid** | **Excipient** |
|---|---|---|
| 30 mg lansoprazole | 17.1 mEq or 500 mg Mg(OH)₂ | 60 mg sucralose |
| | 5.0 mEq or 420 mg NaHCO₃ | 60 mg Croscarmellose sodium |
| | | 70 mg pregelatinized starch |
| | 22.1 mEq or 920 mg total buffer | 30mg HPC |
| | | 25 mg cherry flavor |
| | | 15 mg sodium stearyl fumarate |
| | | 3 mg Red #40 Lake |

**Table 3B12**

| **PPI** | **Antacid** | **Excipient** |
|---|---|---|
| 60 mg esomeprazole | 15.0 mEq or 750 mg Ca(OH)₂ | 60 mg sucralose |
| | 15.0 mEq or 1260 mg NaHCO₃ | 60 mg Croscarmellose sodium |
| | | 60 mg pregelatinized starch |
| | 30.0 mEq or 2010 mg total buffer | 30mg HPC |
| | | 25 mg cherry flavor |
| | | 15 mg sodium stearyl fumarate |
| | | 3 mg Red #40 Lake |

**Table 3B13**

| **PPI** | **Antacid** | **Excipient** |
|---|---|---|
| 40 mg omeprazole | 15.0 mEq or 750 mg Ca(OH)₂ | 60 mg sucralose |
| | 10.0 mEq or 840 mg NaHCO₃ | 60 mg Croscarmellose sodium |
| | | 60 mg pregelatinized starch |
| | 25.0 mEq or 1590 mg total buffer | 30mg HPC |
| | | 15 mg mint flavor |
| | | 15 mg sodium stearyl fumarate |

### Example 4: Methods of Treatment/Prevention of Nocturnal Acid Breakthrough

Patients received the 40 mg capsule described in Table 1A3 (IR CAPSULE), the 40 mg caplet described in Table 2A2 (IR CAPLET), or Prevacid. Gastric pH values were recorded continuously for 24 hours, beginning at approximately 0700 hours on Day 7 until approximately 0700 hours on Day 8, using an ambulatory pH recording system with a disposable dual probe catheter. During all recording periods, gastric pH values were each measured once every 4 seconds. The primary endpoint was the occurrence of NAB, *i.e.,* gastric pH< 4 continuously for more than 1 hour during nighttime (from 2200 hours, Day 7 to 0400 hours, Day 8).

Direct comparisons of the effects on gastric acidity of the 40 mg IR CAPSULE and the 40 mg IR CAPLET formulations with the commercially available enteric-coated Prevacid composition were made. The 40 mg IR CAPSULE and 40 mg IR CAPLET formulations were found to be numerically (and in some cases statistically) superior to Prevacid (30 mg) in controlling nocturnal gastric acid. It was concluded that the 40 mg IR CAPSULE and 40 mg IR CAPLET formulations were more effective in treating/preventing NAB than Prevacid (30 mg).

### Example 5: Methods of Treatment/Prevention of Nocturnal Acid Breakthrough

It was sought to determine the efficacy of the chewable tablets described in Example 3A (IR CHEW TAB) for treating NAB and controlling nocturnal gastric acid. In a preliminary phase of an efficacy study, a group of GERD patients suffering from NAB completed a 7 day "crossover" trial in which they were administered at least one of the following proton pump inhibitor (PPI) drugs: Prevacid (30 mg) or Nexium (40 mg). Subsequently, patients were randomized for another trial in which they received a 20 mg IR CHEW TAB or a 40 mg IR CHEW TAB formulation. Gastric and esophageal pH values were recorded continuously for 24 hours, beginning at approximately 0700 hours on Day 7 until approximately 0700 hours on Day 8, using an ambulatory pH recording system with a disposable dual probe catheter. During all recording periods, gastric and esophageal pH values were each measured once every 4 seconds.

The primary endpoint was the occurrence of NAB, *i.e.,* gastric pH < 4 continuously for more than 1 hour during nighttime (from 2200 hours, Day 7 to 0400 hours, Day 8).

Direct comparisons of the effects on gastric and esophageal acidity of the 20 mg IR CHEW TAB or a40 mg IR CHEW TAB formulation with the commercially available enteric-coated PPIs used were made. The 20 mg IR CHEW TAB and 40 mg IR CHEW TAB formulations were found to be numerically (and in some cases statistically) superior to Prevacid (30 mg) in controlling nighttime gastric acid. The 20 mg IR CHEW TAB and 40 mg IR CHEW TAB formulations were also numerically (and at times) significantly superior to Nexium (40 mg) in controlling nighttime gastric acid. It was also concluded that the 20 mg IR CHEW TAB and 40 mg IR CHEW TAB formulations are more effective for treating/preventing NAB than Prevacid and Nexium.

### Example 6: Compressible Sodium Bicarbonate Preparations

Compressible sodium bicarbonate preparations were prepared by the following fluid bed granulation processes: The granulating agent was added to water through a mesh screen and allowed to de-aerate after dissolving. Sodium bicarbonate was placed into fluid bed granulator bowl and heated. The granulating solution was sprayed into the fluid sodium bicarbonate with conditions of a spray rate of about 15 g/min, atomization pressure of about 20 psi; inlet air temperature of about 70°C and a fluid bed temperature of about 47.7°C. The granulated sodium bicarbonate was then passed through a #20 s/s mesh screen.

**Table 6A: Compressible Sodium Bicarbonate/5% HPC**

| **Reagents** | **Quantity (g)** | **%w/w** |
|---|---|---|
| HPC (for granulation) | 250 | 5 |
| Purified Water USP (for granulation) | 2250 | |
| Sodium Bicarbonate #2, USP | 4750 | 95 |
| **Total** | 5000 | 100 |

**Table 6B: Compressible Sodium Bicarbonate/3% HPC**

| | **Quantity(g)** | **%w/w** |
|---|---|---|
| HPC (for granulation) | 150 | 3 |
| Purified Water USP (for granulation) | 1350 | |
| Sodium Bicarbonate #2, USP | 4850 | 97 |
| **Total** | 5000 | 100 |

### Example 7: Compressible Sodium Bicarbonate Preparations

Compressible sodium bicarbonate preparations are prepared by the following fluid bed granulation processes: The granulating agent is added to water through a mesh screen and allowed to de-aerate after dissolving. Sodium bicarbonate is placed in the fluid bed granulator bowl and heated. The granulating solution is sprayed into the fluid sodium bicarbonate with conditions of a spray rate of about 15 g/min, atomization pressure of about 20 psi; inlet air temperature of about 70°C and a fluid bed temperature of about 47.7°C. The granulated sodium bicarbonate is then passed through a #20 s/s mesh screen.

**Table 7A: Compressible Sodium Bicarbonate/5% Pregelatinized Starch**

| **Reagents** | **Quantity(g)** | **%w/w** |
|---|---|---|
| Pregelatinized Starch (for granulation) | 250 | 5 |
| Purified Water USP (for granulation) | 2250 | |
| Sodium Bicarbonate #2. USP | 4750 | 95 |
| **Total** | 5000 | 100 |

**Table 7B: Compressible Sodium Bicarbonate/10% Pregelatinized Starch**

| **Reagents** | **Quantity(g)** | **%w/w** |
|---|---|---|
| Pregelatinized Starch (for granulation) | 500 | 10 |
| Purified Water USP (for granulation) | 4500 | |
| Sodium Bicarbonate #2, USP | 4500 | 90 |
| **Total** | 5000 | 100 |

**Table 7C: Compressible Sodium Bicarbonate/5% PEG-400**

| **Reagents** | **Quantity(g)..** | **%w/w** |
|---|---|---|
| Polyethylene Glyco1400 (for granulation) | 250 | 5 |
| Purified Water USP (for granulation) | 2250 | |
| Sodium Bicarbonate #2, USP | 4750 | 95 |
| **Total** | 5000 | 100 |

### Example 8: Tablet Formulations

Each formulation contains therapeutically effective doses of PPI and sufficient compressible sodium bicarbonate and high viscosity polymers to sustain the release of PPI. Amounts of compressible sodium bicarbonate are expressed in weight as well as in molar equivalents (mEq). The tablets are prepared by blending the PPI and compressible sodium bicarbonate, and homogeneously blending with excipients as shown in the tables below. The appropriate weight of bulk blended composition is compressed using oval shaped tooling in a rotary tablet press to achieve a hardness of 15-20 kP. The PPI can be in a micronized form.

**Table 8A: Omeprazole (20 mg) Tablet**

| **PPI** | **Compressible NaHCO₃** | **Excipient** |
|---|---|---|
| 20 mg omeprazole per tablet | 5 mEq or 420 mg | 30 mg Croscarmellose sodium |
| | Compressible Sodium | 85mg HPC |
| | Bicarbonate/5% HPC | 6 mg magnesium stearate |

**Table 8B: Omeprazole (40 mg) Tablet**

| **PP** | **Compressible NaHCO₃** | **Excipient** |
|---|---|---|
| 40 mg omeprazole per tablet | 9.5 mEq or 800 mg | 40 mg Croscarmellose sodium |
| | Compressible Sodium | 590 mg Natrosol 250M |
| | Bicarbonate/3% HPC | 9 mg magnesium stearate |

**Table 8C: Lansoprazole (15mg) Tablet**

| **PPI** | **Compressible NaHCO₃··.** | **Excipient** |
|---|---|---|
| 15 mg microencapsulated | 5 mEq or 420 mg | 35 mg Croscarmellose sodium |
| lansoprazole per tablet | Compressible Sodium | 150mg HPC |
| | Bicarbonate/5% Pregelatinized | 6 mg sodium stearyl fumarate |
| | Starch | |

**Table 8D: Lansoprazole (30 mg) Tablet**

| **PPI** | ·**Compressible NaHCO₃··.** | **Excipient** |
|---|---|---|
| 30 mg lansoprazole per tablet | 5 mEq or 420 mg | 20 mg Croscarmellose sodium |
| | Compressible Sodium | 320 mg Natrosol 250M |
| | Bicarbonate/10% Pregelatinized | 5 mg magnesium stearate |
| | Starch antacid | |

**Table 8E: Omeprazole (60 mg) Tablet**

| **PPI** | **Compressible NaHCO₃··.** | **:Excipient** |
|---|---|---|
| 60 mg omeprazole per tablet | 9.5 mEq or 800 mg | 20 mg Croscarmellose sodium |
| | Compressible Sodium | 300mg HPC |
| | Bicarbonate/10% Pregelatinized | 4 mg sodium stearyl fumarate |
| | Starch | |

**Table 8F: Omeprazole (60 mg) Tablet**

| **PPI** | **Compressible NaHCO₃··.** | **Excipient** |
|---|---|---|
| 120 mg omeprazole per tablet | 9.5 mEq or 800 mg | 30 mg Croscarmellose sodium |
| | Compressible Sodium | 170mg HPC |
| | Bicarbonate/5% PEG-400 | 8 mg magnesium stearate |

**Table 8G: Omeprazole (10 mg) Tablet**

| **PPI** | **Compressible NaHCO₃** | **Excipient** |
|---|---|---|
| 10 mg microencapsulated | 9.5 mEq or 800 mg | 18 mg Croscarmellose sodium |
| omeprazole per tablet | Compressible Sodium | 357 mg Natrosol 250M |
| | Bicarbonate/5% Pregelatinized | 7 mg magnesium stearate |
| | Starch | |

**Table 8H: Omeprazole (40 mg) Tablet**

| **PPI** | **Compressible NaHCO₃** | **Excipient** |
|---|---|---|
| 40 mg microencapsulated | 5 mEq or 420 mg | 20 mg Croscarmellose sodium |
| omeprazole per tablet | Compressible Sodium | 260 mg Natrosol 250M |
| | Bicarbonate/5% HPC | 5 mg magnesium stearate |

### Example 9A: Immediate and Sustained Release Multilayer Tablet Formulations

The following examples are illustrations of various aspects of the present invention. The full scope described herein is defined by the claims and the entire specification as filed.

**Table 9A-1 - Immediate Release Formulation for IR/SR Dosage**

| **Ingredients** | **Immediate Release Formulation for IR/SR Dosage** | |
|---|---|---|
| | **mg/tab** | **%** |
| Omeprazole | 20.4 | 2.0 |
| Sodium Bicarbonate 97% /HPC 3% | 907.2 | 90.1 |
| Klucel EXF (HPC) | 34.0 | 3.4 |
| Croscarmellose Sodium | 30.0 | 3.0 |
| Sodium Stearyl Fumarate | 15.0 | 1.5 |
| **Totals:** | **1006.6** | **100** |

The immediate release layer shown in Table 9A-1 was prepared according to the following procedure. Compressible sodium bicarbonate 97%/3% HPC was prepared according to the procedure outlined in Examples 6 and 7. The micronized omeprazole and compressible sodium bicarbonate were mixed for 5 minutes. The croscarmellose sodium and hydroxypropyl cellulose were then added and the mixture was mixed for an additional 10 minutes before adding the sodium stearyl fumarate.

**Table 9A-2 - Sustained Release Formulation for IR/SR Dosage**

| **Ingredients** | **SR Klucel (HPC)** | | **SR Natrosol** | | **SR Methocel** | |
|---|---|---|---|---|---|---|
| | **mg/tab** | **%** | **mg/tab** | **%** | **mg/tab** | **%** |
| Omeprazole | 20.4 | 9.71 | 20.4 | 9.71 | 20.4 | 9.71 |
| Sodium Bicarbonate 97% /HPC 3% | 131.4 | 62.57 | 131.4 | 62.57 | 131.4 | 62.57 |
| Klucel HXF (HPC) | 53.0 | 25.24 | -- | -- | -- | -- |
| Natrosol 250 M (HEC) | -- | -- | 53.0 | 25.24 | -- | -- |
| Methocel 100 M CR (HPMC) | -- | -- | -- | -- | 53.0 | 25.24 |
| FD&C Red #40 Lake | 2.0 | 0.95 | -- | -- | -- | -- |
| D&C Yellow #10 Lake | -- | -- | 2.0 | 0.95 | -- | -- |
| FD&C Blue #2 Lake | -- | -- | | | 2.0 | 0.95 |
| Sodium Stearyl Fumarate | 3.2 | 1.52 | 3.2 | 1.52 | 3.2 | 1.52 |
| **Totals:** | **210.0** | **100** | **210.0** | **100** | **210.0** | **100** |

The sustained release layers shown in Table 9A-2 were prepared according to the following procedure. Compressible sodium bicarbonate 97%/3% HPC was prepared according to the procedure outlined in Examples 6 and 7. The micronized omeprazole and compressible sodium bicarbonate were mixed for 5 minutes. The sustained release polymer (hydroxypropyl cellulose, hydroxy ethyl cellulose or hydroxy propyl methyl cellulose) and color were then added and the mixture was mixed for an additional 10 minutes before adding the sodium stearyl fumarate. Three different immediate release/sustained release formulations were prepared by compressing the immediate release layer in Table 9A-1 with one of the sustained release layers show in Table 9A-2. The physical properties of these three immediate release/sustained release tablets are shown in Table 9A-3.

**Table 9A-3**

| **Bi-Layer Tablet Polymer:** | **Klucel (HPC) Bi-layer Tablets** | **Natrosol Bi-layer Tablets** | **Methocel Bi-layer Tablets** |
|---|---|---|---|
| Average Weight (mg): | 1223.4 | 1226.8 | 1216.9 |
| Average Thickness (mm): | 6.25 | 6.2 | 6.23 |
| Average Hardness (kp): | 19.5 | 19. | 21.3 |
| Friability(%): | 0.1% | 0.1% | 0.1% |

The immediate release layer in Table 9A-1 was combined with the sustained release layer described in Table 9A-4. The sustained release layer of the formulation was prepared by blending the DCP and omeprazole for 5 minutes, adding the HPC and blending for 20 minutes and then adding the sodium stearyl fumarate. The immediate release and sustained release formulations were then pressed together to form a bi-layer tablet. The hardness of the bi-layer tablet was 20.9 kP and the friability was 0.05%.

**Table 9A-4**

| **Ingredient** | **% w/w** | **mg/unit** |
|---|---|---|
| Omeprazole, USP | 9.72 | 20.41 |
| Dicalcium Phosphate (DCP) Dihydrate (Emcompress) | 62.57 | 131.39 |
| HPC HXF (Hydroxypropylcellulose) | 25.23 | 52.99 |
| FD&C Red #40 Lake (LDL) | 0.96 | 2.02 |
| Sodium Stearyl Fumarate NF | 1.52 | 3.19 |
| **Total Caplet Blend** | **100.00** | **210.00** |

The immediate release layer in Table 9A-1 was combined with the sustained release layer described in Table 10A-5. The sustained release layer of the formulation was prepared by blending the magnesium hydroxide and omeprazole for 5 minutes, adding the HPC and color and blending for 20 minutes and then adding the sodium stearyl fumarate. The immediate release and sustained release formulations were then pressed together to form a bi-layer tablet. The hardness of the bi-layer tablet was 20.5 kP and the friability was 0.05%.

**Table 9A-5**

| **Material description** | **%w/w** | **mg/unit** |
|---|---|---|
| Omeprazole, USP | 9.72 | 20.41 |
| Magnesium Hydroxide (Mg(OH)₂ MS95) | 62.57 | 131.39 |
| HPC HXF (Hydroxypropylcellulose) | 25.23 | 52.99 |
| FD&C Red #40 Lake (LDL) | 0.96 | 2.02 |
| Sodium Stearyl Fumarate NF | 1.52 | 3.19 |
| **Total Caplet Blend** | **100.00** | **210.00** |

The immediate release layer in Table 9A-1 was combined with the sustained release layer described in Table 9A-6. The sustained release layer of the formulation was prepared by blending the DCP and omeprazole for 5 minutes, adding the HEC and color and blending for 20 minutes and then adding the sodium stearyl fumarate. The immediate release and sustained release formulations were then pressed together to form a bi- layer tablet. The hardness of the bi-layer tablet was 18.9 kP and the friability was 0.05%.

**Table 9A-6**

| **Material description** | **%w/w** | **mg/unit** |
|---|---|---|
| Omeprazole, USP | 9.72 | 20.41 |
| Dicalcium Phosphate (DCP) Dihydrate (Emcompress) | 62.57 | 131.39 |
| HEC (Natrasol-250M) | 25.23 | 52.99 |
| D&C Yellow #10 Lake (LDL) | 0.96 | 2.02 |
| Sodium Stearyl Fumarate NF | 1.52 | 3.19 |
| **Total Caplet Blend** | **100.00** | **210.00** |

The immediate release layer in Table 9A-1 was combined with the sustained release layer described in Table 9A-7. The sustained release layer of the formulation was prepared by blending the magnesium hydroxide and omeprazole for 5 minutes, adding the HEC and color and blending for 20 minutes and then adding the sodium stearyl fumarate. The immediate release and sustained release formulations were then pressed together to form a bi-layer tablet. The friability was 0.09%.

**Table 9A-7**

| **Material description** | **%w/w** | **mg/unit** |
|---|---|---|
| Omeprazole, USP | 9.72 | 20.41 |
| Magnesium Hydroxide (Mg(OH)₂ MS95) | 62.57 | 131.39 |
| HEC (Natrasol-250M) | 25.23 | 52.99 |
| D&C Yellow #10 Lake (LDL) | 0.96 | 2.02 |
| Sodium Stearyl Fumarate NF | 1.52 | 3.19 |
| **Total Caplet Blend** | **100.00** | **210.00** |

The immediate release layer in Table 9A-1 was combined with the sustained release layer described in Table 9A-8. The sustained release layer of the formulation was prepared by blending the DCP and omeprazole for 5 minutes, adding the HPMC and color and blending for 20 minutes and then adding the sodium stearyl fumarate. The immediate release and sustained release formulations were then pressed together to form a bi-layer tablet. The friability was 0.08%.

**Table 9A-8**

| **Material description** | **%w/w** | **mg/unit** |
|---|---|---|
| Omeprazole, USP | 9.72 | 20.41 |
| Dicalcium Phosphate (DCP) Dihydrate (Emcompress) | 62.57 | 131.40 |
| HPMC (Methocel K100 M) | 25.23 | 52.99 |
| FD&C Blue #2 Lake (LDL) | 0.96 | 2.02 |
| Sodium Stearyl Fumarate NF | 1.52 | 3.19 |
| **Total Caplet Blend** | **100.00** | **210.00** |

The immediate release layer in Table 9A-1 was combined with the sustained release layer described in Table 9A-9. The sustained release layer of the formulation was prepared by blending the magnesium hydroxide and omeprazole for 5 minutes, adding the HPMC and color and blending for 20 minutes and then adding the sodium stearyl fumarate. The immediate release and sustained release formulations were then pressed together to form a bi-layer tablet. The friability was 0.08%.

**Table 9A-9**

| **Material description** | **%w/w** | **mg/unit** |
|---|---|---|
| Omeprazole, USP | 9.72 | 20.41 |
| Magnesium Hydroxide (Mg(OH)₂ MS95) | 62.57 | 131.40 |
| HPMC (Methocel K100 M) | 25.23 | 52.99 |
| FD&C Blue #2 Lake (LDL) | 0.96 | 2.02 |
| Sodium Stearyl Fumarate NF | 1.52 | 3.19 |
| **Total Caplet Blend** | **100.00** | **210.00** |

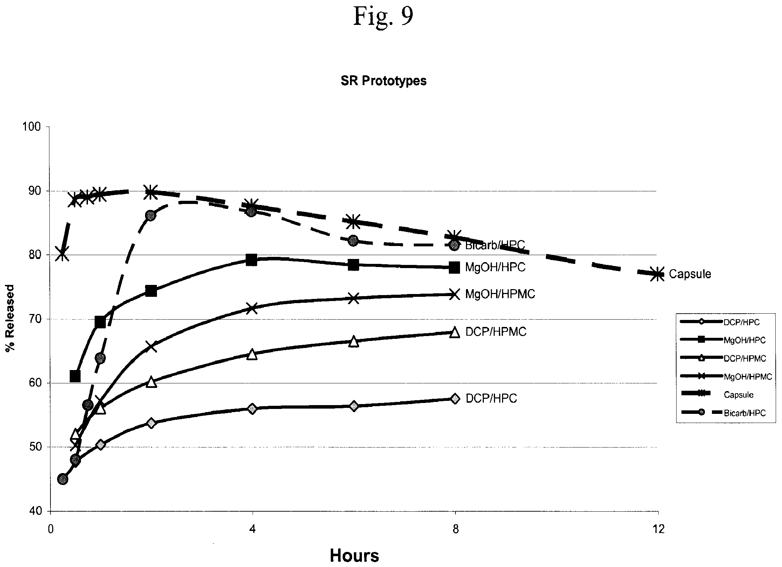

Figure 9 illustrates the dissolution profiles of the intermediate/sustained release formulations described in Tables 1A3, 9A1, 9A2, 9A4, 9A5, 9A8, and 9A9.

### Example 9B: Immediate and Sustained Release Multilayer Tablet Formulations

Amounts of antacid and compressible sodium bicarbonate are expressed in weight as well as in molar equivalents (mEq). The tablets are prepared by blending the PPI and other materials together for each layer in the tables shown below. The layer compositions are compressed using oval shaped tooling in a multilayer tablet press, e.g. a rotary press in bilayer or trilayer tableting mode to achieve a hardness of 15-20 kPa. The PPI can be in a micronized form.

**Table 9B1: Omeprazole (40 mg) Tablet**

| **Immediate Release Layer** | | |
|---|---|---|
| **PPI** | **Antacid** | **Excipient** |
| 20 mg omeprazole base | 5 mEq or 420 mg | 30 mg Croscarmellose sodium |
| | Compressible Sodium | 65 mg HPC |
| | Bicarbonate/3% HPC | 6 mg magnesium stearate |

| **Sustained Release Layer** | | |
|---|---|---|
| **PPI** | **Filler** | **Excipient** |
| 20 mg omeprazole base | 420 mg Dicalcium Phosphate | 85 mg HPC |
| | | 6 mg magnesium stearate |
| | | Red 40 dye |

**Table 9B2: Omeprazole (60 mg) Tablet**

| | | |
|---|---|---|
| **Immediate Release Layer** | | |

| **PPI** | **Antacid** | **Excipient** |
|---|---|---|
| 20 mg omeprazole base | 5.1 mEq or 150 mg Mg(OH)₂ | 30 mg Croscarmellose sodium |
| | 5 mEq or 420 mg | 65 mg HPC |
| | Compressible Sodium | 6 mg sodium stearyl fumarate |
| | Bicarbonate/3% HPC | |

| **Sustained Release Layer** | | |
|---|---|---|
| **PPI** | **Filler** | **Excipient** |
| 40 mg omeprazole base | 420 mg Dicalcium Phospate | 165 mg HPC |
| | | 8 mg sodium stearyl fumarate |
| | | Red 40 dye |

**Table 9B3: Omeprazole (60 mg) Tablet**

| **Immediate Release Layer** | | |
|---|---|---|
| **PPI** | **Antacid** | **Excipient** |
| 20 mg omeprazole magnesium salt | 5.1 mEq or 150 mg Mg(OH)₂ | 30 mg Croscarmellose sodium |
| | 5 mEq or 420 mg | 55 mg Plasdone K-90D |
| | Compressible Sodium | 6 mg sodium stearyl fumarate |
| | Bicarbonate/3% HPC | |

| **Sustained Release Layer** | | |
|---|---|---|
| **PPI** | **Antacid/Filler** | **Excipient** |
| 40 mg omeprazole magnesium salt | 14 mEq or 420 mg | 218 mg Natrosol 250M |
| | Magnesium Hydroxide | 8 mg sodium stearyl fumarate |
| | | Red 40 dye |

**Table 9B4: Omeprazole (80 mg) Tablet**

| **Immediate Release Layer** | | |
|---|---|---|
| **PPI** | **Antacid** | **Excipient** |
| 40 mg omeprazole sodium salt | 5.1 mEq or 150 mg Mg(OH)₂ | 45 mg Croscarmellose sodium |
| | 5 mEq or 420 mg | 50 mg microcrystalline Cellulose |
| | Compressible Sodium | (MCC, PH102) |
| | Bicarbonate/5% Pregelatinized | 10 mg magnesium stearate |
| | Starch | |

| **Sustained Release Layer** | | |
|---|---|---|
| **PPI** | **Antacid/Filler** | **Excipient** |
| 40 mg omeprazole sodium salt | 14 mEq or 420 mg | 165 mg HPC |
| | Magnesium Hydroxide | 8 mg sodium stearyl fumarate |
| | | Red 40 dye |

**Table 9B5: Omeprazole (60 mg) Tablet**

| **Immediate Release Layer** | | |
|---|---|---|
| **PPI** | **Antacid** | **Excipient** |
| 40 mg micro encapsulated omeprazole | 8.6 mEq or 250 mg Mg(OH)₂ | 30 mg Croscarmellose sodium |
| | 5 mEq or 420 mg | 65 mg HPC |
| | Compressible Sodium | 6 mg sodium stearyl fumarate |
| | Bicarbonate/10% Pregelatinized | |
| | Starch | |

| **Sustained Release Layer** | | |
|---|---|---|
| **PPI** | **Filler** | **Excipient** |
| 20 mg micro encapsulated omeprazole | 420 mg Lactose | 218 mg Natrosol 250M |
| | | 8 mg sodium stearyl fumarate |
| | | Red 40 dye |

**Table 9B6: Lansoprazole (45 mg) Tablet**

| **Immediate Release Layer** | | |
|---|---|---|
| **PPI** | **Antacid** | **Excipient** |
| 15 mg micro encapsulated lansoprazole | 6.2 mEq or 180 mg Mg(OH)₂ | 25 mg Croscarmellose sodium |
| | 5 mEq or 420 mg | 40mg HPC |
| | Compressible Sodium | 6 mg sodium stearyl fumarate |
| | Bicarbonate/5% HPC | |

| **Sustained Release Layer** | | |
|---|---|---|
| **PPI** | **Filler** | **Excipient** |
| 30 mg micro encapsulated lansoprazole | 420 mg Lactose | 218 mg HPC |
| | | 8 mg sodium stearyl fumarate |
| | | Red 40 dye |

**Table 9B7: Omeprazole (20 mg) + Lansoprazole (30 mg) Tablet**

| **Immediate Release Layer** | | |
|---|---|---|
| **PPI** | **Antacid** | **Excipient** |
| 20g omeprazole sodium salt | 5.1 mEq or 150 mg Mg(OH)₂ | 25 mg Croscarmellose sodium |
| | 5 mEq or 420 mg | 55 mg Plasdone K-90D |
| | Compressible Sodium | 8 mg magnesium stearate |
| | Bicarbonate/10% Pregelatinized | |
| | Starch | |

| **Sustained Release Layer** | | |
|---|---|---|
| **PPI** | **Filler** | **Excipient** |
| 30 mg micro encapsulated lansoprazole | 420 mg Dicalcium Phosphate | 218 mg HPC |
| | | 8 mg sodium stearyl fumarate |
| | | Red 40 dye |

**Table 9B8: Omeprazole (60 mg) Tablet**

| **Immediate Release Layer** | | |
|---|---|---|
| **PPI** | **Compressible NaHCO₃** | **Excipient** |
| 20 mg omeprazole base | 5 mEq or 420 mg | 30 mg Croscarmellose sodium |
| | Compressible Sodium | 65 mg HPC |
| | Bicarbonate/3% HPC | 6 mg sodium stearyl fumarate |

| **Sustained Release Layer #1** | | |
|---|---|---|
| **PPI** | **Antacid** | **Excipient** |
| 20 mg omeprazole base | 14 mEq or 420 mg | 85 mg Natrosol 250M |
| | Magnesium Hydroxide | 6 mg sodium stearyl fumarate |
| | | Red 40 dye |

| **Sustained Release Layer #2** | | |
|---|---|---|
| **PPI** | **Filler** | **Excipient** |
| 20 mg omeprazole base | 420 mg Dicalcium Phospate | 100 mg HPC |
| | | 6 mg sodium stearyl fumarate |
| | | Red 40 dye |

### Example 10: Immediate Release Formulations for Immediate Release and Sustained Release Capsules

The following immediate release formulations are prepared by the following process: The sodium bicarbonate and omeprazole are combined in a mixer and blended for about 5 minutes. To that mixture, the croscarmellose sodium is added and mixed for about 5 minutes. The blend was then passed through a #20 mesh *s*/*s* screen and then mixed for about 10 minutes. Magnesium stearate or sodium stearyl fumarate is then added to the mixture and blended for about 3 minutes. The immediate release powder was then encapsulated along with sustained release mini-tablets into hard gelatin capsule shells using a manual capsule filler, although an automatic filler can also be used.

**Table 10A1: 0meprazole Immediate Release Formulations**

| **Ingredients** | **A (Mg/Caps)** | **B (Mg/Caps)** | **C (Mg/Caps)** | **D (Mg/Caps)** |
|---|---|---|---|---|
| Omeprazole USP | 20 | 20 | 40 | 40 |
| Sodium Bicarbonate #2, USP | 420 | 420 | 420 | 420 |
| Croscarmellose Sodium NF | 13 | 13 | 13 | 13 |
| Magnesium Stearate NF | 4 | 0 | 4 | 0 |
| Sodium Stearyl Fumarate NF | 0 | 4 | 0 | 4 |
| Totals: | 457 | 457 | 477 | 477 |

### Example 11: Immediate Release and Sustained Release Capsule Formulations

Immediate release and sustained release capsules are prepared with at least one therapeutic dose of PPI to obtain peak bioavailability in approximately 45 minutes in a powder form and at least one therapeutic dose of PPI in sustained bioavailability release for about 4 to 20 hours in mini-tablets. The sustained release mini-tablets were prepared by blending the PPI and other excipients together in the tables shown below. At least one sustained release mini-tablet and immediate release powder were encapsulated together into a hard gelatin capsule (e.g. size 00 capsule).

**Table 11 A: Omeprazole (60 mg) Capsule**

| **Immediate Release Powder** | | |
|---|---|---|
| **PPI** | **Antacid** | **Excipient** |
| 20 mg omeprazole base | 5 mEq or 420 mg | 30 mg Croscarmellose sodium |
| | Compressible Sodium | 10 mg magnesium stearate |
| | Bicarbonate/3% HPC | |

| **Three Sustained Release Mini-tablets** | | |
|---|---|---|
| **PPI/mini-tablet** | **Filler/mini-tablet** | **Excipient/mini-tablet** |
| 13.3 mg omeprazole base | 140 mg Dicalcium Phospate | 30mg HPC |
| | | 2 mg magnesium stearate |

**Table 11 B: Omeprazole (80 mg) Capsule**

| **Immediate Release Powder** | | |
|---|---|---|
| **PPI** | **Antacid** | **Excipient** |
| 40 mg omeprazole magnesium salt | 4.8 mEq or 400 mg | 30 mg Croscarmellose sodium |
| | Sodium Bicarbonate #2, USP | 10 mg sodium stearyl fumarate |
| | 5.1 mEq or 150 Mg(OH)₂ | |

| **Sustained Release Layer** | | |
|---|---|---|
| **PPI/mini-tablet** | **Filler/mini-tablet** | **Excipient/mini-tablet** |
| 13.3 mg omeprazole base | 140 mg Lactose | 50mg HPC |
| | | 2 mg magnesium stearate |

**Table 11 C: Omeprazole (80 mg) Capsule**

| **Immediate Release Powder** | | |
|---|---|---|
| **PPI** | **Antacid** | **Excipient** |
| 40 mg omeprazole magnesium salt | 4.8 mEq or 400 mg | 30 mg Croscarmellose sodium |
| | Compressible Sodium | 10 mg sodium stearyl fumarate |
| | Bicarbonate/3% HPC 5.1 mEq or 150 Mg(OH)₂ | |

| **First Sustained Release Mini-tablet** | | |
|---|---|---|
| **PPI/mini-tablet** | **Filler** | **Excipient/mini-tablet** |
| 13.3 mg omeprazole base | 140 mg Dicalcium Phosphate | 30 mg Natrosol 250M |
| | | 2 mg magnesium stearate |

| **Second Sustained Release Mini-tablet** | | |
|---|---|---|
| **PPI/mini-tablet** | **Antacid** | **Excipient/mini-tablet** |
| 13.3 mg omeprazole base | 4.8 mEq or 140 mg | 50mg HPC |
| | Magnesium Hydroxide | 2 mg magnesium stearate |

| **Third Sustained Release Mini-tablet** | | |
|---|---|---|
| **PPI/mini-tablet** | **Filler** | **Excipient/mini-tablet** |
| 13.3 mg omeprazole base | 140 mg Lactose | 80mg HPC |
| | | 2 mg magnesium stearate |

**Table 11 D: Lansoprazole (15mg)+ Omeprazole (40mg) Capsule**

| **Immediate Release Powder** | | |
|---|---|---|
| **PPI** | **Antacid** | **Excipient** |
| 15 mg microencapsulated lansoprazole | 2.6 mEq or 220 mg | 20 mg Croscarmellose sodium |
| | Compressible Sodium | 6 mg magnesium stearate |
| | Bicarbonate/5% HPC 6.9 mEq or 200 Mg(OH)₂ | |

| **Three Sustained Release Mini-tablets** | | |
|---|---|---|
| **PPI/mini-tablet** | **Filler** | **Excipient/mini-tablet** |
| 13.3 mg omeprazole sodium salt | 140 mg Dicalcium Phospate | 50mg HPC |
| | | 2 mg magnesium stearate |

**Table 11 E: Omeprazole (60 mg) + Lansoprazole (30 mg) Capsule**

| **Immediate Release Powder** | | |
|---|---|---|
| **PPI** | **Antacid** | **Excipient** |
| 40 mg omeprazole magnesium salt | 4.8 mEq or 400 mg | 30 mg Croscarmellose sodium |
| | Sodium Bicarbonate #2, USP | 10 mg sodium stearyl fumarate |
| | 5.1 mEq or 150Mg(OH)₂ | |

| **First Sustained Release Mini-tablet** | | |
|---|---|---|
| **PPI/mini-tablet** | **Filler** | **Excipient/mini-tablet** |
| 30 mg lansoprazole base | 140 mg Dicalcium Phosphate | 60 mg Natrosol 250M |
| | | 4 mg magnesium stearate |

| **Second and Third Sustained Release Mini-tablets** | | |
|---|---|---|
| **PPI/mini-tablet** | **Filler** | **Excipient/mini-tablet** |
| 10 mg omeprazole base | 140 mg Lactose | 50mg HPC |
| | | 2 mg magnesium stearate |

## Claims

1. A pharmaceutical composition in the form of a capsule which achieves an *in vitro* initial rise in pH within about 4 minutes comprising:
(a) 10 mg to 100 mg of at least one acid labile bicyclic-aryl-imidazole proton pump inhibiting agent;
(b) at least one anatacid in an amount sufficient to increase gastric fluid pH to a pH that prevents acid degradation of at least some of the proton pump inhibitor in the gastric fluid; wherein the antacid comprises at least 400 mg of NaHCO₃; and
(c) 0.5 wt-% to 3 wt-% of the hydrophilic lubricant sodium stearyl fumarate

2. A pharmaceutical composition as claimed in claim 1 wherein the proton pump inhibitor is omeprazole, esomeprazole, or lansoprazole, or a pharmaceutically acceptable salt thereof.

3. A pharmaceutical composition as claimed in claim 1 which comprises at least 800 mg of NaHCO₃.

4. A pharmaceutical composition as claimed in claim 1 wherein the composition further comprises greater than 1 wt-% of a disintegrant.

5. The pharmaceutical composition according to claim 4, wherein the composition further comprises between 2 wt-% to 6 wt-% croscarmellose sodium.

6. The pharmaceutical composition according to claim 1, wherein the proton pump inhibitor is omeprazole.

## Patentansprüche

1. Pharmazeutische Zusammensetzung in der Form einer Kapsel, die einen anfänglichen *In-vitro*-Anstieg im pH-Wert innerhalb etwa 4 Minuten erreicht, Folgendes umfassend:
(a) 10 mg bis 100 mg wenigstens eines säurelabilen bicyclischen Arylimidazol Protonenpumpenhemmstoffes;
(b) wenigstens ein Antazidum in einer Menge, die ausreichend ist, einen pH-Wert der Magenflüssigkeit auf einen pH-Wert zu erhöhen, der einen Säureabbau von wenigstens einem Teil des Protonenpumpeninhibitors in der Magenflüssigkeit verhindert; wobei das Antazidum wenigstens 400 mg NaHCO₃ umfasst; und
(c) 0,5 Gew.-% bis 3 Gew.-% des hydrophilen Natriumstearylfumaratgleitmittels.

2. Pharmazeutische Zusammensetzung nach Anspruch 1, wobei der Protonenpumpeninhibitor Omeprazol, Esomeprazol oder Lansoprazol oder ein pharmazeutisch unbedenkliches Salz davon ist.

3. Pharmazeutische Zusammensetzung nach Anspruch 1, die wenigstens 800 mg NaHCO₃ umfasst.

4. Pharmazeutische Zusammensetzung nach Anspruch 1, wobei die Zusammensetzung ferner mehr als 1 Gew.-% eines Zerfallsmittels umfasst.

5. Pharmazeutische Zusammensetzung nach Anspruch 4, wobei die Zusammensetzung ferner zwischen 2 Gew.-% bis 6 Gew.-% Croscarmellose-Natrium umfasst.

6. Pharmazeutische Zusammensetzung nach Anspruch 1, wobei der Protonenpumpeninhibitor Omeprazol ist.

## Revendications

1. Composition pharmaceutique sous la forme d'une capsule qui donne une augmentation initiale *in vitro* de pH en moins d'environ 4 minutes, comprenant :
(a) 10 à 100 mg d'au moins un agent d'inhibition de pompe à protons à base d'aryl-imidazole bicyclique à acide labile ;
(b) au moins un antiacide en quantité suffisante pour accroître le pH de fluide gastrique à une valeur qui empêche la dégradation d'acide d'au moins une partie de l'inhibiteur de pompe à protons dans le fluide gastrique ; l'antiacide comprenant au moins 400 mg de NaHCO₃ ;
(c) 0,5 à 3 % en poids du fumarate de stéaryl sodium lubrifiant hydrophile.

2. Composition pharmaceutique selon la revendication 1, dans laquelle l'inhibiteur de pompe à protons est l'oméprazole, l'ésoméprazole ou le lansoprazole ou un sel pharmaceutiquement acceptable de ceux-ci.

3. Composition pharmaceutique selon la revendication 1, qui comprend au moins 800 mg de NaHCO₃.

4. Composition pharmaceutique selon la revendication 1, la composition comprenant en outre plus de 1 % en poids d'un désintégrant.

5. Composition pharmaceutique selon la revendication 4, la composition comprenant en outre entre 2 et 6 % en poids de croscarmellose sodique.

6. Composition pharmaceutique selon la revendication 1, dans laquelle l'inhibiteur de pompe à protons est l'oméprazole.
